# EUROPEAN PATENT APPLICATION

(11) **EP 3 988 122 A1**
(43) Date of publication of application: **27.04.2022**
(21) Application number: 20903323.2
(22) Date of filing: 13.11.2020
(51) Int. Cl.: A61K 47/64, A61K 38/17, A61K 45/00, A61K 9/00, A61K 47/38, A61K 47/36, A61K 31/196, A61K 31/07, A61P 27/02, C07K 19/00

(54) **APPLICATION OF TRANSTHYRETIN IN ENTERING EYE AND PREPARING DROP**

(30) Priority: 17.12.2019 CN 201911302937; 05.06.2020 CN 202010506950; 16.09.2020 CN 202010976582; 16.09.2020 CN 202010974997
(71) Applicant: Yizhou (Shanghai) Biological Medicine Co, Ltd., China (Shanghai) Pilot Free Trade Zone Shanghai 201203 (CN)
(72) Inventor: XIN, Yu, Wuxi, Jiangsu 214122 (CN)
(74) Representative: Mewburn Ellis LLP
(86) International application number: PCT/CN2020/128588
(87) International publication number: WO 2021/120937

(57) **Abstract**

The present invention provides an application of transthyretin serving as a carrier for a protein and/or polypeptide drug to enter an eye through an eye barrier. The transthyretin is a protein consisting of amino acid as shown in SEQ ID NO: 1 or a mutation thereof or a modification thereof. Further provided are an application of transthyretin and/or a fusion protein of the transthyretin and a drug in preparation of a drop, and a drop. The drug is a protein and/or polypeptide drug. The transthyretin has good biocompatibility and safety in human bodies, can effectively convey a foreign protein and/or polypeptide into the eye, and achieves an effect of treating eye diseases.

SEQUENCE LISTING

<110>  Í¯åû£¨ÉÏ°££©Ò½ÁÆÆ÷ÐµÓÐÏÞ¹«Ë¾

<120>  ×ª¼××´ÏÙËØµ°°×½øÈëÑÛÄÚÒÔ¼°ÔÚÖÆ±¸µÎ¼ÁÖÐµÄÓ¦ÓÃ

<130>  P20406281CF

<150>  201911302937.3
<151>  2019-12-17

<150>  202010506950.7
<151>  2020-06-05

<150>  202010976582.2
<151>  2020-09-16

<150>  202010974997.6
<151>  2020-09-16

<160>  11

<170>  PatentIn version 3.5

<210>  1
<211>  127
<212>  PRT
<213>  Homo sapiens

<400>  1

Gly Pro Thr Gly Thr Gly Glu Ser Lys Cys Pro Leu Met Val Lys Val
1               5                   10                  15

Leu Asp Ala Val Arg Gly Ser Pro Ala Ile Asn Val Ala Val His Val
            20                  25                  30

Phe Arg Lys Ala Ala Asp Asp Thr Trp Glu Pro Phe Ala Ser Gly Lys
        35                  40                  45

Thr Ser Glu Ser Gly Glu Leu His Gly Leu Thr Thr Glu Glu Glu Phe
    50                  55                  60

Val Glu Gly Ile Tyr Lys Val Glu Ile Asp Thr Lys Ser Tyr Trp Lys
65                  70                  75                  80

Ala Leu Gly Ile Ser Pro Phe His Glu His Ala Glu Val Val Phe Thr
                85                  90                  95

Ala Asn Asp Ser Gly Pro Arg Arg Tyr Thr Ile Ala Ala Leu Leu Ser
            100                 105                 110

Pro Tyr Ser Tyr Ser Thr Thr Ala Val Val Thr Asn Pro Lys Glu
        115                 120                 125

<210>  2
<211>  381
<212>  DNA
<213>  Artificial Sequence

<220>
<223>  TTR³ÉÊìÆ¬¶ÎÓÅ»¯µÄ°ËÈáÐòÁÐ

<400>  2
ggtccgaccg gtaccggtga atctaaatgc ccgctgatgg ttaaagttct ggacgctgtt      60

cgtggttctc cggctatcaa cgttgctgtt cacgttttcc gtaaagctgc tgacgacacc     120

tgggaaccgt tcgcttctgg taaaacctct gaatctggtg aactgcacgg tctgaccacc     180

gaagaagaat tcgttgaagg tatctacaaa gttgaaatcg acaccaaatc ttactggaaa     240

gctctgggta tctctccgtt ccacgaacac gctgaagttg ttttcaccgc taacgactct     300

ggtccgcgtc gttacaccat cgctgctctg ctgtctccgt actcttactc taccaccgct     360

gttgttacca acccgaaaga a                                               381


<210>  3
<211>  4693
<212>  DNA
<213>  Artificial Sequence

<220>
<223>  ÖÊÁ£ÕûÌå°ËÈáÐòÁÐ

<400>  3
aattcttaag aaggagatat acatatgaga ggatcgcatc atcatcatca tcatggatcc      60

ggtccgaccg gtaccggtga atctaaatgc ccgctgatgg ttaaagttct ggacgctgtt     120

cgtggttctc cggctatcaa cgttgctgtt cacgttttcc gtaaagctgc tgacgacacc     180

tgggaaccgt tcgcttctgg taaaacctct gaatctggtg aactgcacgg tctgaccacc     240

gaagaagaat tcgttgaagg tatctacaaa gttgaaatcg acaccaaatc ttactggaaa     300

gctctgggta tctctccgtt ccacgaacac gctgaagttg ttttcaccgc taacgactct     360

ggtccgcgtc gttacaccat cgctgctctg ctgtctccgt actcttactc taccaccgct     420

gttgttacca acccgaaaga atgaaagctt ctgttttggc ggatgagaga agattttcag     480

cctgatacag attaaatcag aacgcagaag cggtctgata aaacagaatt tgcctggcgg     540

cagtagcgcg gtggtcccac ctgaccccat gccgaactca gaagtgaaac gccgtagcgc     600

cgatggtagt gtggggtctc cccatgcgag agtagggaac tgccaggcat caaataaaac     660

gaaaggctca gtcgaaagac tgggcctttc gttttatctg ttgtttgtcg gtgaacgctc     720

tcctgagtag gacaaatccg ccgggagcgg atttgaacgt tgcgaagcaa cggcccggag     780

ggtggcgggc aggacgcccg ccataaactg ccaggcatca aattaagcag aaggccatcc     840

tgacggatgg cctttttgcg tttctacaaa ctcttttgtt tatttttcta aatacattca     900

aatatgtatc cgctcatgag acaataaccc tgataaatgc ttcaataata ttgaaaaagg 960

aagagtatga gtattcaaca tttccgtgtc gcccttattc ccttttttgc ggcattttgc 1020

cttcctgttt ttgctcaccc agaaacgctg gtgaaagtaa aagatgctga agatcagttg 1080

ggtgcacgag tgggttacat cgaactggat ctcaacagcg gtaagatcct tgagagtttt 1140

cgccccgaag aacgttttcc aatgatgagc acttttaaag ttctgctatg tggcgcggta 1200

ttatcccgtg ttgacgccgg gcaagagcaa ctcggtcgcc gcatacacta ttctcagaat 1260

gacttggttg agtactcacc agtcacagaa aagcatctta cggatggcat gacagtaaga 1320

gaattatgca gtgctgccat aaccatgagt gataacactg cggccaactt acttctgaca 1380

acgatcggag gaccgaagga gctaaccgct tttttgcaca acatggggga tcatgtaact 1440

cgccttgatc gttgggaacc ggagctgaat gaagccatac caaacgacga gcgtgacacc 1500

acgatgcctg tagcaatggc aacaacgttg cgcaaactat taactggcga actacttact 1560

ctagcttccc ggcaacaatt aatagactgg atggaggcgg ataaagttgc aggaccactt 1620

ctgcgctcgg cccttccggc tggctggttt attgctgata aatctggagc cggtgagcgt 1680

gggtctcgcg gtatcattgc agcactgggg ccagatggta agccctcccg tatcgtagtt 1740

atctacacga cggggagtca ggcaactatg gatgaacgaa atagacagat cgctgagata 1800

ggtgcctcac tgattaagca ttggtaactg tcagaccaag tttactcata tatactttag 1860

attgatttaa aacttcattt ttaatttaaa aggatctagg tgaagatcct ttttgataat 1920

ctcatgacca aaatccctta acgtgagttt tcgttccact gagcgtcaga ccccgtagaa 1980

aagatcaaag gatcttcttg agatcctttt tttctgcgcg taatctgctg cttgcaaaca 2040

aaaaaaccac cgctaccagc ggtggtttgt ttgccggatc aagagctacc aactcttttt 2100

ccgaaggtaa ctggcttcag cagagcgcag ataccaaata ctgtccttct agtgtagccg 2160

tagttaggcc accacttcaa gaactctgta gcaccgccta catacctcgc tctgctaatc 2220

ctgttaccag tggctgctgc cagtggcgat aagtcgtgtc ttaccgggtt ggactcaaga 2280

cgatagttac cggataaggc gcagcggtcg ggctgaacgg ggggttcgtg cacacagccc 2340

agcttggagc gaacgaccta caccgaactg agatacctac agcgtgagct atgagaaagc 2400

gccacgcttc ccgaagggag aaaggcggac aggtatccgg taagcggcag ggtcggaaca 2460

ggagagcgca cgagggagct tccaggggga aacgcctggt atctttatag tcctgtcggg 2520

tttcgccacc tctgacttga gcgtcgattt ttgtgatgct cgtcaggggg gcggagccta 2580

tggaaaaacg ccagcaacgc ggccttttta cggttcctgg ccttttgctg gccttttgct 2640

cacatgttct ttcctgcgtt atcccctgat tctgtggata accgtattac cgcctttgag 2700

tgagctgata ccgctcgccg cagccgaacg accgagcgca gcgagtcagt gagcgaggaa 2760

gcggaagagc gcctgatgcg gtattttctc cttacgcatc tgtgcggtat ttcacaccgc 2820

atatatggtg cactctcagt acaatctgct ctgatgccgc atagttaagc cagtatacac 2880

tccgctatcg ctacgtgact gggtcatggc tgcgccccga cacccgccaa cacccgctga 2940

cgcgccctga cgggcttgtc tgctcccggc atccgcttac agacaagctg tgaccgtctc 3000

cgggagctgc atgtgtcaga ggttttcacc gtcatcaccg aaacgcgcga ggcagctgcg 3060

gtaaagctca tcagcgtggt cgtgaagcga ttcacagatg tctgcctgtt catccgcgtc 3120

cagctcgttg agtttctcca gaagcgttaa tgtctggctt ctgataaagc gggccatgtt 3180

aagggcggtt ttttcctgtt tggtcacttg atgcctccgt gtaaggggga atttctgttc 3240

atgggggtaa tgataccgat gaaacgagag aggatgctca cgatacgggt tactgatgat 3300

gaacatgccc ggttactgga acgttgtgag ggtaaacaac tggcggtatg gatgcggcgg 3360

gaccagagaa aaatcactca gggtcaatgc cagcgcttcg ttaatacaga tgtaggtgtt 3420

ccacagggta gccagcagca tcctgcgatg cagatccgga acataatggt gcagggcgct 3480

gacttccgcg tttccagact ttacgaaaca cggaaaccga agaccattca tgttgttgct 3540

caggtcgcag acgttttgca gcagcagtcg cttcacgttc gctcgcgtat cggtgattca 3600

ttctgctaac cagtaaggca accccgccag cctagccggg tcctcaacga caggagcacg 3660

atcatgcgca cccgtggcca ggacccaacg ctgcccgaga tgcgccgcgt gcggctgctg 3720

gagatggcgg acgcgatgga tatgttctgc caagggttgg tttgcgcatt cacagttctc 3780

cgcaagaatt gattggctcc aattcttgga gtggtgaatc cgttagcgag gtgccgccgg 3840

cttccattca ggtcgaggtg gcccggctcc atgcaccgcg acgcaacgcg gggaggcaga 3900

caaggtatag ggcggcgcct acaatccatg ccaacccgtt ccatgtgctc gccgaggcgg 3960

cataaatcgc cgtgacgatc agcggtccag tgatcgaagt taggctggta agagccgcga 4020

gcgatccttg aagctgtccc tgatggtcgt catctacctg cctggacagc atggcctgca 4080

acgcgggcat cccgatgccg ccggaagcga gaagaatcat aatggggaag gccatccagc 4140

ctcgcgtcgc gaacgccagc aagacgtagc ccagcgcgtc ggccgccatg ccggcgataa 4200

tggcctgctt ctcgccgaaa cgtttggtgg cgggaccagt gacgaaggct tgagcgaggg 4260

cgtgcaagat tccgaatacc gcaagcgaca ggccgatcat cgtcgcgctc cagcgaaagc 4320

ggtcctcgcc gaaaatgacc cagagcgctg ccggcacctg tcctacgagt tgcatgataa 4380

agaagacagt cataagtgcg gcgacgatag tcatgccccg cgcccaccgg aaggagctga 4440

ctgggttgaa ggctctcaag ggcatcggtc gacgctctcc cttatgcgac tcctgcatta 4500

ggaagcagcc cagtagtagg ttgaggccgt tgagcaccgc cgccgcaagg aatggtgcat 4560

gcatcgatca ccacaattca gcaaattgtg aacatcatca cgttcatctt tccctggttg 4620

ccaatggccc attttcctgt cagtaacgag aaggtcgcga attcaggcgc tttttagact 4680

ggtcgtaatg aac                                                    4693

<210>  4
<211>  420
<212>  DNA
<213>  Artificial Sequence

<220>
<223>  His-tag-TTR

<400>  4
atgagaggat cgcatcatca tcatcatcat ggatccggtc cgaccggtac cggtgaatct      60

aaatgcccgc tgatggttaa agttctggac gctgttcgtg gttctccggc tatcaacgtt     120

gctgttcacg ttttccgtaa agctgctgac gacacctggg aaccgttcgc ttctggtaaa     180

acctctgaat ctggtgaact gcacggtctg accaccgaag aagaattcgt tgaaggtatc     240

tacaaagttg aaatcgacac caaatcttac tggaaagctc tgggtatctc tccgttccac     300

gaacacgctg aagttgtttt caccgctaac gactctggtc cgcgtcgtta caccatcgct     360

gctctgctgt ctccgtactc ttactctacc accgctgttg ttaccaaccc gaaagaatga     420

<210>  5
<211>  1137
<212>  DNA
<213>  Artificial Sequence

<220>
<223>  His-tag-TTR-GFP

<400>  5
atgcgtggtt ctcaccacca ccaccaccac ggttctggtc cgaccggtac cggtgaatct      60

aaatgcccgc tgatggttaa agttctggac gctgttcgtg gttctccggc tatcaacgtt     120

gctgttcacg ttttccgtaa agctgctgac gacacctggg aaccgttcgc ttctggtaaa     180

acctctgaat ctggtgaact gcacggtctg accaccgaag aagaatttgt tgaaggtatc     240

tacaaagttg aaatcgacac caaatcttac tggaaagctc tgggtatctc tccgttccac     300

gaacacgctg aagttgtttt caccgctaac gactctggtc cgcgtcgtta caccatcgct     360

gctctgctgt ctccgtactc ttactctacc accgctgttg ttaccaaccc gaaagaaggt     420

tctatgtcta aaggtgaaga actgttcacc ggtgttgttc cgatcctggt tgaactggac     480

ggtgacgtta acggtcacaa attctctgtt tctggtgaag gtgaaggtga cgctacctac     540

ggtaaactga ccctgaaatt catctgcacc accggtaaac tgccggttcc gtggccgacc     600

ctggttacca ccttcaccta cggtgttcag tgcttctctc gttacccgga ccacatgaaa     660

cagcacgact tcttcaaatc tgctatgccg gaaggttacg ttcaggaacg taccatcttc     720

ttcaaagacg acggtaacta caaaacccgt gctgaagtta aattcgaagg tgacaccctg     780

gttaaccgta tcgaactgaa aggtatcgac ttcaaagaag acggtaacat cctgggtcac     840

aaactggaat acaactacaa ctctcacaac gtttacatca tggctgacaa acagaaaaac 900

ggtatcaaag ttaacttcaa aatccgtcac aacatcgaag acggttctgt tcagctggct 960

gaccactacc agcagaacac cccgatcggt gacggtccgg ttctgctgcc ggacaaccac 1020

tacctgtcta cccagtctgc tctgtctaaa gacccgaacg aaaaacgtga ccacatggtt 1080

ctgctggaat ttgttaccgc tgctggtatc acccacggta tggacgaact gtacaaa 1137

<210> 6
<211> 864
<212> DNA
<213> Artificial Sequence

<220>
<223> His-tag-TTR-Lysozyme

<400> 6
atgcgtggtt ctcaccacca ccaccaccac ggttctggtc cgaccggtac cggtgaatct 60

aaatgcccgc tgatggttaa agttctggac gctgttcgtg gttctccggc tatcaacgtt 120

gctgttcacg ttttccgtaa agctgctgac gacacctggg aaccgttcgc ttctggtaaa 180

acctctgaat ctggtgaact gcacggtctg accaccgaag aagaatttgt tgaaggtatc 240

tacaaagttg aaatcgacac caaatcttac tggaaagctc tgggtatctc tccgttccac 300

gaacacgctg aagttgtttt caccgctaac gactctggtc cgcgtcgtta caccatcgct 360

gctctgctgt ctccgtactc ttactctacc accgctgttg ttaccaaccc gaaagaaggt 420

tctatgcgtt ctctgctgat cctggttctg tgcttcctgc cgctggctgc tctgggtaaa 480

gttttcggtc gttgcgaact ggctgctgct atgaaacgtc acggtctgga caactaccgt 540

ggttactctc tgggtaactg ggtttgcgct gctaaattcg aatctaactt caacacccag 600

gctaccaacc gtaacaccga cggttctacc gactacggta tcctgcagat caactctcgt 660

tggtggtgca acgacggtcg taccccgggt tctcgtaacc tgtgcaacat cccgtgctct 720

gctctgctgt cttctgacat caccgcttct gttaactgcg ctaaaaaaat cgtttctgac 780

ggtaacggta tgaacgcttg ggttgcttgg cgtaaccgtt gcaaaggtac cgacgttcag 840

gcttggatcc gtggttgccg tctg 864

<210> 7
<211> 1581
<212> DNA
<213> Artificial Sequence

<220>
<223> His-tag-TTR-Ovalbumin

<400> 7
atgcgtggtt ctcaccacca ccaccaccac ggttctggtc cgaccggtac cggtgaatct 60

aaatgcccgc tgatggttaa agttctggac gctgttcgtg gttctccggc tatcaacgtt 120

gctgttcacg ttttccgtaa agctgctgac gacacctggg aaccgttcgc ttctggtaaa 180

acctctgaat ctggtgaact gcacggtctg accaccgaag aagaatttgt tgaaggtatc 240

tacaaagttg aaatcgacac caaatcttac tggaaagctc tgggtatctc tccgttccac 300

gaacacgctg aagttgtttt caccgctaac gactctggtc cgcgtcgtta caccatcgct 360

gctctgctgt ctccgtactc ttactctacc accgctgttg ttaccaaccc gaaagaaggt 420

tctatgggtt ctatcggtgc tgcttctatg gaattttgct tcgacgtttt caaagaactg 480

aaagttcacc acgctaacga aaacatcttc tactgcccga tcgctatcat gtctgctctg 540

gctatggttt acctgggtgc taaagactct acccgtaccc agatcaacaa agttgttcgt 600

ttcgacaaac tgccgggttt cggtgactct atcgaagctc agtgcggtac ctctgttaac 660

gttcactctt ctctgcgtga catcctgaac cagatcacca aaccgaacga cgtttactct 720

ttctctctgg cttctcgtct gtacgctgaa gaacgttacc cgatcctgcc ggaatacctg 780

cagtgcgtta aagaactgta ccgtggtggt ctggaaccga tcaacttcca gaccgctgct 840

gaccaggctc gtgaactgat caactcttgg gttgaatctc agaccaacgg tatcatccgt 900

aacgttctgc agccgtcttc tgttgactct cagaccgcta tggttctggt taacgctatc 960

gttttcaaag gtctgtggga aaaagctttc aaagacgaag acacccaggc tatgccgttc 1020

cgtgttaccg aacaggaatc taaaccggtt cagatgatgt accagatcgg tctgttccgt 1080

gttgcttcta tggcttctga aaaaatgaaa atcctggaac tgccgttcgc ttctggtacc 1140

atgtctatgc tggttctgct gccggacgaa gtttctggtc tggaacagct ggaatctatc 1200

atcaacttcg aaaaactgac cgaatggacc tcttctaacg ttatggaaga acgtaaaatc 1260

aaagtttacc tgccgcgtat gaaaatggaa gaaaaataca acctgacctc tgttctgatg 1320

gctatgggta tcaccgacgt tttctcttct tctgctaacc tgtctggtat ctcttctgct 1380

gaatctctga aaatctctca ggctgttcac gctgctcacg ctgaaatcaa cgaagctggt 1440

cgtgaagttg ttggttctgc tgaagctggt gttgacgctg cttctgtttc tgaagaattt 1500

cgtgctgacc acccgttcct gttctgcatc aaacacatcg ctaccaacgc tgttctgttc 1560

ttcggtcgtt gcgtttctcc g 1581

<210> 8
<211> 4311
<212> DNA
<213> Artificial Sequence

<220>
<223> pET-21aÖÊÁ£ÖØ¹¹°óµÄÐòÁÐ

<400> 8
aattcttaag aaggagatat acatatggga tcccatcatc atcatcatca ttgactgcag 60

ccaagcttct gttttggcgg atgagagaag attttcagcc tgatacagat taaatcagaa 120

cgcagaagcg gtctgataaa acagaatttg cctggcggca gtagcgcggt ggtcccacct 180

gaccccatgc cgaactcaga agtgaaacgc cgtagcgccg atggtagtgt ggggtctccc 240

catgcgagag tagggaactg ccaggcatca aataaaacga aaggctcagt cgaaagactg 300

ggcctttcgt tttatctgtt gtttgtcggt gaacgctctc ctgagtagga caaatccgcc 360

gggagcggat ttgaacgttg cgaagcaacg gcccggaggg tggcgggcag gacgcccgcc 420

ataaactgcc aggcatcaaa ttaagcagaa ggccatcctg acggatggcc tttttgcgtt 480

tctacaaact cttttgttta tttttctaaa tacattcaaa tatgtatccg ctcatgagac 540

aataaccctg ataaatgctt caataatatt gaaaaaggaa gagtatgagt attcaacatt 600

tccgtgtcgc ccttattccc ttttttgcgg cattttgcct tcctgttttt gctcacccag 660

aaacgctggt gaaagtaaaa gatgctgaag atcagttggg tgcacgagtg ggttacatcg 720

aactggatct caacagcggt aagatccttg agagttttcg ccccgaagaa cgttttccaa 780

tgatgagcac ttttaaagtt ctgctatgtg gcgcggtatt atcccgtgtt gacgccgggc 840

aagagcaact cggtcgccgc atacactatt ctcagaatga cttggttgag tactcaccag 900

tcacagaaaa gcatcttacg gatggcatga cagtaagaga attatgcagt gctgccataa 960

ccatgagtga taacactgcg gccaacttac ttctgacaac gatcggagga ccgaaggagc 1020

taaccgcttt tttgcacaac atgggggatc atgtaactcg ccttgatcgt tgggaaccgg 1080

agctgaatga agccatacca aacgacgagc gtgacaccac gatgcctgta gcaatggcaa 1140

caacgttgcg caaactatta actggcgaac tacttactct agcttcccgg caacaattaa 1200

tagactggat ggaggcggat aaagttgcag gaccacttct gcgctcggcc cttccggctg 1260

gctggtttat tgctgataaa tctggagccg gtgagcgtgg gtctcgcggt atcattgcag 1320

cactggggcc agatggtaag ccctcccgta tcgtagttat ctacacgacg gggagtcagg 1380

caactatgga tgaacgaaat agacagatcg ctgagatagg tgcctcactg attaagcatt 1440

ggtaactgtc agaccaagtt tactcatata tactttagat tgatttaaaa cttcattttt 1500

aatttaaaag gatctaggtg aagatccttt ttgataatct catgaccaaa atcccttaac 1560

gtgagttttc gttccactga gcgtcagacc ccgtagaaaa gatcaaagga tcttcttgag 1620

atcctttttt tctgcgcgta atctgctgct tgcaaacaaa aaaaccaccg ctaccagcgg 1680

tggtttgttt gccggatcaa gagctaccaa ctctttttcc gaaggtaact ggcttcagca 1740

gagcgcagat accaaatact gtccttctag tgtagccgta gttaggccac cacttcaaga 1800

actctgtagc accgcctaca tacctcgctc tgctaatcct gttaccagtg gctgctgcca 1860

gtggcgataa gtcgtgtctt accgggttgg actcaagacg atagttaccg gataaggcgc 1920

agcggtcggg ctgaacgggg ggttcgtgca cacagcccag cttggagcga acgacctaca 1980

ccgaactgag atacctacag cgtgagctat gagaaagcgc cacgcttccc gaagggagaa 2040

aggcggacag gtatccggta agcggcaggg tcggaacagg agagcgcacg agggagcttc 2100

cagggggaaa cgcctggtat ctttatagtc ctgtcgggtt tcgccacctc tgacttgagc 2160

gtcgattttt gtgatgctcg tcaggggggc ggagcctatg gaaaaacgcc agcaacgcgg 2220

cctttttacg gttcctggcc ttttgctggc cttttgctca catgttcttt cctgcgttat 2280

cccctgattc tgtggataac cgtattaccg cctttgagtg agctgatacc gctcgccgca 2340

gccgaacgac cgagcgcagc gagtcagtga gcgaggaagc ggaagagcgc ctgatgcggt 2400

attttctcct tacgcatctg tgcggtattt cacaccgcat atatggtgca ctctcagtac 2460

aatctgctct gatgccgcat agttaagcca gtatacactc cgctatcgct acgtgactgg 2520

gtcatggctg cgccccgaca cccgccaaca cccgctgacg cgccctgacg ggcttgtctg 2580

ctcccggcat ccgcttacag acaagctgtg accgtctccg ggagctgcat gtgtcagagg 2640

ttttcaccgt catcaccgaa acgcgcgagg cagctgcggt aaagctcatc agcgtggtcg 2700

tgaagcgatt cacagatgtc tgcctgttca tccgcgtcca gctcgttgag tttctccaga 2760

agcgttaatg tctggcttct gataaagcgg gccatgttaa gggcggtttt ttcctgtttg 2820

gtcacttgat gcctccgtgt aagggggaat ttctgttcat gggggtaatg ataccgatga 2880

aacgagagag gatgctcacg atacgggtta ctgatgatga acatgcccgg ttactggaac 2940

gttgtgaggg taaacaactg gcggtatgga tgcggcggga ccagagaaaa atcactcagg 3000

gtcaatgcca gcgcttcgtt aatacagatg taggtgttcc acagggtagc cagcagcatc 3060

ctgcgatgca gatccggaac ataatggtgc agggcgctga cttccgcgtt tccagacttt 3120

acgaaacacg gaaaccgaag accattcatg ttgttgctca ggtcgcagac gttttgcagc 3180

agcagtcgct tcacgttcgc tcgcgtatcg gtgattcatt ctgctaacca gtaaggcaac 3240

cccgccagcc tagccgggtc ctcaacgaca ggagcacgat catgcgcacc cgtggccagg 3300

acccaacgct gcccgagatg cgccgcgtgc ggctgctgga gatggcggac gcgatggata 3360

tgttctgcca agggttggtt tgcgcattca cagttctccg caagaattga ttggctccaa 3420

ttcttggagt ggtgaatccg ttagcgaggt gccgccggct tccattcagg tcgaggtggc 3480

ccggctccat gcaccgcgac gcaacgcggg gaggcagaca aggtataggg cggcgcctac 3540

aatccatgcc aacccgttcc atgtgctcgc cgaggcggca taaatcgccg tgacgatcag 3600

cggtccagtg atcgaagtta ggctggtaag agccgcgagc gatccttgaa gctgtccctg 3660

atggtcgtca tctacctgcc tggacagcat ggcctgcaac gcgggcatcc cgatgccgcc 3720

ggaagcgaga agaatcataa tggggaaggc catccagcct cgcgtcgcga acgccagcaa 3780

gacgtagccc agcgcgtcgg ccgccatgcc ggcgataatg gcctgcttct cgccgaaacg 3840

tttggtggcg ggaccagtga cgaaggcttg agcgagggcg tgcaagattc cgaataccgc     3900

aagcgacagg ccgatcatcg tcgcgctcca gcgaaagcgg tcctcgccga aaatgaccca     3960

gagcgctgcc ggcacctgtc ctacgagttg catgataaag aagacagtca taagtgcggc     4020

gacgatagtc atgccccgcg cccaccggaa ggagctgact gggttgaagg ctctcaaggg     4080

catcggtcga cgctctccct tatgcgactc ctgcattagg aagcagccca gtagtaggtt     4140

gaggccgttg agcaccgccg ccgcaaggaa tggtgcatgc atcgatcacc acaattcagc     4200

aaattgtgaa catcatcacg ttcatctttc cctggttgcc aatggcccat tttcctgtca     4260

gtaacgagaa ggtcgcgaat tcaggcgctt tttagactgg tcgtaatgaa c              4311

<210>  9
<211>  127
<212>  PRT
<213>  Rattus norvegicus

<400>  9

Gly Pro Gly Gly Ala Gly Glu Ser Lys Cys Pro Leu Met Val Lys Val
1               5                   10                  15

Leu Asp Ala Val Arg Gly Ser Pro Ala Val Asp Val Ala Val Lys Val
            20                  25                  30

Phe Lys Lys Thr Ala Asp Gly Ser Trp Glu Pro Phe Ala Ser Gly Lys
        35                  40                  45

Thr Ala Glu Ser Gly Glu Leu His Gly Leu Thr Thr Asp Glu Lys Phe
    50                  55                  60

Thr Glu Gly Val Tyr Arg Val Glu Leu Asp Thr Lys Ser Tyr Trp Lys
65                  70                  75                  80

Ala Leu Gly Ile Ser Pro Phe His Glu Tyr Ala Glu Val Val Phe Thr
                85                  90                  95

Ala Asn Asp Ser Gly His Arg His Tyr Thr Ile Ala Ala Leu Leu Ser
            100                 105                 110

Pro Tyr Ser Tyr Ser Thr Thr Ala Val Val Ser Asn Pro Gln Asn
        115                 120                 125

<210>  10
<211>  127
<212>  PRT
<213>  Mus musculus

<400>  10

Gly Pro Ala Gly Ala Gly Glu Ser Lys Cys Pro Leu Met Val Lys Val
1               5                   10                  15

Leu Asp Ala Val Arg Gly Ser Pro Ala Val Asp Val Ala Val Lys Val
            20                  25                  30

Phe Lys Lys Thr Ser Glu Gly Ser Trp Glu Pro Phe Ala Ser Gly Lys
        35                  40                  45

Thr Ala Glu Ser Gly Glu Leu His Gly Leu Thr Thr Asp Glu Lys Phe
    50                  55                  60

Val Glu Gly Val Tyr Arg Val Glu Leu Asp Thr Lys Ser Tyr Trp Lys
65                  70                  75                  80

Thr Leu Gly Ile Ser Pro Phe His Glu Phe Ala Asp Val Val Phe Thr
                85                  90                  95

Ala Asn Asp Ser Gly His Arg His Tyr Thr Ile Ala Ala Leu Leu Ser
            100                 105                 110

Pro Tyr Ser Tyr Ser Thr Thr Ala Val Val Ser Asn Pro Gln Asn
        115                 120                 125

<210>  11
<211>  122
<212>  PRT
<213>  Artificial Sequence

<220>
<223>  ÈËÔ´TTR-CL

<400>  11

Gly Pro Thr Gly Thr Gly Glu Ser Lys Cys Pro Leu Met Val Lys Val
1               5                   10                  15

Leu Asp Ala Val Arg Gly Ser Pro Ala Ile Asn Val Ala Val His Val
            20                  25                  30

Phe Arg Lys Ala Ala Asp Asp Thr Trp Glu Pro Phe Ala Ser Gly Lys
        35                  40                  45

Thr Ser Glu Ser Gly Glu Leu His Gly Leu Thr Thr Glu Glu Glu Phe
    50                  55                  60

Val Glu Gly Ile Tyr Lys Val Glu Ile Asp Thr Lys Ser Tyr Trp Lys
65                  70                  75                  80

Ala Leu Gly Ile Ser Pro Phe His Glu His Ala Glu Val Val Phe Thr
                85                  90                  95

Ala Asn Asp Ser Gly Pro Arg Arg Tyr Thr Ile Ala Ala Leu Leu Ser
            100                 105                 110

Pro Tyr Ser Tyr Ser Thr Thr Ala Val Val
        115                 120

## Description

The present disclosure claims the priority of the Chinese patent application 201911302937.3 filed on December 17, 2019, the Chinese patent application 202010506950.7 filed on June 5, 2020, the Chinese patent application 202010976582.2 filed on September 16, 2020, and the Chinese patent application 202010974997.6 filed on September 16, 2020, the contents of which are incorporated herein by their entireties.

### Technical Field

The present invention relates to the field of pharmaceutical technology, and particularly relates to a use of transthyretin serving as a carrier for a protein and/or polypeptide medicament entering an eye through an ocular barrier. The present invention also relates to a use of transthyretin, and/or a fusion protein comprising transthyretin and a protein and/or polypeptide medicament in preparing a drops; and further relates to an ocular preparation containing transthyretin, a method for preparing the preparation thereof, and a use thereof in treating ocular diseases associated with ocular angiogenesis and/or ocular retina leakage, etc.

### Background

Diabetic Retinopathy, abbreviated as "DR", is a clinical manifestation of diabetic microangiopathy and one of the most serious complications of diabetes, which has become one of the major blinding eye diseases. The main clinical symptoms are microvascular obstruction, microangioma, hemorrhage, venous dilatation, macular edema, angiogenesis, massive vitreous hemorrhage, intraocular fibrosis, retinal detachment, etc., occurring gradually in the microenvironment of long-term hyperglycemia and hypoxia in the eye.

Age-related macular degeneration (AMD) is an aging change in the structure of the macular. The main manifestation is a decrease in the ability of retinal pigment epithelial cells to phagocytically digest the disc membrane of the out segment of the optic cells, resulting in the retention of incompletely digested disc membrane remnants in the basal cell protoplasm and their excretion extracellularly to the Bruch membrane, where they are deposited and formed drusen. Due to the particularity of macular structure and function, this kind of change is more obvious. Drusen can also be seen in the elderly with normal vision, but a variety of secondary pathological changes lead to macular degeneration. Or rupture of Bruch membrane was caused, and choroidal capillaries entered RPE (retinal pigment epithelium) and subepithelial retinal nerve through the ruptured Bruch membrane, forming choroidal neovascularization, i.e., subretinal neovascularization. The structural abnormalities of the neovascularization wall leads to vascular leakage and hemorrhage, which in turn trigger a series of secondary pathological changes. Age-related macular degeneration occurs mostly at the age of 45 or above, and its prevalence increases with age, making it a main disease that currently causes blindness in the elderly.

For premature infants under 36 weeks of gestation with low birth weight and prolonged oxygen inhalation, the unvascularized retina suffers from fibroangioma hyperplasia and contraction, further causing traction retinal detachment and blindness, which is called retinopathy of prematurity (ROP). It has previously been known as Terry's syndrome or retrolental fibroplasia, but the latter only reflects the advanced manifestations of the disease. The incidence of ROP can reach 60%-80% in patients with shorter pregnancy or lower birth weight. The etiology is due to excessive oxygen uptake of premature infants after birth in a hyperoxia environment inside the incubator, and when the premature infants is in a relatively hypoxic state after being removed from the incubator, the incompletely vascularized retina is thus vasoconstricted and a vascular proliferation is occurred due to oxygen.

For the treatment of ocular diseases, especially the aforementioned ocular diseases such as diabetic retinopathy, age-related macular degeneration, retinopathy of prematurity, and other ocular diseases such as retinitis pigmentosa and neurological changes caused by glaucoma, effective drug delivery into the eye is needed. However, effective drug delivery into the eye is challenging due to the presence of ocular multi-level barriers. As a result, ophthalmic drug delivery systems have recently received widespread attention.

According to the ocular anatomy, the ocular barrier mainly includes tear barrier, corneal/conjunctival barrier, and blood-ocular barrier. While protecting the eyes, multi-level barriers can effectively block the invasion of exogenous chemical and biological molecules, but also brings great obstacles to the delivery of drugs. At present, the common ocular drug administration methods mainly include conventional eye drops, subconjunctival injection, scleral administration and intravitreal injection. Among them, conventional eye drops has a short contact time with the ocular surface, and some exogenous drugs and proteins are difficult to effectively enter the eye due to its structure and characteristics, while subconjunctival injection, scleral administration and intravitreal injection usually result in a certain degree of trauma. Retinal laser treatment, vitrectomy combined with silicone oil tamponade can cause great damages and vision recovery is not ideal.

The most conventional treatment for DR, AMD and ROP is currently intravitreal injection of anti-vegf antibodies, but as described above, this is usually results in a certain degree of trauma, and multiple dosing must be performed with a long interval.

At present, there are also some patents which design functional small peptides that can effectively break through various ocular barriers to reach the eye, such as the patents of Zheng Ying, Xu Xun et al. (A novel small peptide that inhibits angiogenesis and application thereof, CN201310052714.2), Su Li et al. (A small peptide that inhibits angiogenesis and application thereof, CN201310058978.9), and Yang Xiaolu et al. (A small molecule polypeptide that prevents and/or treats inflammatory reaction and application thereof, CN201210581759.4).

All the above studies have certain limitations. Intraocular injection may cause certain trauma, and the functional small peptides have low delivery efficiency and short half-life. Therefore, it is necessary to find safer, more stable and effective drugs and drug administration methods that are less or no harmful to the eyes.

### Content of the present invention

In order to overcome the shortcomings, including the absence of a medicament and an administering method that can deliver the medicament into an eye in a safe, stable, effective manner with no or less damage to the eye for treating ocular diseases such as diabetic retinopathy (DR), age-related macular degeneration (AMD) and retinopathy of prematurity (ROP) in the prior art due to the presence of an ocular barrier, the present disclosure provides a use of transthyretin (TTR) serving as a carrier for a protein and/or polypeptide medicament entering an eye through an ocular barrier, and a use of transthyretin, and/or a fusion protein comprising transthyretin and a protein and/or polypeptide medicament in the preparation of a drops, and a drops.

Through extensive experiments, the present inventors unexpectedly discovered that transthyretin can effectively enter the vitreous body and fundus oculi through the corneal barrier and also effectively deliver exogenous protein and/or polypeptide in the manner of eye dropping by using a drops rather than the conventional injection in the art. Therefore, a suitable protein and/or polypeptide medicament can be chosen to be delivered to the eye via transthyretin, depending on the pathological characteristics of ocular diseases, so as to treat ocular diseases. Drops have promising prospects in the pharmaceutical field in terms of application as an alternative to injectable drugs.

Through extensive experiments, the present inventors also unexpectedly discovered that after entering the vitreous body and fundus oculi through the corneal barrier, transthyretin can inhibit eyeball retinal leakage and reduce the number of retinal neovascularization significantly, thus effectively alleviating ocular diseases such as DR, AMD and ROP. That is, without fusing with a protein and/or polypeptide medicament, transthyretin itself can treat or alleviate ocular diseases such as DR, AMD and ROP with sufficient therapeutic concentration and therapeutic duration (i.e., very long half-life).

Moreover, a person skilled in the art only knows that hyaluronic acid has an effect of moisturizing, but does not know it can increase the permeation of transthyretin. The present inventors further discovered that in the preparation of a drops such as eyedrops, hyaluronic acid can increase the permeation of transthyretin in vitreous cavity and fundus oculi, thereby increasing the concentration of transthyretin effectively.

To address the above-mentioned technical problems, the first aspect of the present disclosure provides a use of transthyretin serving as a carrier for a protein and/or polypeptide medicament entering an eye through an ocular barrier, wherein the transthyretin is represented by (a), (b) or (c):
(a) a protein consisting of an amino acid sequence of SEQ ID NO: 1;
(b) a protein derived from (a) with an inhibitory function of neovascularization, which is shown by a sequence in which one or more amino acids are substituted, deleted or added in the amino acid sequence of (a);
(c) a protein having a sequence with a hydrophilic modification or hydrophobic modification in the amino acid sequence of (a) or (b).

Preferably, in (b), the protein derived from (a) is a protein in which 22 amino acids are substituted in the amino acid sequence of (a).

Preferably, in (b), the protein derived from (a) is a protein in which 25 amino acids are substituted in the amino acid sequence of (a).

Preferably, in (b), the protein derived from (a) is a protein in which T3, T5, 126, N27, H31, R34, A36, A37, D38, D39, T40, S50, E61, E63, V65, 168, K70, 173, A81, H90, E92, P102, R104, T123, K126 and/or E127 are substituted in the amino acid sequence of (a). More preferably, in (b), the protein derived from (a) is a protein in which T3G, T5A, I26V, N27D, H31K, R34K, A36T, D39G, T40S, S50A, E61D, E63K, V65T, I68V, K70R, I73L, H90Y, P102H, R104H, T123S, K126Q and E127N are substituted in the amino acid sequence of (a). More preferably, in (b), the protein derived from (a) is a protein in which T3G, T5A, I26V, N27D, H31K, R34K, A36T, A37S, D38E, D39G, T40S, S50A, E61D, E63K, I68V, K70R, I73L, A81T, H90F, E92D, P102H, R104H, T123S, K126Q and E127N are substituted in the amino acid sequence of (a).

Preferably, in (b), the protein derived from (a) is a protein in which 5 amino acids are deleted from the amino acid sequence of (a). More preferably, in (b), the protein derived from (a) is a protein in which a deletion occurs at positions 123-127 of the amino acid sequence of (a).

Preferably, in (b), the protein derived from (a) has an amino acid sequence of SEQ ID NO: 9, SEQ ID NO: 10 or SEQ ID NO: 11.

Preferably, in (c), the hydrophilic modification or hydrophobic modification occurs at the cysteine at position 10 of the amino acid sequence of (a). Preferably, the hydrophobic modification is a modification with a long chain hydrophobic fragment such as *n*-dodecane. In one preferred embodiment of the present disclosure, the protein is a protein in which the cysteine at position 10 of the amino acid sequence of (a) is modified with *n*-dodecane via maleiamide. In the present disclosure, the cysteine at position 10 of the amino acid sequence of (a) can also be linked with a fluorescent label such as 5-aminofluorescein, to facilitate tracking the modified protein.

Preferably, the transthyretin is expressed with the protein and/or polypeptide medicament as a fusion; the fusion is preferably a fusion that the protein and/or polypeptide medicament are fused at the N-terminus or C-terminus of the transthyretin.

Preferably, the transthyretin is expressed with the protein and/or polypeptide medicament as a fusion in a microbial cell, followed by a purification of the fusion. The microbial cell can be an *E. coli,* and the *E coli* includes, but is not limited to, *E*. *coli* BL21, *E. coli* BL21 (DE3), *E. coli* JM109, *E. coli* DH5α, *E. coli* K12 *or E. coli* TOP10. The purification is preferably to remove endotoxin by an endotoxin absorption column (e.g. using Pierce^{™} High Capacity Endotoxin Removal Spin Columns, ThermoFisher) and then remove residue bacteria by a filter membrane with a pore size of 0.22 µm.

Preferably, the protein and/or polypeptide medicament include, but are not limited to, lysozyme, albumin and/or EGFR antibody, with a molecular weight of no more than 45 kDa. Wherein, the lysozyme is preferably hen egg white lysozyme with GenBank Accession No.: AAL69327.1. Wherein, the albumin is preferably ovalbumin.

Preferably, the protein and/or polypeptide medicament comprises a protein and/or polypeptide medicament for treating ocular diseases associated with ocular retina leakage and/or retinal neovascularization such as diabetic retinopathy, age-related macular degeneration and/or retinopathy of prematurity.

Preferably, the transthyretin is encoded by a nucleotide sequence of SEQ ID NO: 2.

Preferably, the transthyretin is expressed by a recombinant expression vector, wherein the recombinant expression vector has a plasmid backbone comprising a rhamnose inducible promoter, preferably a rhaPBAD promoter.

Preferably, the transthyretin is expressed by a recombinant expression vector, the recombinant expression vector has a plasmid backbone of pET-21a or a vector having 25% or more homology therewith, and the vector having 25% or more homology therewith preferably has a sequence of SEQ ID NO: 8.

Preferably, the recombinant plasmid expressing transthyretin has a nucleotide sequence of SEQ ID NO: 3.

Preferably, the transthyretin is expressed in a microbial cell (in the form of a transformant), and can further followed by a purification of the transthyretin; the microbial cell can be an *E. coli,* and the *E coli* includes, but is not limited to, *E. coli* BL21, *E. coli* BL21 (DE3), *E. coli* JM109, *E. coli* DH5α, *E. coli* K12 or *E. coli* TOP10. The purification can be removing endotoxin by an endotoxin absorption column (e.g. using Pierce^{™} High Capacity Endotoxin Removal Spin Columns, ThermoFisher) and removing residue bacteria by a filter membrane with a pore size of 0.22 µm.

Preferably, the transthyretin is expressed by culturing the transformant comprising a gene of the transthyretin until the bacteria obtained reaches an OD₆₀₀ of 1.5-2.0 (such as 1.6, 1.7, 1.8 or 1.9).

Preferably, expression of the transthyretin is induced by a reagent for inducible expression, wherein the reagent for inducible expression has a mass volume percentage of 0.1-2%, such as 0.2%, 0.3%, 0.4%, 0.5%, 0.7%, 0.8%, 1.2% or 1.6%, while the expression is preferably induced for a period of 8-20 h, such as 10 h, 12 h, 14 h, 16 h, 17 h, 18 h or 19 h; wherein the reagent for inducible expression is preferably rhamnose or IPTG.

In one preferred embodiment of the present disclosure, the use is a use that administering the fusion protein, which is obtained by expressing the transthyretin and the protein and/or polypeptide medicament as a fusion, to the eye in the manner of eye-dropping (e.g., prepared in the form of drops, such as eye drops), at a dose of 0.3-0.8 nmol protein per eye, 1-3 times per day. The drops can be administered by twice per day, one drop each time, for 3 months. The drops can be administered by once per day, one drop each time, for 5 days. The drops can be administered by twice per day, one drop each time, for 2 weeks.

To address the above-mentioned technical problems, the second aspect of the present disclosure provides a use of transthyretin and/or a fusion protein consisting of a transthyretin and a medicament in the preparation of drops, wherein the medicament is a protein and/or polypeptide medicament, and the transthyretin is represented by (a), (b) or (c):
(a) a protein consisting of an amino acid sequence of SEQ ID NO: 1;
(b) a protein derived from (a) with an inhibitory function of neovascularization, which is shown by a sequence in which one or more amino acids are substituted, deleted or added in the amino acid sequence of (a);
(c) a protein having a sequence with a hydrophilic modification or hydrophobic modification in the amino acid sequence of (a) or (b).

Preferably, in (b), the protein derived from (a) is a protein in which 22 amino acids are substituted in the amino acid sequence of (a).

Preferably, in (b), the protein derived from (a) is a protein in which 25 amino acids are substituted in the amino acid sequence of (a).

Preferably, in (b), the protein derived from (a) is a protein in which T3, T5, 126, N27, H31, R34, A36, A37, D38, D39, T40, S50, E61, E63, V65, 168, K70, 173, A81, H90, E92, P102, R104, T123, K126 and/or E127 are substituted in the amino acid sequence of (a). More preferably, in (b), the protein derived from (a) is a protein in which T3G, T5A, I26V, N27D, H31K, R34K, A36T, D39G, T40S, S50A, E61D, E63K, V65T, I68V, K70R, I73L, H90Y, P102H, R104H, T123S, K126Q and E127N are substituted in the amino acid sequence of (a). More preferably, in (b), the protein derived from (a) is a protein in which T3G, T5A, I26V, N27D, H31K, R34K, A36T, A37S, D38E, D39G, T40S, S50A, E61D, E63K, I68V, K70R, I73L, A81T, H90F, E92D, P102H, R104H, T123S, K126Q and E127N are substituted in the amino acid sequence of (a).

Preferably, in (b), the protein derived from (a) is a protein in which 5 amino acids are deleted from the amino acid sequence of (a). More preferably, in (b), the protein derived from (a) is a protein in which a deletion occurs at positions 123-127 of the amino acid sequence of (a).

Preferably, in (b), the protein derived from (a) has an amino acid sequence of SEQ ID NO: 9, SEQ ID NO: 10 or SEQ ID NO: 11.

Preferably, in (c), the hydrophilic modification or hydrophobic modification occurs at the cysteine at position 10 of the amino acid sequence of (a). Preferably, the hydrophobic modification is a modification with a long chain hydrophobic fragment such as *n*-dodecane. In one preferred embodiment of the present disclosure, the protein is a protein in which the cysteine at position 10 of the amino acid sequence of (a) is modified with *n*-dodecane via maleiamide. In the present disclosure, the cysteine at position 10 of the amino acid sequence of (a) can also be linked with a fluorescent label such as 5-aminofluorescein, to facilitate tracking the modified protein.

Preferably, the fusion protein has a content of 4-30 µmol/L, preferably 10-15 µmol/L.

Preferably, the transthyretin has a content of 4-30 µmol/L, preferably 5-30 µmol/L, more preferably 10-20 µmol/L, such as 10, 15, 20 µmol/L.

Preferably, the drops further comprises saline. In the present disclosure, the consumption of the saline can be determined in accordance with the standard established in the art.

Preferably, the drops further comprises a surfactant, wherein the surfactant, for example, Tween 80, preferably has a content of 5% (v/v).

Preferably, the drops further comprises hyaluronic acid, and the hyaluronic acid has a content of mass volume percentage of no more than 6%, preferably 1-4%, more preferably 2%.

Preferably, the drops is preferably an eye drops.

Preferably, the drops is a drops that inhibits ocular retina leakage and/or reduces the number of retinal neovascularization; preferably a drops that treats diabetic retinopathy, age-related macular degeneration and/or retinopathy of prematurity.

Preferably, the drops is administered 1-3 times per day, preferably at an amount of 0.3-0.8 nmol protein per eye at each time.

Preferably, the drops is administered twice per day, one drop each time, for 3 months; and/or, the drops is administered once per day, one drop each time, for 5 days; and/or, the drops is administered twice per day, one drop each time, for 2 weeks.

Preferably, the transthyretin is encoded by a nucleotide sequence of SEQ ID NO: 2.

Preferably, the transthyretin is expressed by a recombinant expression vector, wherein the recombinant expression vector has a plasmid backbone comprising a rhamnose inducible promoter, preferably a rhaPBAD promoter.

Preferably, the transthyretin is expressed by a recombinant expression vector, the recombinant expression vector has a plasmid backbone of pET-21a or a vector with 25% or more homology therewith, and preferably the vector with 25% or more homology therewith has a sequence of SEQ ID NO: 8.

Preferably, the recombinant plasmid expressing transthyretin is encoded by a nucleotide sequence of SEQ ID NO: 3.

Preferably, the transthyretin is expressed in a microbial cell (in the form of a transformant), and can further followed by a purification of the transthyretin; the microbial cell can be an *E. coli,* and the *E coli* includes, but is not limited to, *E. coli* BL21, *E. coli* BL21 (DE3), *E. coli* JM109, *E. coli* DH5α, *E. coli* K12 or *E. coli* TOP10. The purification can be removing endotoxin by an endotoxin absorption column (e.g. using Pierce^{™} High Capacity Endotoxin Removal Spin Columns, ThermoFisher) and removing residue bacteria by a filter membrane with a pore size of 0.22 µm.

Preferably, the transthyretin is expressed by culturing the transformant comprising a gene of the transthyretin until the bacteria obtained reaches an OD₆₀₀ of 1.5-2.0 (such as 1.6, 1.7, 1.8 or 1.9).

Preferably, expression of the transthyretin is induced by a reagent for inducible expression, wherein the reagent for inducible expression has a mass volume percentage of 0.1-2%, such as 0.2%, 0.3%, 0.4%, 0.5%, 0.7%, 0.8%, 1.2% or 1.6%, while the expression is preferably induced for a period of 8-20 h, such as 10 h, 12 h, 14 h, 16 h, 17 h, 18 h or 19 h; wherein the reagent for inducible expression is preferably rhamnose or IPTG.

Preferably, the fusion protein has a sequence of SEQ ID NO: 6 or SEQ ID NO: 7.

Preferably, the transthyretin is expressed with the protein and/or polypeptide medicament as a fusion; the fusion is preferably a fusion that the protein and/or polypeptide medicament is fused at the N-terminus or C-terminus of the transthyretin; wherein the transthyretin is expressed with the protein and/or polypeptide medicament as a fusion in a microbial cell, followed by a purification of the fusion. The microbial cell can be an *E. coli,* and the *E coli* includes, but is not limited to, *E. coli* BL21, *E*. *coli* BL21 (DE3), *E. coli* JM109, *E. coli* DH5α, *E. coli* K12 or *E. coli* TOP10. The purification is preferably removing endotoxin by an endotoxin absorption column and then removing residue bacteria by a filter membrane with a pore size of 0.22 µm.

Preferably, the protein and/or polypeptide medicament include, but are not limited to, lysozyme, albumin and/or EGFR antibody, with a molecular weight of no more than 45 kDa. Wherein, the lysozyme is preferably hen egg white lysozyme with GenBank Accession No.: AAL69327.1. Wherein, the albumin is preferably ovalbumin.

Preferably, the protein and/or polypeptide medicament comprises a protein and/or polypeptide medicament for treating ocular diseases associated with ocular retina leakage and/or retinal neovascularization such as diabetic retinopathy, age-related macular degeneration and/or retinopathy of prematurity.

The present inventors also unexpectedly discovered during the experiment that materials, for example, carboxymethyl cellulose or salts thereof such as sodium carboxymethyl cellulose, chondroitin sulfate or salts thereof such as chondroitin sulfate A sodium salt, dextran such as dextran 70, hyaluronic acid, can mutual check and synergistically cooperate with transthyretin and/or the fusion protein to form an organic whole, thus allowing for the synergistic treatment of ocular diseases associated with ocular neovascularization and/or ocular retinal leakage, such as diabetic retinopathy, age-related macular degeneration and/or retinopathy of prematurity, ultimately achieving a better therapeutic effect. However, a person skilled in the art only knows that materials, for example, hyaluronic acid, carboxymethyl cellulose or salts thereof such as sodium carboxymethyl cellulose, chondroitin sulfate or salts thereof such as chondroitin sulfate A sodium salt, dextran such as dextran 70 or hyaluronic acid has effects such as moisturizing, which generally can be used to prepare an eye drops to effectively relieve eye dryness, etc.

Based on this, the drops described above can further comprise a pharmaceutically acceptable excipient, wherein the pharmaceutically acceptable excipient is one or more selected from "carboxymethyl cellulose or salts thereof, solvates thereof, solvates of pharmaceutically acceptable salts thereof, or, crystal forms thereof', "chondroitin sulfate or salts thereof, solvates thereof, solvates of pharmaceutically acceptable salts thereof, or, crystal forms thereof', "dextran or salts thereof, solvates thereof, solvates of pharmaceutically acceptable salts thereof, or, crystal forms thereof' and "hyaluronic acid or salts thereof, solvates thereof, solvates of pharmaceutically acceptable salts thereof, or, crystal forms thereof'.

Preferably, the carboxymethyl cellulose or salts thereof, solvates thereof, solvates of pharmaceutically acceptable salts thereof, or, crystal forms thereof has a viscosity of 800-1200 CP.

Preferably, the chondroitin sulfate is chondroitin sulfate A.

Preferably, the dextran is dextran 70. The dextran 70 generally has a molecular weight in the range of 64000-76000.

Preferably, the hyaluronic acid has a molecular weight of 10000-500000.

In the present disclosure, the "pharmaceutically acceptable" generally refers to non-toxic, safe, and suitable for patient use. The "patient" is preferably a mammal, more preferably a human.

In the present disclosure, the "salts" in the "or salts thereof" is usually a pharmaceutically acceptable salt, which usually refers to the salt prepared by a compound in the present disclosure with a relatively non-toxic, pharmaceutically acceptable acid or base. When the compound in the present disclosure comprises a relatively acidic functional group, the base addition salt can be obtained by contacting the prototype of the compound with a sufficient amount of a pharmaceutically acceptable base in a pure solution or a suitable inert solvent. The pharmaceutically acceptable base addition salts include, but are not limited to, lithium salt, sodium salt, potassium salt, calcium salt, aluminum salt, magnesium salt, zinc salt, bismuth salt, ammonium salt, and diethanolamine salt. When the compound in the present disclosure contains a relatively basic functional group, the acid addition salt can be obtained by contacting the prototype of the compound with a sufficient amount of a pharmaceutically acceptable acid in a pure solution or a suitable inert solvent. The pharmaceutically acceptable acids comprise inorganic acids, wherein the inorganic acids include, but are not limited to, hydrochloric acid, hydrobromic acid, hydroiodic acid, nitric acid, carbonic acid, phosphoric acid, phosphorous acid, sulfuric acid, and the like. The pharmaceutically acceptable acids comprise organic acids, wherein the organic acids include, but are not limited to, acetic acid, propionic acid, oxalic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, salicylic acid, tartaric acid, methanesulfonic acid, isonicotinic acid, acid citric acid, oleic acid , tannic acid, pantothenic acid, hydrogen tartrate, ascorbic acid, gentisic acid, fumaric acid, gluconic acid, saccharic acid, formic acid, ethanesulfonic acid, pamoic acid (i.e., 4, 4'-methylene-bis( 3-hydroxy-2-naphthoic acid)), amino acids (such as glutamic acid or arginine), etc. When the compound in the present disclosure contains relatively acidic and relatively basic functional groups, it can be converted into a base addition salt or an acid addition salt. The details can be found in Berge et al., "Pharmaceutical Salts", Journal of Pharmaceutical Science 66: 1-19 (1977), or, Handbook of Pharmaceutical Salts: Properties, Selection, and Use (P. Heinrich Stahl and Camille G. Wermuth, ed., Wiley-VCH, 2002).

In one preferred embodiment, the term "salts" described in the "carboxymethyl cellulose or salts thereof' can be sodium salts or calcium salts, such as sodium carboxymethyl cellulose.

In one preferred embodiment, the term "salts" described in the "chondroitin sulfate or salts thereof' can be sodium salts or calcium salts, such as chondroitin sulfate A sodium salt.

In the present disclosure, the term "solvates" refers to a substance formed by combining the compound in the present disclosure with a stoichiometric or non-stoichiometric solvent. The solvent molecules in the solvate can be present in an ordered or non-ordered arrangement. The solvents include, but are not limited to, water, methanol, ethanol and the like.

In the present disclosure, the term "pharmaceutically acceptable salts" and "solvate" in the "solvate of a pharmaceutically acceptable salt" are as described above. The term "pharmaceutically acceptable salts" refers to a substance formed by combining the compound 1 of the present disclosure, 2 prepared by the relatively non-toxic, pharmaceutically acceptable acidic or basic preparation, with a stoichiometric or non-stoichiometric solvents. The "solvate of a pharmaceutically acceptable salt" includes, but is not limited to, hydrochloric acid monohydrate of the excipient described in the present disclosure.

In the present disclosure, the term "excipient", "pharmaceutically acceptable salts", "solvate" and "solvate of pharmaceutically acceptable salts", as well as "compound" and "glycoside" described below, can be present in a crystal form or an amorphous form. The term "crystal form" means that the ions or molecules therein are arranged strictly and periodically in a three-dimensional space in a certain way, and have a pattern of periodic recurrence at a regular intervals; depending on the periodic arrangement described above, multiple crystal forms may exist, i.e., polymorphism. The term "amorphous" refers to a haphazard distribution of ions or molecules therein, i.e., there is no periodic arrangement between ions and molecules.

Preferably, in the drops, the carboxymethyl cellulose or salts thereof, solvates thereof, solvates of pharmaceutically acceptable salts thereof, or, crystal forms thereof has a concentration of 0-8 mg/mL except 0 (i.e., 0<concentration≤8 mg/mL), preferably 2, 4, 6 or 8 mg/mL.

Preferably, in the drops, the chondroitin sulfate or salts thereof, solvates thereof, solvates of pharmaceutically acceptable salts thereof, or, crystal forms thereof has a concentration of 0-40 mg/mL except 0 (i.e., 0<concentration≤40 mg/mL), preferably 10, 20, 30 or 40 mg/mL.

Preferably, in the drops, the dextran or salts thereof, solvates thereof, solvates of pharmaceutically acceptable salts thereof, or, crystal forms thereof has a concentration of 0-0.8 mg/mL except 0 (i.e., 0<concentration≤0.8 mg/mL), more preferably 0.2, 0.4, 0.6 or 0.8 mg/mL.

Preferably, in the drops, the hyaluronic acid or salts thereof, solvates thereof, solvates of pharmaceutically acceptable salts thereof, or, crystal forms thereof has a content of no more than 6% by mass volume percentage, preferably 1-4%, more preferably 2%.

In one preferred embodiment, the drops consists of 10 µmol/L transthyretin, 6 mg/mL carboxymethyl cellulose sodium and saline.

In one preferred embodiment, the drops consists of 10 µmol/L transthyretin, 0.4 mg/mL dextran 70 and saline.

In one preferred embodiment, the drops consists of 10 µmol/L transthyretin, 20 mg/mL chondroitin sulfate A sodium salt and saline.

In one preferred embodiment, the drops consists of 10 µmol/L transthyretin, 2% hyaluronic acid and saline.

When the drops comprises the pharmaceutically acceptable excipient described above, the method for preparing the drops comprises mixing the pharmaceutically acceptable excipient and the transthyretin and/or the fusion protein.

The present inventors unexpectedly discovered during the experiment that when co-administering the transthyretin and/or the fusion protein with a specific compound to the eye, the compound is able to synergistically cooperate and co-effect with the transthyretin and/or the fusion protein, which can treat or relieve ocular diseases associated with ocular retinal neovascularization and/or ocular retinal leakage effectively, making the treatment more effective.

Based on this, the drops described above can further comprise a compound, pharmaceutically acceptable salts thereof, glycoside thereof, solvates thereof, solvates of a pharmaceutically acceptable salt thereof, or crystal form thereof; the compound is one or more selected from diclofenac, vitamin A and luteolin.

Wherein, diclofenac and sodium salts thereof: diclofenac and sodium salts thereof belong to non-steroidal anti-inflammatory drugs, which have obvious analgesic, anti-inflammatory and antipyretic effects. Diclofenac sodium eye drops are used to treat uveitis, keratitis, and scleritis, and inhibit the formation of corneal neovascularization; to treat inflammatory reactions after intraocular surgery, laser curtain plasty or various eye injuries; to inhibit miosis during cataract surgery; to relieve pain and reduce inflammation after photorefractive keratectomy; to treat vernal conjunctivitis, seasonal allergic conjunctivitis and other allergic eye diseases; to prevent and treat inflammation and cystoid macular edema after cataract and intraocular lens surgery; and to promote the formation of filtering vesicles after glaucoma filtering surgery. Diclofenac sodium eye drops are used for ocular surface or open ocular trauma. The current marketed product is Difel with a content of 1 mg/mL. The structural formulas of diclofenac and diclofenac sodium are as follows:

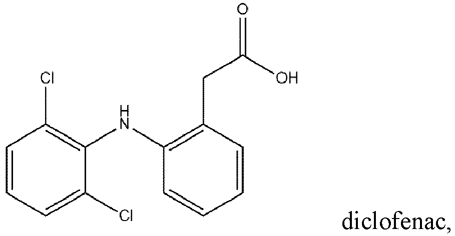

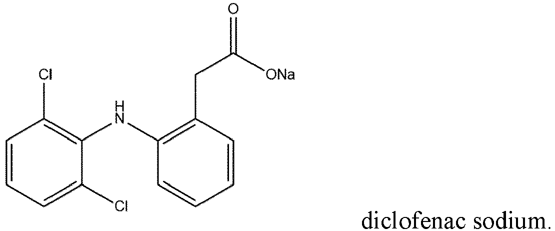

Vitamin A: vitamin A, which comprises retinol (A1) and 3-dehydroretinol (A2), is a type of fat-soluble vitamin that is stable to heat, acid and alkali. Vitamin A has a variety of physiological functions such as promoting growth and reproduction, maintaining bones, epithelial tissues, vision and mucosal epithelial normal secretion. Vitamin A and its analogues can prevent precancerous lesions. Vitamin A deficiency is manifested by growth retardation and decreased dark adaptability, resulting in night blindness. Vitamin A eye drops are used to promote the growth of corneal cells and for the treatment of dry eye. The structural formulas of retinol (A1) and 3-dehydroretinol (A2) are as follows:

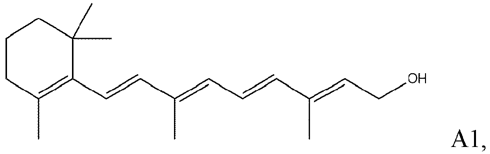

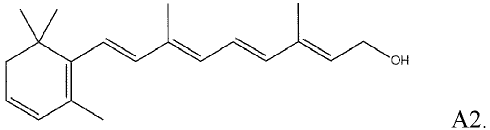

Luteolin: luteolin is a natural flavonoid compound that exists in various plants. It has various pharmacological activities, such as anti-inflammatory, anti-allergic, uric acid lowering, anti-tumor, anti-bacterial, anti-viral, etc. It is mainly used clinically for cough relief, expectorant, anti-inflammatory, urate lowering, treatment of cardiovascular diseases, and treatment of amyotrophic lateral sclerosis, SARS, hepatitis, etc. Luteolin, mostly in the form of glycosides, exists in a variety of plants. The structural formulas of luteolin and glycoside form thereof are as follows:

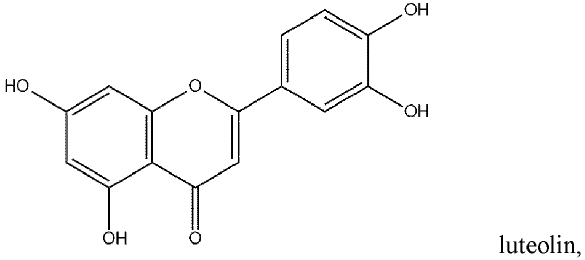

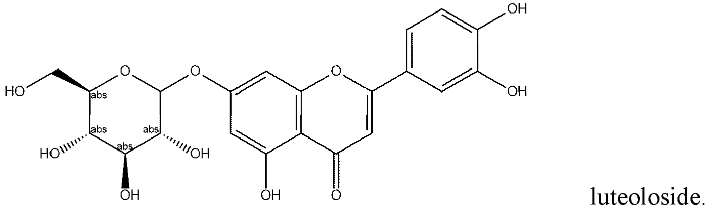

In the present disclosure, the salt is typically pharmaceutically acceptable salt, as described above. In one preferred example, the pharmaceutically acceptable salt is a sodium salt, for example, diclofenac sodium.

Preferably, the glycoside is luteoloside.

Preferably, the vitamin A is vitamin A1 and/or vitamin A2. In one preferred example of the present disclosure, the vitamin A is purchased from SinoPharm, the SinoPharm code is CATOCCHM700908, CAS: 68-26-8.

Preferably, in the drops, the diclofenac (or such as sodium salts thereof, diclofenac sodium) has a content of 5-20 µmol/L, such as 10 µmol/L.

Preferably, in the drops, the vitamin A preferably has a content of 2-10 µmol/L, such as 5 µmol/L.

Preferably, in the drops, the luteolin (or the forms of glycoside thereof, luteoloside) has a content of 2-10 µmol/L, such as 5 µmol/L.

In one preferred example, the drops consists of 10 µmol/L transthyretin, an excipient, 10 µmol/L diclofenac sodium and saline, wherein the excipient is any one of 6 mg/mL sodium carboxymethyl cellulose, 0.4 mg/mL dextran 70, 20 mg/mL chondroitin sulfate A sodium salt, and 2% (mass volume percentage) hyaluronic acid.

In one preferred example, the drops consists of 10 µmol/L transthyretin, an excipient, 5 µmol/L vitamin A, 5% (v/v) Tween 80 and saline, wherein the excipient is any one of 6 mg/mL sodium carboxymethyl cellulose, 0.4 mg/mL dextran 70, 20 mg/mL chondroitin sulfate A sodium salt, and 2% (mass volume percentage) hyaluronic acid.

In one preferred example, the drops consists of 10 µmol/L transthyretin, an excipient, 5 µmol/L luteoloside and saline, wherein the excipient is any one of 6 mg/mL sodium carboxymethyl cellulose, 0.4 mg/mL dextran 70, 20 mg/mL chondroitin sulfate A sodium salt, and 2% (mass volume percentage) hyaluronic acid.

When the drops comprises the compound described above, the method for preparing the drops comprises mixing the compound and the transthyretin and/or the fusion protein.

To address the above-mentioned technical problems, the third aspect of the present disclosure provides a drops (for example, in the form of eye drops) comprising a transthyretin, and/or a fusion protein consisting of a transthyretin and a medicament; wherein the medicament is protein and/or polypeptide medicament, and the transthyretin is represented by (a), (b) or (c):
(a) a protein consisting of an amino acid sequence of SEQ ID NO: 1;
(b) a protein derived from (a) with an inhibitory function of neovascularization, which is shown by a sequence in which one or more amino acids are substituted, deleted or added in the amino acid sequence of (a);
(c) a protein having a sequence with a hydrophilic modification or hydrophobic modification in the amino acid sequence of (a) or (b).

Preferably, in (b), the protein derived from (a) is a protein in which 22 amino acids are substituted in the amino acid sequence of (a).

Preferably, in (b), the protein derived from (a) is a protein in which 25 amino acids are substituted in the amino acid sequence of (a).

Preferably, in (b), the protein derived from (a) is a protein in which T3, T5, 126, N27, H31, R34, A36, A37, D38, D39, T40, S50, E61, E63, V65, 168, K70, 173, A81, H90, E92, P102, R104, T123, K126 and/or E127 are substituted in the amino acid sequence of (a). More preferably, in (b), the protein derived from (a) is a protein in which T3G, T5A, I26V, N27D, H31K, R34K, A36T, D39G, T40S, S50A, E61D, E63K, V65T, I68V, K70R, I73L, H90Y, P102H, R104H, T123S, K126Q and E127N are substituted in the amino acid sequence of (a). More preferably, in (b), the protein derived from (a) is a protein in which T3G, T5A, I26V, N27D, H31K, R34K, A36T, A37S, D38E, D39G, T40S, S50A, E61D, E63K, I68V, K70R, I73L, A81T, H90F, E92D, P102H, R104H, T123S, K126Q and E127N are substituted in the amino acid sequence of (a).

Preferably, in (b), the protein derived from (a) is a protein in which 5 amino acids are deleted from the amino acid sequence of (a). More preferably, in (b), the protein derived from (a) is a protein in which a deletion occurs at positions 123-127 of the amino acid sequence of (a).

Preferably, in (b), the protein derived from (a) has an amino acid sequence of SEQ ID NO: 9, SEQ ID NO: 10 or SEQ ID NO: 11.

Preferably, in (c), the hydrophilic modification or hydrophobic modification occurs at the cysteine at position 10 of the amino acid sequence of (a). Preferably, the hydrophobic modification is a modification with a long chain hydrophobic fragment such as *n*-dodecane. In one preferred embodiment of the present disclosure, the protein is a protein in which the cysteine at position 10 of the amino acid sequence of (a) is modified with *n*-dodecane via maleiamide. In the present disclosure, the cysteine at position 10 of the amino acid sequence of (a) can also be linked with a fluorescent label such as 5-aminofluorescein, to facilitate tracking the modified protein.

Preferably, when the drops comprises the fusion protein consisted of a transthyretin and a protein and/or polypeptide medicament, the fusion protein has a content of 4-30 µmol/L, preferably 10-15 µmol/L.

Preferably, when the drops comprises the transthyretin, the transthyretin has a content of 4-30 µmol/L, preferably 5-30 µmol/L, more preferably 10-20 µmol/L, such as 10, 15, 20 µmol/L.

Preferably, the drops further comprise saline. In the present disclosure, the consumption of the saline can be determined in accordance with the standard established in the art.

Preferably, the drops further comprise a surfactant, wherein the surfactant, for example, Tween 80, preferably has a content of 5% (v/v).

Preferably, the drops further comprises hyaluronic acid, and the hyaluronic acid has a content of mass volume percentage of no more than 6%, preferably 1-4%, more preferably 2%.

In one preferred embodiment of the present disclosure, the drops (for example, in the form of eye drops) further comprises saline and 1-6% hyaluronic acid.

In one preferred embodiment of the present disclosure, the drops (for example, in the form of eye drops) further comprises saline and 1-4% hyaluronic acid.

In one preferred embodiment of the present disclosure, the drops (for example, in the form of eye drops) further comprises saline and 2% hyaluronic acid.

Preferably, the drops is preferably an eye drops.

Preferably, the drops is a drops that inhibits ocular retina leakage and/or reduces the number of retinal neovascularization; preferably a drops that treats diabetic retinopathy, age-related macular degeneration and/or retinopathy of prematurity.

Preferably, the drops is administered 1-3 times per day, preferably at an amount of 0.3-0.8 nmol protein per eye at each time.

Preferably, the drops is administered twice per day, one drop each time, for 3 months; and/or, the drops is administered once per day, one drop each time, for 5 days; and/or, the drops is administered twice per day, one drop each time, for 2 weeks.

Preferably, the transthyretin is encoded by a nucleotide sequence of SEQ ID NO: 2.

Preferably, the transthyretin is expressed by a recombinant expression vector, wherein the recombinant expression vector has a plasmid backbone comprising a rhamnose inducible promoter, preferably a rhaPBAD promoter.

Preferably, the transthyretin is expressed by a recombinant expression vector, the recombinant expression vector has a plasmid backbone of pET-21a or a vector with 25% or more homology therewith, and preferably the vector with 25% or more homology therewith has a sequence of SEQ ID NO: 8.

Preferably, the recombinant plasmid expressing transthyretin is encoded by a nucleotide sequence of SEQ ID NO: 3.

Preferably, the transthyretin is expressed in a microbial cell (in the form of a transformant), and can further followed by a purification of the transthyretin; wherein the microbial cell can be an *E. coli,* and the *E coli* includes, but is not limited to, *E*. *coli* BL21, *E. coli* BL21 (DE3), *E. coli* JM109, *E. coli* DH5α, *E. coli* K12 or *E. coli* TOP10. The purification can be removing endotoxin by an endotoxin absorption column (e.g. using Pierce^{™} High Capacity Endotoxin Removal Spin Columns, ThermoFisher) and removing residue bacteria by a filter membrane with a pore size of 0.22 µm.

Preferably, the transthyretin is expressed by culturing the transformant comprising a gene of the transthyretin until the bacteria obtained reaches an OD₆₀₀ of 1.5-2.0 (such as 1.6, 1.7, 1.8 or 1.9).

Preferably, expression of the transthyretin is induced by a reagent for inducible expression, wherein the reagent for inducible expression has a mass volume percentage of 0.1-2%, such as 0.2%, 0.3%, 0.4%, 0.5%, 0.7%, 0.8%, 1.2% or 1.6%, while the expression is preferably induced for a period of 8-20 h, such as 10 h, 12 h, 14 h, 16 h, 17 h, 18 h or 19 h; the reagent for inducible expression is preferably rhamnose or IPTG.

Preferably, the fusion protein has a sequence of SEQ ID NO: 6 or SEQ ID NO: 7.

Preferably, the transthyretin is expressed with the protein and/or polypeptide medicament as a fusion; wherein the fusion is preferably a fusion that the protein and/or polypeptide medicament are fused at the N-terminus or C-terminus of the transthyretin; the transthyretin is expressed with the protein and/or polypeptide medicament as a fusion in a microbial cell, followed by a purification of the fusion. The microbial cell can be an *E. coli,* and the *E coli* includes, but is not limited to, *E*. *coli* BL21, *E. coli* BL21 (DE3), *E. coli* JM109, *E. coli* DH5α, *E. coli* K12 or *E. coli* TOP10. The purification is preferably to remove endotoxin by an endotoxin absorption column and then remove residue bacteria by a filter membrane with a pore size of 0.22 µm.

Preferably, the protein and/or polypeptide medicament include, but are not limited to, lysozyme, albumin and/or EGFR antibody, with a molecular weight of no more than 45 kDa. Wherein, the lysozyme is preferably hen egg white lysozyme with GenBank Accession No.: AAL69327.1. Wherein, the albumin is preferably ovalbumin.

Preferably, the protein and/or polypeptide medicament comprises a protein and/or polypeptide medicament for treating ocular diseases associated with ocular retina leakage and/or retinal neovascularization such as diabetic retinopathy, age-related macular degeneration and/or retinopathy of prematurity.

In one preferred embodiment of the present disclosure, the drops (such as eye drops) described above comprises 5-20 µmol/L of transthyretin and is administered twice per day, one drop each time, for 3 months.

In one preferred embodiment of the present disclosure, the drops (such as eye drops) described above comprises 10-15 µmol/L of transthyretin and is administered twice per day.

In one preferred embodiment of the present disclosure, the drops (such as eye drops) described above comprises 10 µmol/L of transthyretin and is administered twice per day.

In one preferred embodiment of the present disclosure, the drops (such as eye drops) described above comprises 5-20 µmol/L of transthyretin and is administered once per day, one drop each time, for 5 days.

In one preferred embodiment of the present disclosure, the drops (such as eye drops) described above comprises 10-15 µmol/L of transthyretin and is administered once per day.

In one preferred embodiment of the present disclosure, the drops (such as eye drops) described above comprises 10 µmol/L of transthyretin and is administered once per day.

In one preferred embodiment of the present disclosure, the drops (such as eye drops) described above comprises 5-20 µmol/L of transthyretin and is administered twice per day, one drop each time, lasting for 2 weeks.

In one preferred embodiment of the present disclosure, the drops (such as eye drops) described above comprises 10-15 µmol/L of transthyretin and is administered twice per day.

In one preferred embodiment of the present disclosure, the drops (such as eye drops) described above comprises 10 µmol/L of transthyretin and is administered twice per day.

In the present disclosure, the dosage of eye dropping and the content of transthyretin of eye dropping listed is advisory. It should be understood by a person skilled in the art that, the dosage and the amount when moderately adjusted for administration of a suitable subject according to the present disclosure shall also be within the scope of the present disclosure.

The present inventors also unexpectedly discovered during the experiment that materials, for example, carboxymethyl cellulose or salts thereof such as sodium carboxymethyl cellulose, chondroitin sulfate or salts thereof such as chondroitin sulfate A sodium salt, dextran such as dextran 70, hyaluronic acid, can mutual check and synergistically cooperate with transthyretin and/or the fusion protein to form an organic whole, thus allowing for the synergistic treatment of ocular diseases associated with ocular neovascularization and/or ocular retinal leakage, such as diabetic retinopathy, age-related macular degeneration and/or retinopathy of prematurity, ultimately achieving a better therapeutic effect. However, a person skilled in the art only knows that materials, for example, carboxymethyl cellulose or salts thereof such as sodium carboxymethyl cellulose, chondroitin sulfate or salts thereof such as chondroitin sulfate A sodium salt, dextran such as dextran 70 or hyaluronic acid has effects such as moisturizing, which generally can be used to prepare an eye drops to effectively relieve eye dryness, etc.

Based on this, the drops described above can further comprise a pharmaceutically acceptable excipient, the pharmaceutically acceptable excipient is one or more selected from "carboxymethyl cellulose or salts thereof, solvates thereof, solvates of pharmaceutically acceptable salts thereof, or, crystal forms thereof', "chondroitin sulfate or salts thereof, solvates thereof, solvates of pharmaceutically acceptable salts thereof, or, crystal forms thereof', "dextran or salts thereof, solvates thereof, solvates of pharmaceutically acceptable salts thereof, or, crystal forms thereof' and "hyaluronic acid or salts thereof, solvates thereof, solvates of pharmaceutically acceptable salts thereof, or, crystal forms thereof'.

Preferably, the carboxymethyl cellulose or salts thereof, solvates thereof, solvates of pharmaceutically acceptable salts thereof, or, crystal forms thereof has a viscosity of 800-1200 CP.

Preferably, the chondroitin sulfate is chondroitin sulfate A.

Preferably, the dextran is dextran 70. The dextran 70 generally has a molecular weight in the range of 64000-76000.

Preferably, the hyaluronic acid has a molecular weight of 10000-500000.

In one preferred embodiment, the term "salts" described in the "carboxymethyl cellulose or salts thereof' can be sodium salts or calcium salts, such as sodium carboxymethyl cellulose.

In one preferred embodiment, the term "salts" described in the "chondroitin sulfate or salts thereof' can be sodium salts or calcium salts, such as chondroitin sulfate A sodium salt.

Preferably, in the drops, the carboxymethyl cellulose or salts thereof, solvates thereof, solvates of pharmaceutically acceptable salts thereof, or, crystal forms thereof has a concentration of 0-8 mg/mL except 0 (i.e., 0<concentration≤8 mg/mL), preferably 2, 4, 6 or 8 mg/mL.

Preferably, in the drops, the chondroitin sulfate or salts thereof, solvates thereof, solvates of pharmaceutically acceptable salts thereof, or, crystal forms thereof has a concentration of 0-40 mg/mL except 0 (i.e., 0<concentration≤40 mg/mL), preferably 10, 20, 30 or 40 mg/mL.

Preferably, in the drops, the dextran or salts thereof, solvates thereof, solvates of pharmaceutically acceptable salts thereof, or, crystal forms thereof has a concentration of 0-0.8 mg/mL except 0 (i.e., 0<concentration≤0.8 mg/mL), more preferably 0.2, 0.4, 0.6 or 0.8 mg/mL.

Preferably, in the drops, the hyaluronic acid or salts thereof, solvates thereof, solvates of pharmaceutically acceptable salts thereof, or, crystal forms thereof has a content of no more than 6% by mass volume percentage, preferably 1-4%, more preferably 2%.

In one preferred embodiment, the drops consists of 10 µmol/L transthyretin, 6 mg/mL carboxymethyl cellulose sodium and saline.

In one preferred embodiment, the drops consists of 10 µmol/L transthyretin, 0.4 mg/mL dextran 70 and saline.

In one preferred embodiment, the drops is consisted of 10 µmol/L of transthyretin, 20 mg/mL chondroitin sulfate A sodium salt and saline.

In one preferred embodiment, the drops consists of 10 µmol/L transthyretin, 2% hyaluronic acid and saline.

When the drops comprises the pharmaceutically acceptable excipient described above, the method for preparing the drops comprises mixing the pharmaceutically acceptable excipient and the transthyretin and/or the fusion protein.

The present inventors unexpectedly discovered during the experiment that when co-administering the transthyretin and/or the fusion protein with a specific compound to the eye, the compound is able to synergistically cooperate and co-effect with the transthyretin and/or the fusion protein, which can treat or relieve ocular diseases associated with ocular retinal neovascularization and/or ocular retinal leakage effectively, making the treatment more effective.

Based on this, the drops described above can further comprise a compound, pharmaceutically acceptable salts thereof, glycoside thereof, solvates thereof, solvates of a pharmaceutically acceptable salt thereof, or crystal form thereof; the compound is one or more selected from diclofenac, vitamin A and luteolin.

In the present disclosure, the salt is typically a pharmaceutically acceptable salt, as described above. In one preferred example, the pharmaceutically acceptable salt is a sodium salt, for example, diclofenac sodium.

Preferably, the glycoside is luteoloside.

Preferably, the vitamin A is vitamin A1 and/or vitamin A2. In one preferred example of the present disclosure, the vitamin A is purchased from SinoPharm, and the SinoPharm code is CATOCCHM700908, CAS: 68-26-8.

Preferably, in the drops, the diclofenac (or such as sodium salts thereof, diclofenac sodium) has a content of 5-20 µmol/L, such as 10 µmol/L.

Preferably, in the drops, the vitamin A preferably has a content of 2-10 µmol/L, such as 5 µmol/L.

Preferably, in the drops, the luteolin (or the forms of glycoside thereof, luteoloside) has a content of 2-10 µmol/L, such as 5 µmol/L.

In one preferred example, the drops consists of 10 µmol/L transthyretin, an excipient, 10 µmol/L diclofenac sodium and saline, wherein the excipient is any one of 6 mg/mL sodium carboxymethyl cellulose, 0.4 mg/mL dextran 70, 20 mg/mL chondroitin sulfate A sodium salt, and 2% (mass volume percentage) hyaluronic acid.

In one preferred example, the drops consists of 10 µmol/L transthyretin, an excipient, 5 µmol/L vitamin A, 5% (v/v) Tween 80 and saline, wherein the excipient is any one of 6 mg/mL sodium carboxymethyl cellulose, 0.4 mg/mL dextran 70, 20 mg/mL chondroitin sulfate A sodium salt, and 2% (mass volume percentage) hyaluronic acid.

In one preferred example, the drops consists of 10 µmol/L transthyretin, an excipient, 5 µmol/L luteoloside and saline, wherein the excipient is any one of 6 mg/mL sodium carboxymethyl cellulose, 0.4 mg/mL dextran 70, 20 mg/mL chondroitin sulfate A sodium salt, and 2% (mass volume percentage) hyaluronic acid.

When the drops comprises the compound described above, the method for preparing the drops comprises mixing the compound and the transthyretin and/or the fusion protein.

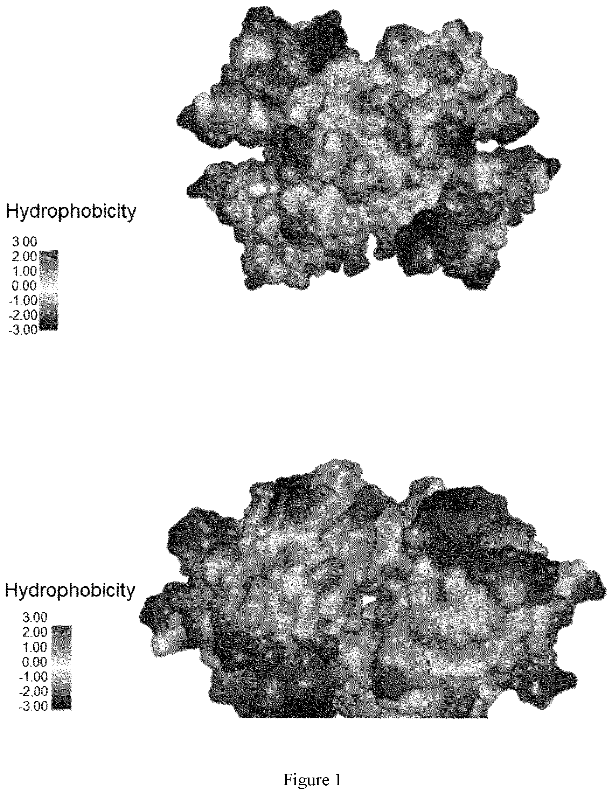

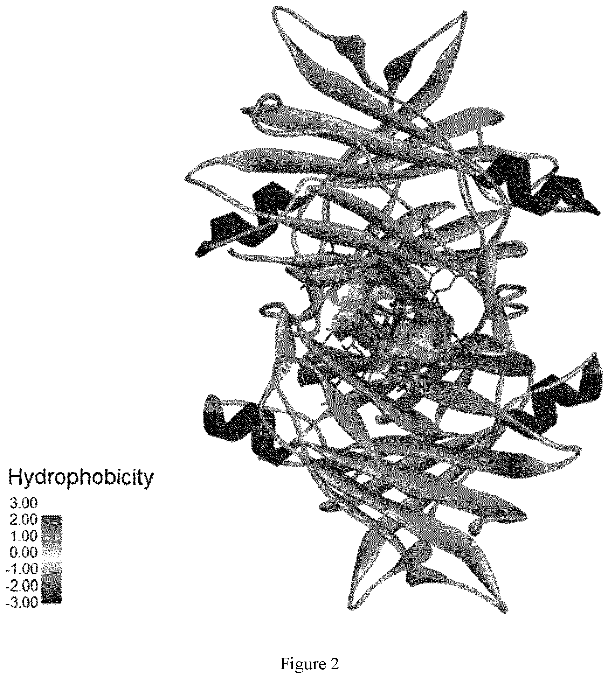

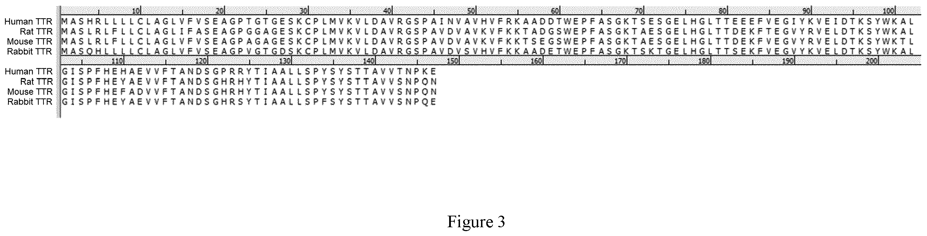

In the present disclosure, the transthyretin is a tetrameric carrier protein that can transport thyroid hormones in plasma and cerebrospinal fluid. Studies have found that the entry of transthyretin into cells is mediated by the high-density lipoprotein receptor SRB1 (Landers, KA, et al., Transthyretin uptake in placental cells is regulated by the high-density lipoprotein receptor, scavenger receptor class B member 1. Mol Cell Endocrinol, 2018. 474: p. 89-96). From the structural point of view (see Figure 1 for the three-dimensional structure), the surface of the tetramer of transthyretin has significant hydrophilic (dark) and hydrophobic domains (light), and the core hydrophobic domain of transthyretin is capable of carrying thyroxine molecules with strong hydrophobicity span across various cells (see Figure 2). In addition, the amino acid sequences of transthyretin from different species are highly conserved, and the amino acid sequence similarity between human transthyretin and transthyretin derived from SD rats and C57BL/6 mice is >95% (see Figure 3).

The present inventors found during the preparation of drops that the recombinant expression level of transthyretin in *E. coli* was extremely low, mostly in the form of insoluble inclusion bodies. Commonly used methods to increase protein expression include expression carrier selection, optimization of fermentation conditions (temperature, pH, time, concentration of inducer, etc.), or molecular chaperone co-expression assistance, etc., but the present inventors have tried the above known methods, and none of them can effectively improve the expression level of transthyretin in *E. coli.* After extensive experiments, the present inventors found that after replacing the promoter (such as T7 promoter) of the plasmid, e.g., using rhamnose inducible promoter (such as rhaPBAD promoter) to replace the promoter of the pET-21a plasmid, or after codon optimization of the nucleotide sequence of TTR (nucleotide sequence as shown in SEQ ID NO: 2), or after sequence reconstruction of the pET-21a plasmid itself, or after optimizing the OD value when expressing the protein, the amount of reagents that induce protein expression, and the time for inducing expression, an efficient production plasmid for the mature transthyretin fragment (NCBI Reference Sequence: NP_000362.1) can be constructed, which results in a significant increased expression of the transthyretin finally obtained. Therefore, the present disclosure further provides a gene expressing transthyretin, a recombinant expression vector and a transformant thereof, a recombinant plasmid for expressing transthyretin, and a method for expressing transthyretin, etc. When the gene expressing transthyretin, the recombinant plasmid for expressing transthyretin, or the method for expressing transthyretin in the present disclosure is used, the expression level of transthyretin is significantly increased. Particularly:

The fourth aspect of the present disclosure also provides a gene expressing transthyretin, which has a nucleotide sequence of SEQ ID NO: 2.

The fifth aspect of the present disclosure also provides a recombinant expression vector comprising the gene as described in the fourth aspect of the present disclosure.

Preferably, the recombinant expression vector has a backbone vector comprising a rhamnose inducible promoter; the rhamnose inducible promoter is preferably rhaPBAD promoter.

Preferably, the recombinant expression vector has a backbone vector of pET-21a or a vector has 25% or more homology with pET-21a, and the vector has 25% or more homology with pET-21a preferably has a sequence of SEQ ID NO: 8.

More preferably, the recombinant expression vector has a nucleotide sequence of SEQ ID NO: 3.

The sixth aspect of the present disclosure also provides a recombinant plasmid having a sequence of SEQ ID NO: 8.

The seventh aspect of the present disclosure also provides a recombinant plasmid expressing transthyretin, wherein the recombinant plasmid comprises a plasmid backbone and an expression fragment of transthyretin,
wherein, the backbone plasmid has a rhamnose inducible promoter; and/or, the backbone vector of the backbone plasmid is pET-21a or a vector with 25% or more homology therewith, and the vector with 25% or more homology therewith preferably has a sequence of SEQ ID NO: 8.

Preferably, a nucleic acid for encoding an expression fragment of the transthyretin has a nucleotide sequence of SEQ ID NO: 2.

Preferably, the rhamnose inducible promoter is rhaPBAD promoter.

More preferably, the recombinant plasmid has a nucleotide sequence of SEQ ID NO: 3.

The eighth aspect of the present disclosure also provides a transformant comprising the gene as described in the fourth aspect of the present disclosure, or the recombinant expression vector as described in the fifth aspect of the present disclosure, or the recombinant plasmid as described in the sixth or seventh aspect of the present disclosure.

Preferably, the gene as described in the fourth aspect of the present disclosure, or the recombinant expression vector as described in the fifth aspect of the present disclosure, or the recombinant plasmid as described in the sixth or seventh aspect of the present disclosure is introduced to a host, and the host is an *E. coli,* preferably *E*. *coli* BL21 (DE3), *E. coli* TG1, *E. coli* JM109, *E. coli* DH5α, *E. coli* K12 or *E. coli* TOP10.

The ninth aspect of the present disclosure also provides a method for preparing transthyretin comprising the following steps:
(1) obtaining the transformant as described in the eighth aspect of the present disclosure;
(2) screening the transformant, and expressing and purifying the protein.

Preferably, the expressing means expressing transthyretin by culturing the transformant until the bacteria obtained reaches an OD₆₀₀ of 1.5-2.0 (such as 1.6, 1.7, 1.8 or 1.9).

Preferably, the expressing represents an inducible expressing using a reagent for inducible expression, wherein the reagent for inducible expression has a mass volume percentage of 0.1-2%, such as 0.2%, 0.3%, 0.4%, 0.5%, 0.7%, 0.8%, 1.2% or 1.6%, and the expression is preferably induced for a period of 8-20 h, such as 10 h, 12 h, 14 h, 16 h, 17 h, 18 h or 19 h; and the reagent for inducible expression is preferably rhamnose or IPTG.

The tenth aspect of the present disclosure also provides a method for expressing transthyretin comprising the steps of obtaining a transformant comprising the gene of transthyretin, and screening the transformant, and the steps of expressing and purifying the transthyretin.

Preferably, the expressing represents an inducible expressing using a reagent for inducible expression, wherein the reagent for inducible expression has a mass volume percentage of 0.1-2%, such as 0.2%, 0.3%, 0.4%, 0.5%, 0.7%, 0.8%, 1.2% or 1.6%, and the expression is preferably induced for a period of 8-20 h, such as 10 h, 12 h, 14 h, 16 h, 17 h, 18 h or 19 h; and the reagent for inducible expression is preferably rhamnose or IPTG.

Based on this, through extensive experiments, the present inventors also unexpectedly found that after the transthyretin enters the vitreous body and fundus oculi through corneal barrier, transthyretin can inhibit eyeball retinal leakage and reduce the number of retinal neovascularization significantly, alleviate the ocular diseases such as DR, AMD, and ROP effectively. Therefore, the present disclosure also provides a use of transthyretin in preparing the medicament for inhibiting ocular retinal leakage and/or reducing the number of retinal neovascularization. For example, a use in preparing a medicament for treating ocular diseases such as diabetic retinopathy, age-related macular degeneration and/or retinopathy of prematurity.

The eleventh aspect of the present disclosure also provides a use of the transthyretin and/or the drops as described in the third aspect of the present disclosure in preparing the medicament for inhibiting ocular retina leakage and/or reducing the number of retinal neovascularization; preferably in preparing the medicament for treating diabetic retinopathy, age-related macular degeneration and/or retinopathy of prematurity.

Preferably, the transthyretin is present in the form of non-injection dosage, the non-injectable dosage form is preferably a liniment or a drops, wherein the liniment is preferably a cream (e.g., eye ointment) or a gel (e.g. ophthalmic gel), the drops is for example an eye drops (the components in the drops, the dosage used, etc. can be as described above).

Preferably, the transthyretin is expressed by using the gene as described in the fourth aspect of the present disclosure, the recombinant expression vector as described in the fifth aspect of the present disclosure, or the recombinant plasmid as described in the sixth or seventh aspect of the present disclosure, or the transformant as described in the eighth aspect of the present disclosure.

The twelfth aspect of the present disclosure also provides a use of the transthyretin and/or the drops as described in the third aspect of the present disclosure in treating ocular diseases. The ocular diseases are preferably ocular diseases associated with ocular retina leakage and/or retinal neovascularization, more preferably diabetic retinopathy, age-related macular degeneration and/or retinopathy of prematurity. The transthyretin is preferably as described above. The conditions, dosages, administering manner, etc. adopted in the use can be applied as described above.

The thirteenth aspect of the present disclosure also provides a method for treating ocular diseases associated with ocular retina leakage and/or retinal neovascularization such as diabetic retinopathy, age-related macular degeneration and/or retinopathy of prematurity, comprising administering the transthyretin as described above and/or the drops as described in the third aspect of the present disclosure. The conditions, dosages, administering manner, etc. of treatment in the method can be applied as described above.

The fourteenth aspect of the present disclosure also provides a method for delivering the exogenous protein and/or polypeptide by using the transthyretin described above into an eye through ocular barrier.

In one preferred example of the present disclosure, the fusion protein described above is prepared as follows: (1) ligating the encoding genes of transthyretin and pharmaceutical protein and/or polypeptide with pET-21a (+) to obtain a recombinant plasmid; (2) transforming the recombinant plasmid constructed in step (1) into the host cell for expression; (3) inducing with 0.1-0.5 mM IPTG for 8-16 h, when TB medium is used, fermentation temperature is 30-40°C and OD₆₀₀ reaches 1.5-2.0; (4) purifying the target protein by nickel column affinity absorption, removing endotoxin by an endotoxin absorption column (e.g., using Pierce^{™} High Capacity Endotoxin Removal Spin Columns, ThermoFisher), followed by removing residue bacteria by a filter membrane with a pore size of 0.22 µm.

In one preferred example of the present disclosure, the expression and purification of transthyretin comprise the following steps: transforming constructed pETx-rhaPBAD-*ttr* plasmid (the entire nucleotide sequence of the plasmid is SEQ ID NO: 3) into *E*. *coli* BL21 (DE3) cells, culturing the recombinant *E*. *coli* BL21 (DE3) cells obtained in LB medium to prepare an inoculum, then inoculating 5% of the inoculum into 5 L of TB medium, incubating at a temperature of 37°C and paddle speed of 150 rpm until OD₆₀₀ of the culture reaches 1.5-2.0, and inducing for 16-20 h by adding 0.4-2% of rhamnose. The endotoxin is removed by an endotoxin absorption column (e.g. using Pierce^{™} High Capacity Endotoxin Removal Spin Columns, ThermoFisher) and residue bacteria is removed by a filter membrane with a pore size of 0.22 µm.

Based on this, the present inventors unexpectedly discovered during the experiment that materials, for example, carboxymethyl cellulose or salts thereof such as sodium carboxymethyl cellulose, chondroitin sulfate or salts thereof such as chondroitin sulfate A sodium salt, dextran such as dextran 70, hyaluronic acid, can mutual check and synergistically cooperate with transthyretin and/or the fusion protein to form an organic whole, thus allowing for the synergistic treatment of ocular diseases associated with ocular neovascularization and/or ocular retinal leakage, such as diabetic retinopathy, age-related macular degeneration and/or retinopathy of prematurity, ultimately achieving a better therapeutic effect.

The fifteenth aspect of the present disclosure also provides an ocular preparation comprising a transthyretin and a pharmaceutically acceptable excipient, and the pharmaceutically acceptable excipient is one or more selected from "carboxymethyl cellulose or salts thereof, solvates thereof, solvates of pharmaceutically acceptable salts thereof, or, crystal forms thereof', "chondroitin sulfate or salts thereof, solvates thereof, solvates of pharmaceutically acceptable salts thereof, or, crystal forms thereof', "dextran or salts thereof, solvates thereof, solvates of pharmaceutically acceptable salts thereof, or, crystal forms thereof' and "hyaluronic acid or salts thereof, solvates thereof, solvates of pharmaceutically acceptable salts thereof, or, crystal forms thereof'.

Preferably, the carboxymethyl cellulose or salts thereof, solvates thereof, solvates of pharmaceutically acceptable salts thereof, or, crystal forms thereof has a viscosity of 800-1200 CP.

Preferably, the chondroitin sulfate is chondroitin sulfate A.

Preferably, the dextran is dextran 70. The dextran 70 is generally has a molecular weight in the range of 64000-76000.

Preferably, the hyaluronic acid has a molecular weight of 10000-500000.

In one preferred embodiment, the term "salts" described in the "carboxymethyl cellulose or salts thereof' can be sodium salts or calcium salts, such as sodium carboxymethyl cellulose.

In one preferred embodiment, the term "salts" described in the "chondroitin sulfate or salts thereof' can be sodium salts or calcium salts, such as chondroitin sulfate A sodium salt.

Preferably, in the drops, the carboxymethyl cellulose or salts thereof, solvates thereof, solvates of pharmaceutically acceptable salts thereof, or, crystal forms thereof has a concentration of 0-8 mg/mL except 0 (i.e., 0<concentration≤8 mg/mL), preferably 2, 4, 6 or 8 mg/mL.

Preferably, in the drops, the chondroitin sulfate or salts thereof, solvates thereof, solvates of pharmaceutically acceptable salts thereof, or, crystal forms thereof has a concentration of 0-40 mg/mL except 0 (i.e., 0<concentration≤40 mg/mL), preferably 10, 20, 30 or 40 mg/mL.

Preferably, in the drops, the dextran or salts thereof, solvates thereof, solvates of pharmaceutically acceptable salts thereof, or, crystal forms thereof has a concentration of 0-0.8 mg/mL except 0 (i.e., 0<concentration≤0.8 mg/mL), more preferably 0.2, 0.4, 0.6 or 0.8 mg/mL.

Preferably, in the drops, the hyaluronic acid or salts thereof, solvates thereof, solvates of pharmaceutically acceptable salts thereof, or, crystal forms thereof has a content of no more than 6% by mass volume percentage, preferably 1-4%, more preferably 2%.

Preferably, in the ocular preparation, the transthyretin is represented by (a), (b) or (c):
(a) a protein consisting of an amino acid sequence of SEQ ID NO: 1;
(b) a protein derived from (a) with an inhibitory function of neovascularization, which is shown by a sequence in which one or more amino acids are substituted, deleted or added in the amino acid sequence of (a);
(c) a protein having a sequence with a hydrophilic modification or hydrophobic modification in the amino acid sequence of (a) or (b).

Wherein, in (b), the protein derived from (a) can have an amino acid sequence of SEQ ID NO: 9, SEQ ID NO: 10 or SEQ ID NO: 11.

Wherein, in (c), the hydrophilic modification or hydrophobic modification can occur at the cysteine at position 10 of the amino acid sequence of (a), preferably is a modification at the cysteine at position 10 of the amino acid sequence of (a) with a long chain hydrophobic fragment such as *n*-dodecane, or, is a modification at the cysteine at position 10 of the amino acid sequence of (a) with *n*-dodecane via maleiamide.

Preferably, the transthyretin contained in the ocular preparation has a content of 4-30 µmol/L, preferably 5-30 µmol/L, more preferably 10-20 µmol/L, such as 10, 15 or 20 µmol/L.

Preferably, the ocular preparation may also comprise other pharmaceutically acceptable excipients commonly used in the art, such as saline. In the present disclosure, the consumption of the saline can be determined in accordance with the standard established in the art.

Preferably, the ocular preparation can be in the form of a product conventionally applied to the eye in the art, for example, it can be a drops such as an eye drops, a spray, a gel, or an ocular liposome, etc.

Preferably, the ocular preparation is an ocular preparation for inhibiting ocular retinal leakage and/or reducing the number of retinal neovascularization; the ocular preparation is preferably an ocular preparation for treating diabetic retinopathy, age-related macular degeneration and/or retinopathy of prematurity.

Preferably, the ocular preparation is administered 1-3 times per day at a preferred dosage of 0.3-0.8 nmol protein per eye.

Preferably, the ocular preparation is administered as one drop twice a day for 3 months.

Preferably, the ocular preparation is administered as one drop once a day for 5 days.

Preferably, the ocular preparation is administered as one drop twice a day for 2 weeks.

In one preferred embodiment, the ocular preparation consists of 10 µmol/L of transthyretin, 6 mg/mL sodium carboxymethyl cellulose and saline.

In one preferred embodiment, the ocular preparation consists of 10 µmol/L of transthyretin, 0.4 mg/mL dextran 70 and saline.

In one preferred embodiment, the ocular preparation consists of 10 µmol/L of transthyretin, 20 mg/mL chondroitin sulfate A sodium salt and saline.

In one preferred embodiment, the ocular preparation consists of 10 µmol/L of transthyretin, 2% hyaluronic acid and saline.

Based on this, the present inventors unexpectedly discovered in the experiment that when co-administering transthyretin and/or the fusion protein with a specific compound to the eye, the compound is able to synergistically cooperate and co-effect with the transthyretin and/or the fusion protein, which can treat or relieve ocular diseases associated with ocular retinal neovascularization and/or ocular retinal leakage effectively, making the treatment more effective.

The sixteenth aspect of the present disclosure also provides an ocular preparation comprising a compound, pharmaceutically acceptable salts thereof, glycoside thereof, solvates thereof, solvates of a pharmaceutically acceptable salt thereof, or crystal form thereof, and transthyretin; the compound is one or more selected from diclofenac, vitamin A and luteolin.

In one preferred example, the pharmaceutically acceptable salt is a sodium salt, for example, diclofenac sodium.

Preferably, the glycoside is luteoloside.

Preferably, the vitamin A is vitamin A1 and/or vitamin A2. In one preferred example of the present disclosure, the vitamin A is purchased from SinoPharm, with a code of CATOCCHM700908, CAS: 68-26-8.

Preferably, in the ocular preparation, the transthyretin has a content of 4-30 µmol/L, preferably, 5-30 µmol/L, more preferably, 10-20 µmol/L, such as 10, 15 or 20 µmol/L.

Preferably, in the ocular preparation, the diclofenac (or such as sodium salts thereof, diclofenac sodium) has a content of 5-20 µmol/L, such as 10 µmol/L.

Preferably, in the ocular preparation, the vitamin A preferably has a content of 2-10 µmol/L, such as 5 µmol/L.

Preferably, in the ocular preparation, the luteolin (or the forms of glycoside thereof, luteoloside) has a content of 2-10 µmol/L, such as 5 µmol/L.

Preferably, in the ocular preparation, the transthyretin is represented by (a), (b) or (c):
(a) a protein consisting of an amino acid sequence of SEQ ID NO: 1;
(b) a protein derived from (a) with an inhibitory function of neovascularization, which is shown by a sequence in which one or more amino acids are substituted, deleted or added in the amino acid sequence of (a);
(c) a protein having a sequence with a hydrophilic modification or hydrophobic modification in the amino acid sequence of (a) or (b).

Wherein, in (b), the protein derived from (a) would have an amino acid sequence of SEQ ID NO: 9, SEQ ID NO: 10 or SEQ ID NO: 11.

Wherein, in (c), the hydrophilic modification or hydrophobic modification can occur at the cysteine at position 10 of the amino acid sequence of (a), preferably is a modification at the cysteine at position 10 of the amino acid sequence of (a) with a long chain hydrophobic fragment such as *n*-dodecane, or, is a modification at the cysteine at position 10 of the amino acid sequence of (a) with *n*-dodecane via maleiamide.

Preferably, the ocular preparation further comprises a pharmaceutically acceptable excipient such as saline. In the present disclosure, the consumption of the saline can be determined in accordance with the standard established in the art.

Preferably, the ocular preparation further comprises a pharmaceutically acceptable excipient such as surfactant, wherein the surfactant, for example, is Tween 80, and the content of which is 5% (v/v).

Preferably, the ocular preparation further comprises a pharmaceutically acceptable excipient, the excipient is sodium carboxymethyl cellulose, dextran 70, chondroitin sulfate A sodium salt or hyaluronic acid. The sodium carboxymethyl cellulose has a concentration of 0-8 mg/mL except 0 (i.e., 0<concentration≤8 mg/mL), preferably 2, 4, 6 or 8 mg/mL. The dextran 70 has a concentration of 0-0.8 mg/mL except 0 (i.e., 0<concentration≤0.8 mg/mL), more preferably 0.2, 0.4, 0.6 or 0.8 mg/mL. The chondroitin sulfate A sodium salt has a concentration of 0-40 mg/mL except 0 (i.e., 0<concentration≤40 mg/mL), preferably 10, 20, 30 or 40 mg/mL. The hyaluronic acid has a content of no more than 6% by mass volume percentage, preferably 1-4%, more preferably 2%.

Preferably, the ocular preparation can be in the form of a product conventionally applied to the eye in the art, for example, it can be a drops such as an eye drops, a spray, a gel, or an ocular liposome, etc.

Preferably, the ocular preparation is administered 1-3 times per day at a preferred dosage of 0.3-0.8 nmol protein per eye.

Preferably, the ocular preparation is administered as one drop twice a day for 3 months.

Preferably, the ocular preparation is administered as one drop once a day for 5 days.

Preferably, the ocular preparation is administered as one drop twice a day for 2 weeks.

In one preferred example, the ocular preparation consists of 10 µmol/L transthyretin, an excipient, 10 µmol/L diclofenac sodium and saline, the excipient is any one of 6 mg/mL sodium carboxymethyl cellulose, 0.4 mg/mL dextran 70, 20 mg/mL chondroitin sulfate A sodium salt, and 2% (mass percent volume) hyaluronic acid.

In one preferred example, the ocular preparation consists of 10 µmol/L transthyretin, an excipient, 5 µmol/L vitamin A, 5% (v/v) Tween 80 and saline, wherein the excipient is any one of 6 mg/mL sodium carboxymethyl cellulose, 0.4 mg/mL dextran 70, 20 mg/mL chondroitin sulfate A sodium salt, and 2% (mass volume percentage) hyaluronic acid.

In one preferred example, the ocular preparation consists of 10 µmol/L transthyretin, an excipient, 5 µmol/L luteoloside and saline, wherein the excipient is any one of 6 mg/mL sodium carboxymethyl cellulose, 0.4 mg/mL dextran 70, 20 mg/mL chondroitin sulfate A sodium salt, and 2% (mass volume percentage) hyaluronic acid.

Preferably, the ocular preparation is an ocular preparation for inhibiting ocular retina leakage and/or reducing the number of retinal neovascularization; the ocular preparation is preferably an ocular preparation for treating diabetic retinopathy, age-related macular degeneration and/or retinopathy of prematurity.

On the basis of conforming to common knowledge in the field, the above-mentioned preferred conditions can be combined arbitrarily to obtain preferred embodiments of the present disclosure.

The reagents and raw materials used in the present disclosure are all commercially available.

In the present disclosure, the pharmaceutically acceptable excipients etc. can usually be obtained commercially, for example, from SinoPharm Group.

In the present disclosure, the mentioned compounds, etc. can usually be obtained commercially, for example, from SinoPharm Group, J&K Scientific, etc.

In the present disclosure, the term "comprise or include" can refer to that in addition to the ingredients listed below, there are other ingredients; in some cases, it may also refer to "consisting of", i.e., including only the ingredients listed below and no other ingredients.

The abbreviations of amino acids in the present invention are conventional in the art unless otherwise specified, and the amino acids corresponding to the specific abbreviations are shown in Table 1-1.

**Table 1-1**

| Amino Acid | Three letter code | Single letter code | Amino Acid | Three letter code | Single letter code |
|---|---|---|---|---|---|
| Alanine | Ala | A | Leucine | Leu | L |
| Arginine | Arg | R | Lysine | Lys | K |
| Asparagine | Asn | N | Methionine | Met | M |
| Aspartic acid | Asp | D | Phenylalanine | Phe | F |
| Cysteine | Cys | C | Proline | Pro | P |
| Glutamine | Gln | Q | Serine | Ser | S |
| Glutamic acid | Glu | E | Threonine | Thr | T |
| Glycine | Gly | G | Tryptophan | Trp | w |
| Histidine | His | H | Tyrosine | Tyr | Y |
| Isoleucine | Ile | I | Valine | Val | V |

The codons corresponding to the amino acids are also conventional in the art, and the correspondence between specific amino acids and codons are shown in Table 1-2.

**Table 1-2**

| **First Nucleotide** | **Second Nucleotide** | | | | **Third Nucleotide** |
|---|---|---|---|---|---|
| | **T** | **C** | **A** | **G** | |
| **T** | F | S | Y | C | T |
| | F | S | Y | C | C |
| | L | S | Stop codon | Stop codon | A |
| | L | S | Stop codon | w | G |
| **C** | L | P | H | R | T |
| | L | P | H | R | C |
| | L | P | Q | R | A |
| | L | P | Q | R | **G** |
| **A** | I | T | N | S | **T** |
| | I | T | N | S | **C** |
| | I | T | K | R | **A** |
| | M | T | K | R | **G** |
| G | V | A | D | G | **T** |
| | V | A | D | G | **C** |
| | V | A | E | G | **A** |
| | V | A | E | G | **G** |

The positive and progressive effects of the present invention are: transthyretin has good biocompatibility and safety in human bodies, and not only can cross an ocular barrier itself, but also can effectively convey exogenous protein and/or polypeptide into the eye to achieve the effects of treating ocular diseases, thereby having promising prospects in the pharmaceutical field in terms of application as an alternative to injectable drugs. In the present disclosure, by using the manner of eye-dropping instead of directly injection when treating ocular diseases such as DR, AMD, ROP, etc., transthyretin can enter the vitreous body and fundus oculi through corneal barrier, inhibit eyeball retina leakage significantly, and reduce the number of retinal neovascularization significantly, thus effectively alleviating ocular diseases such as DR, AMD and ROP.

### Brief description of the drawings

Figure 1 shows the three-dimensional structure of transthyretin (TTR) (PDB ID: 1ICT).
Figure 2 shows the core hydrophobic domain of TTR carrying strongly hydrophobic thyroxine molecules across various cells.
Figure 3 shows that the amino acid sequence similarity among human TTR, TTR derived from SD rats, C57BL/6 mice and rabbits is >95%, wherein the amino acid sequence of TTR derived from SD rats is shown in SEQ ID NO: 9, the amino acid sequence of TTR derived from C57BL/6 mice is shown in SEQ ID NO: 10.
Figure 4 shows the plasmid profile of pET-21a (+)-His-tag-TTR-X. The front end of the gene sequence is expressed in fusion with His-tag sequence, and ligated to the plasmid by the two restriction enzyme sites *Nde* I and *EcoR* I; "X" represents the protein fused with TTR.
Figure 5 shows the electrophoretograms of the expression of human transthyretin in *E. coli* BL21 (DE3), the purified product of the fusion protein and standards of green fluorescent protein, hen egg white lysozyme and ovalbumin.
Figure 6 shows the content of TTR in cornea, vitreous body and fundus oculi samples (retina and choroid) of C57BL/6 mice and SD rats after eye dropping.

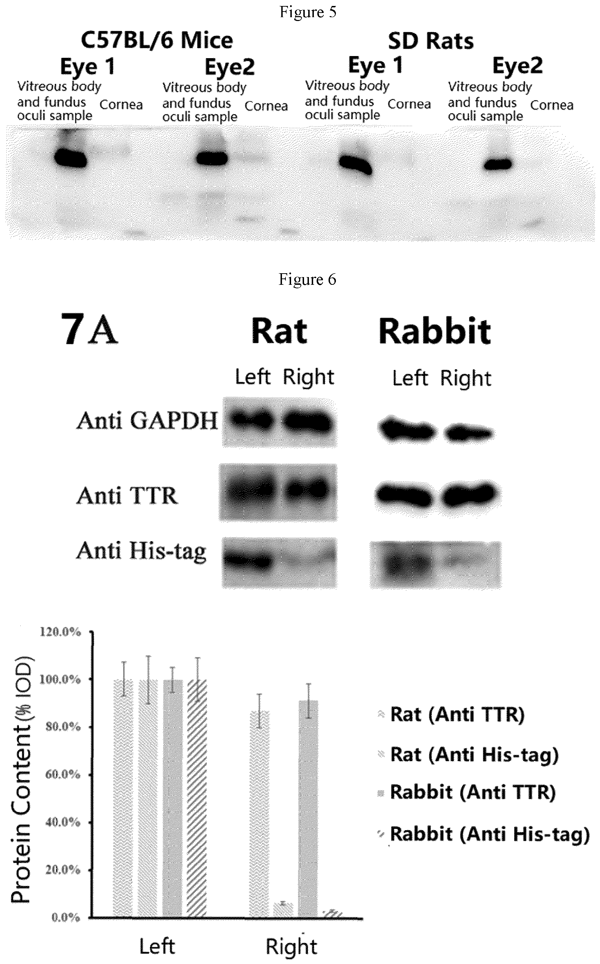

Figure 7A-D shows the western-blot profile of the target protein in vitreous cavity after eye dropping for 2 weeks with human transthyretin and the fusion protein thereof to rats and rabbits, the left eye is dropping eye, and the right eye is control eye. Since the TTRs derived from human/rats/rabbits have high homology, the vitreous body sample has background TTR positive signal detected by anti-TTR antibody after TTR eye dropping; and the positive signal in the dropping eye increases significantly when detected by anti-His-tag antibody, which illustrates that human TTR can effectively enter the eye and reach the vitreous body; and exogenous protein such as GFP, Lysozyme and Ovalbumin can effectively enter the eye after fusion expression with TTR, while proteins described above cannot enter without fusion expression.
Figure 8 shows the retinal leakage and the number of retinal neovascularization of SD rats induced by STZ after eye dropping of TTR.
Figure 9 shows that the eye dropping of TTR prevents the modeling process of ROP.
Figure 10 shows that the eye dropping of TTR inhibits the pathological process of ROP.
Figure 11 shows that the eye dropping of TTR inhibits the pathological process of AMD model.
Figure 12 shows the chemical modification process of human TTR.
Figure 13A shows that the protein structure is TTR dimer, the diclofenac ligand molecule is represented by arrows, and one molecule of TTR dimer can bind to two molecules of diclofenac.
Figure 13B shows the interaction between diclofenac and amino acid residues of TTR.
Figure 14A shows that by molecular simulation with Discovery studio software, it is found that vitamin A1 can bind stably to the hydrophobic passage of a TTR polymer, wherein the protein structure is TTR dimer, and vitamin A1 ligand molecule is represented by arrows, one molecule of TTR dimer can bind to one molecule of vitamin A1.
Figure 14B shows the interaction between vitamin A1 and amino acid residues of TTR.
Figure 15A shows that by molecular simulation with Discovery studio software, it is found that vitamin A2 can bind stably to the hydrophobic passage of a TTR polymer, wherein the protein structure is TTR dimer, vitamin A2 ligand molecule is represented by arrows, and one molecule of TTR dimer can bind to one molecule of vitamin A2.
Figure 15B shows the interaction between vitamin A2 and amino acid residues of TTR.
Figure 16A shows that by molecular simulation with Discovery studio software, it is found that luteoloside can bind stably to the hydrophobic passage of a TTR polymer, wherein the protein structure is TTR dimer, luteoloside ligand molecule is represented by arrows, and one molecule of TTR dimer can bind to one molecule of luteoloside.
Figure 16B shows the interaction between luteoloside and amino acid residues of TTR.
Figure 17A shows that by molecular simulation with Discovery studio software, it is found that sulfamethoxazole can bind stably to the hydrophobic passage of a TTR polymer, wherein the protein structure is TTR dimer, sulfamethoxazole ligand molecule is represented by arrows, and one molecule of TTR dimer can bind to one molecule of sulfamethoxazole.
Figure 17B shows the interaction between sulfamethoxazole and amino acid residues of TTR.

### Detailed description of the preferred embodiment

The following examples further illustrate the present disclosure, but the present disclosure is not limited thereto. In the following examples, the experimental methods without specific conditions are selected according to conventional methods and conditions, or the product specification.

### Part I: Preparation of transthyretin (TTR) and a fusion protein thereof

### Example 1 Preparation of transthyretin

The preparation of transthyretin comprises the following steps:
(1) Construction of recombinant plasmid pET-21a (+)-His-tag-TTR: the His-tag-TTR having the nucleotide sequence of SEQ ID NO: 4 (wherein the amino acid sequence of the TTR used is shown in SEQ ID NO: 1, and pET-21a was purchased from ATCC) was synthesized, and ligated to pET-21a (+) with Nde I and EcoR I, followed by verifying a successful construction by sequencing (the sequencing was performed by Nanjing GenScript Biotechnology Ltd., the same below).
(2) Expression and purification of recombinant TTR: the plasmid pET-21a (+)-His-tag-TTR constructed in step (1) was transformed into *E. coli* BL21 (DE3) cells, and the recombinant *E. coli* BL21 (DE3) cells obtained were cultured in LB medium to prepare an inoculum, then inoculating 5% of the inoculum into 5 L of TB medium, incubating at a temperature of 37°C and paddle speed of 150 rpm until OD₆₀₀ of the culture reaches 1.5-2.0; 0.1-0.5 mM IPTG was added to induce for 8-16 h (Table 1). The bacteria was broken by high pressure homogenization, and TTR was prepared by nickel column affinity adsorption of the supernatant thereof. The endotoxin of the protein obtained was removed by an endotoxin absorption column (Pierce^{™} High Capacity Endotoxin Removal Spin Columns, ThermoFisher) and the residue bacteria was removed by a filter membrane with a pore size of 0.22 µm. The TTR protein yield was determined and the results are shown in Table 1. Wet cells with 17 mg/g and more of protein yield were obtained with induction of 0.3-0.5 mM IPTG for 12-14 h when OD₆₀₀ was 1.5-1.8.

**Table 1 Expression of TTR under different induction conditions**

| (1) The protein yields were obtained by adding different concentrations of IPTG when the bacteria were cultured to an OD₆₀₀ of 1.8 and induced at a temperature of 37°C for 12 h. | | | | | |
|---|---|---|---|---|---|
| IPTG (mM) | 0.1 | 0.2 | 0.3 | 0.4 | 0.5 |
| Protein yield (mg/g wet cells) | 5.4±0.6 | 7.6±0.3 | 20.8±1.6 | 19.4±0.9 | 20.2±1.1 |

| (2) The protein yields were obtained by adding 0.3 mM of IPTG when the bacteria were cultured to different values of OD₆₀₀ values and induced at a temperature of 37°C for 12 h. | | | | | |
|---|---|---|---|---|---|
| OD₆₀₀ | 1.5 | 1.6 | 1.8 | 1.9 | 2.0 |
| Protein yield (mg/g wet cells) | 17.4±1.2 | 19.2±1.5 | 20.8±1.6 | 16.3±1.2 | 14.4±1.3 |

| (3) The protein yields were obtained by adding 0.3 mM of IPTG when the bacteria were cultured to an OD₆₀₀ of 1.8 for different times at a temperature of 37°C. | | | | | |
|---|---|---|---|---|---|
| Induction time (h) | 8 | 10 | 12 | 14 | 16 |
| Protein yield (mg/g wet cells) | 9.3±0.8 | 11.4±1.2 | 20.8±1.6 | 17.3±2.2 | 15.8±1.7 |

### Example 2: Preparation of transthyretin-green fluorescent protein fusion protein

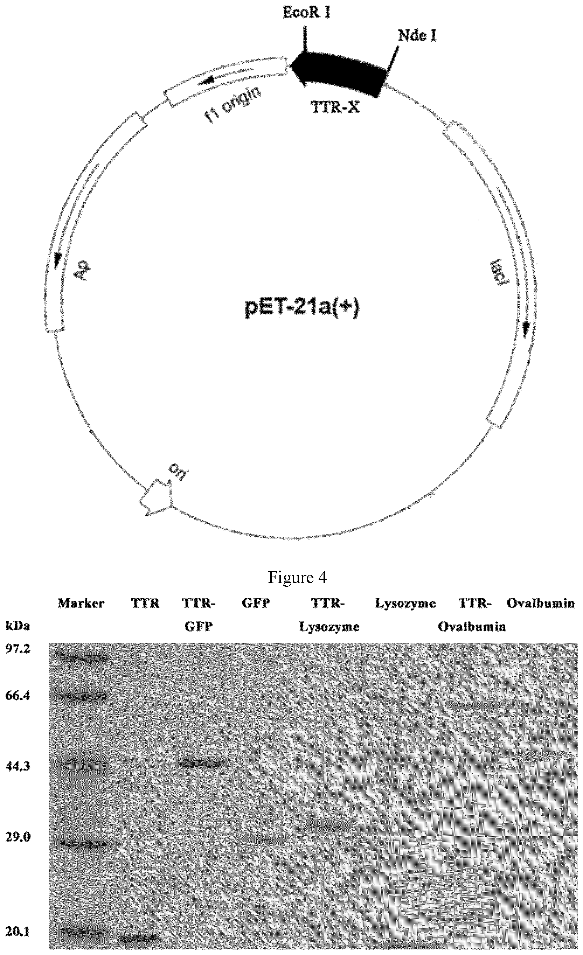

The preparation of the fusion protein comprising transthyretin-green fluorescent protein (TTR-GFP) comprises the following steps:
(1) Construction of recombinant plasmid pET-21a (+)-His-tag-TTR-GFP (the plasmid profile of the following recombinant plasmid pET-21a (+)-His-tag-TTR-X is shown in Figure 4): the His-tag-TTR-GFP having the nucleotide sequence of SEQ ID NO: 5 was synthesized, and ligated to pET-21a (+) with Nde I and EcoR I, followed by verifying a successful construction by sequencing.
(2) Expression and purification of TTR-GFP fusion protein: the recombinant plasmid constructed in step (1) was transformed into *E. coli* BL21 (DE3) cells, and the recombinant *E. coli* BL21 (DE3) cells obtained were cultured in LB medium to prepare an inoculum, then inoculating 5% of the inoculum into 5 L of TB medium, incubating at a temperature of 37°C and paddle speed of 150 rpm until OD₆₀₀ of the culture reaches 1.5-2.0; 0.1-0.5 mM IPTG was added to induce for 8-16 h. The bacteria was broken by high pressure homogenization, and TTR-GFP fusion protein was prepared by nickel column affinity adsorption of the supernatant thereof. The endotoxin of the protein obtained was removed by an endotoxin absorption column (Pierce^{™} High Capacity Endotoxin Removal Spin Columns, ThermoFisher) and the residue bacteria was removed by a filter membrane with a pore size of 0.22 µm. The TTR-GFP protein yield was determined and the results are shown in Table 2. Wet cells with 10 mg/g and more of protein yield were obtained with induction of 0.3-0.5 mM IPTG for 12 h when OD₆₀₀ was 1.5-1.9.

**Table 2 Expression of TTR-GFP under different induction conditions**

| (1) The protein yields were obtained by adding different concentrations of IPTG when the bacteria were cultured to an OD₆₀₀ of 1.8 and induced at a temperature of 37°C for 12 h. | | | | | |
|---|---|---|---|---|---|
| IPTG (mM) | 0.1 | 0.2 | 0.3 | 0.4 | 0.5 |
| Protein yield(mg/g wet cells) | 3.2±0.1 | 8.5±0.6 | 16.4±2.2 | 14.2±1.4 | 10.3±0.7 |

| (2) The protein yields were obtained by adding 0.3 mM of IPTG when the bacteria were cultured to different values of OD₆₀₀ values and induced at a temperature of 37°C for 12 h. | | | | | |
|---|---|---|---|---|---|
| OD₆₀₀ | 1.5 | 1.6 | 1.8 | 1.9 | 2.0 |
| Protein yield (mg/g wet cells) | 10.2±0.9 | 14.3±1.1 | 16.4±2.2 | 11.6±1.8 | 9.6±1.0 |

| (3) The protein yields were obtained by adding 0.3 mM of IPTG when the bacteria were cultured to an OD₆₀₀ of 1.8 for different times at a temperature of 37°C. | | | | | |
|---|---|---|---|---|---|
| Induction time (h) | 8 | 10 | 12 | 14 | 16 |
| Protein yield (mg/g wet cells) | 5.3±0.4 | 7.4±0.5 | 16.4±2.2 | 8.6±1.0 | 6.6±0.5 |

### Example 3: Preparation of transthyretin-hen egg white lysozyme fusion protein

The preparation of the fusion protein comprising transthyretin-hen egg white lysozyme (TTR-Lysozyme) comprises the following steps:
(1) Construction of recombinant plasmid pET-21a (+)-His-tag-TTR-Lysozyme: the His-tag-TTR-Lysozyme having the nucleotide sequence of SEQ ID NO: 6 was synthesized, and ligated to pET-21a (+) with Nde I and EcoR I, followed by verifying a successful construction by sequencing.
(2) Expression and purification of TTR-Lysozyme fusion protein: the recombinant plasmid pET-21a (+)-His-tag-TTR-Lysozyme constructed in step (1) was transformed into *E. coli* BL21 (DE3) cells, and the recombinant *E. coli* BL21 (DE3) cells obtained were cultured in LB medium to prepare an inoculum, then inoculating 5% of the inoculum into 5 L of TB medium, incubating at a temperature of 37°C and paddle speed of 150 rpm until OD₆₀₀ of the culture reaches 1.5-2.0; 0.1-0.5 mM IPTG was added to induce for 8-16 h. The bacteria was broken by high pressure homogenization, and TTR-Lysozyme fusion protein was prepared by nickel column affinity adsorption of the supernatant thereof. The endotoxin of the protein obtained was removed by an endotoxin absorption column (Pierce^{™} High Capacity Endotoxin Removal Spin Columns, ThermoFisher) and the residue bacteria was removed by a filter membrane with a pore size of 0.22 µm. The TTR-Lysozyme protein yield was determined and the results are shown in Table 3.

**Table 3 Expression of TTR-Lysozyme under different induction conditions**

| (1) The protein yields were obtained by adding different concentrations of IPTG when the bacteria were cultured to an OD₆₀₀ of 1.8 and induced at a temperature of 37°C for 12 h. | | | | | |
|---|---|---|---|---|---|
| IPTG (mM) | 0.1 | 0.2 | 0.3 | 0.4 | 0.5 |
| Protein yield (mg/g wet cells) | 8.8±1.3 | 20.1±1.9 | 33.4±4.8 | 21.4±2.6 | 13.3±2.0 |

| (2) The protein yields were obtained by adding 0.3 mM of IPTG when the bacteria were cultured to different values of OD₆₀₀ values and induced at a temperature of 37°C for 12 h. | | | | | |
|---|---|---|---|---|---|
| OD₆₀₀ | 1.5 | 1.6 | 1.8 | 1.9 | 2.0 |
| Protein yield (mg/g wet cells) | 17.3±2.2 | 24.6±3.1 | 33.4±4.8 | 27.2±1.9 | 14.6±1.8 |

| (3) The protein yields were obtained by adding 0.3 mM of IPTG when the bacteria were cultured to an OD₆₀₀ of 1.8 for different times at a temperature of 37°C. | | | | | |
|---|---|---|---|---|---|
| Induction time (h) | 8 | 10 | 12 | 14 | 16 |
| Protein yield (mg/g wet cells) | 20.2±1.4 | 28.8±3.4 | 33.4±4.8 | 30.3±2.7 | 25.5±3.0 |

### Example 4: Preparation of transthyretin-ovalbumin fusion protein

The preparation of the fusion protein comprising transthyretin-ovalbumin (TTR-Ovalbumin) comprises the following steps:
(1) Construction of recombinant plasmid pET-21a (+)-His-tag-TTR-Ovalbumin: the His-tag-TTR-Ovalbumin having the nucleotide sequence of SEQ ID NO: 7 was synthesized, and ligated to pET-21a (+) with Nde I and EcoR I, followed by verifying a successful construction by sequencing.
(2) Expression and purification of TTR-Ovalbumin fusion protein: the recombinant plasmid pET-21a (+)-His-tag-TTR-Ovalbumin constructed in step (1) was transformed into *E. coli* BL21 (DE3) cells, and the recombinant *E. coli* BL21 (DE3) cells obtained were cultured in LB medium to prepare an inoculum, then inoculating 5% of the inoculum into 5 L of TB medium, incubating at a temperature of 37°C and paddle speed of 150 rpm until OD₆₀₀ of the culture reaches 1.5-2.0; 0.1-0.5 mM IPTG was added to induce for 8-16 h. The bacteria was broken by high pressure homogenization, and TTR-Ovalbumin fusion protein was prepared by nickel column affinity adsorption of the supernatant thereof. The endotoxin of the protein obtained was removed by an endotoxin absorption column (Pierce^{™} High Capacity Endotoxin Removal Spin Columns, ThermoFisher) and the residue bacteria was removed by a filter membrane with a pore size of 0.22 µm. The TTR- Ovalbumin protein yield was determined and the results are shown in Table 4.

**Table 4 Expression of TTR-Ovalbumin under different induction conditions**

| (1) The protein yields were obtained by adding different concentrations of IPTG when the bacteria were cultured to an OD₆₀₀ of 1.8 and induced at a temperature of 37°C for 12 h. | | | | | |
|---|---|---|---|---|---|
| IPTG (mM) | 0.1 | 0.2 | 0.3 | 0.4 | 0.5 |
| Protein yield (mg/g wet cells) | 4.2±0.3 | 6.3±0.4 | 9.8±0.7 | 7.3±0.6 | 7.5±1.2 |

| (2) The protein yields were obtained by adding 0.3 mM of IPTG when the bacteria were cultured to different values of OD₆₀₀ values and induced at a temperature of 37°C for 12 h. | | | | | |
|---|---|---|---|---|---|
| OD₆₀₀ | 1.5 | 1.6 | 1.8 | 1.9 | 2.0 |
| Protein yield (mg/g wet cells) | 6.7±0.5 | 8.3±1.1 | 9.8±0.7 | 8.4±1.2 | 7.3±0.6 |

| (3) The protein yields were obtained by adding 0.3 mM of IPTG when the bacteria were cultured to an OD₆₀₀ of 1.8 for different times at a temperature of 37°C. | | | | | |
|---|---|---|---|---|---|
| Induction time (h) | 8 | 10 | 12 | 14 | 16 |
| Protein yield (mg/g wet cells) | 5.5±0.4 | 8.5±1.0 | 9.8±0.7 | 7.4±0.7 | 5.8±0.8 |

Figure 5 shows the electrophoretograms of the expression of transthyretin in *E. coli* BL21 (DE3), the purified product of the fusion protein and standards of green fluorescent protein, hen egg white lysozyme and ovalbumin. It can be illustrated that the proteins described above were correctly expressed.

### Example 5: Recombinant preparation of human transthyretin

(1) Construction of recombinant plasmid pETx-rhaPBAD-*ttr*: plasmid pET-21a (purchased from ATCC) was reformed and reconstructed (the reconstructed plasmid was different from the original plasmid pET-21a at about 75%, and the specific sequence thereof is shown in SEQ ID NO: 8), and T7 promoter was replaced by rhaPBAD promoter (rhamnose inducible), and optimized nucleotide sequence of human TTR (as shown in SEQ ID NO: 2, the amino acid sequence of TTR is shown in SEQ ID NO: 1) was ligated thereto simultaneously. The entire plasmid obtained has a nucleotide sequence of SEQ ID NO: 3. The plasmid was subject to be verified a successful construction by sequencing (the sequencing was performed by Nanjing GenScript Biotechnology Ltd.).
(2) Expression and purification of recombinant human TTR: the plasmid pETx-rhaPBAD-*ttr* constructed in step (1) was transformed into *E. coli* BL21 (DE3) cells, and the recombinant *E. coli* BL21 (DE3) cells obtained were cultured in LB medium to prepare an inoculum, then inoculating 5% of the inoculum into 5 L of TB medium, incubating at a temperature of 37°C and paddle speed of 150 rpm until OD₆₀₀ of the culture reaches 1.5-2.0; 0.4-2% (mass volume percentage) rhamnose was added to induce for 16-20 h (Table 5). The bacteria was broken by high pressure homogenization, and human TTR was prepared by nickel (Ni⁺) column chromatography of the supernatant thereof. The endotoxin of the protein obtained was removed by an endotoxin absorption column (Pierce^{™} High Capacity Endotoxin Removal Spin Columns, ThermoFisher) and the residue bacteria was removed by a filter membrane with a pore size of 0.22 µm. The human TTR protein yield was shown in Table 5. Wet cells with 50 mg/g and more of protein yield were obtained with induction of 1.6-2% rhamnose for 18-19 h when OD₆₀₀ was 1.8-2.0.

**Table 5 Recombinant expression of human TTR**

| (1) The protein yields were obtained by adding different concentrations of rhamnose when the bacteria were cultured to an OD₆₀₀ of 1.8 and induced at a temperature of 37°C for 18 h. | | | | | |
|---|---|---|---|---|---|
| Rhamnose (%) | 0.4 | 0.8 | 1.2 | 1.6 | 2.0 |
| Protein yield (mg/g wet cells) | 22.3±3.6 | 32.5±4.3 | 44.8±5.2 | 50.9±4.8 | 51.8±5.3 |

| (2) The protein yields were obtained by adding 1.6% rhamnose when the bacteria were cultured to different values of OD₆₀₀ values and induced at a temperature of 37°C for 18 h. | | | | | |
|---|---|---|---|---|---|
| OD₆₀₀ | 1.5 | 1.6 | 1.8 | 1.9 | 2.0 |
| Protein yield (mg/g wet cells) | 33.3±3.2 | 38.2±4.1 | 50.9±4.8 | 53.2±4.8 | 54.1±5.3 |

| (3) The protein yields were obtained by adding 1.6% rhamnose when the bacteria were cultured to an OD₆₀₀ of 1.8 for different times at a temperature of 37°C. | | | | | |
|---|---|---|---|---|---|
| Induction time (h) | 16 | 17 | 18 | 19 | 20 |
| Protein yield (mg/g wet cells) | 29.9±2.8 | 37.6±4.2 | 50.9±4.8 | 52.2±5.2 | 45.8±4.7 |

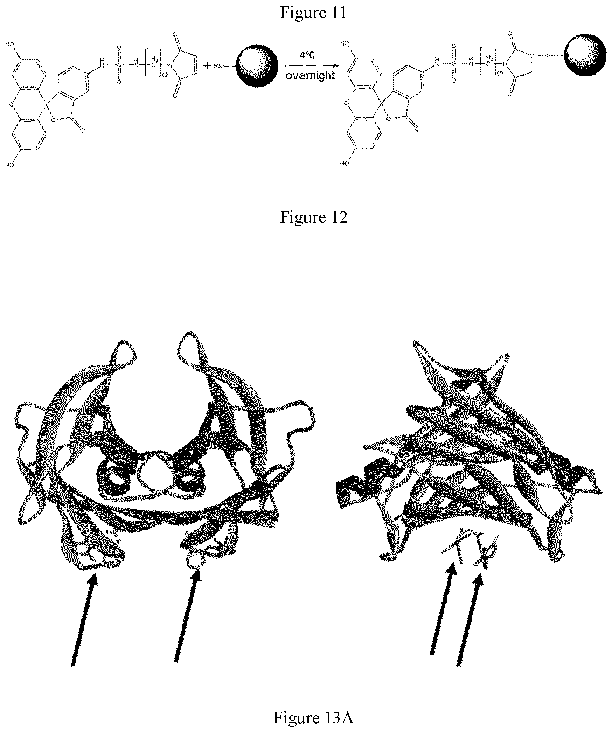

(3) The steps for the expression and purification of recombinant rat TTR and mouse TTR are as described above, except that the optimized nucleotide sequence of human TTR was replaced with the nucleotide sequence of corresponding rat/mouse TTR. Wherein, the amino acid sequence of rat TTR is shown in SEQ ID NO: 9, and the amino acid sequence of mouse TTR is shown in SEQ ID NO: 10.
(4) The protein product of a human mature TTR losing C terminus-TNPKE was obtained according to the steps of recombinant expression described above, and was named human TTR-CL, having the amino acid sequence of SEQ ID NO: 11.
(5) chemical modification of human TTR: a chemical modification group, which is a hydrophobic modification fragment comprising maleiamide, *n*-dodecane and 5-aminofluorescein (Ex 490 nm, Em 520 nm), was designed and synthesized, followed by target chemical modification with the only cysteine (C) residue in the human TTR recombinant expressed and purified as described above. Human TTR reacted with the chemical modification group in a molar ratio of 1:5 (the reaction process is shown in Figure 12) at 4°C with slow shaking. After the reaction was terminated, the remaining chemical modification agent was discarded by ultrafiltration, and TTR was concentrated. The samples were detected by fluorescence spectrometer with excitation at 490 nm and emission at 520 nm, indicating a successful targeting modification at the unique cysteine residue of TTR, which is named human TTR-Modified.

### Example 6: Comparison of nucleotide sequences of human TTR before and after optimization

T7 promoter in the reconstructed plasmid pET-21a as described in Example 5 was replaced with rhaPBAD promoter (rhamnose inducible), followed by ligation to human TTR with an unoptimized nucleotide sequence, thereby obtaining a reconstructed recombinant plasmid pETx-rhaPBAD-ttr (unoptimized), i.e., the only difference between the recombinant plasmid obtained and the recombinant plasmid pETx-rhaPBAD-ttr in Example 5 is whether TTR is optimized. The recombinant human TTR was expressed and purified with the same method described in part (2) of Example 5, and the results are shown in Table 6. It is indicated from the table that TTR proteins are all expressed, and the protein yield of 17 mg/g and more of wet cells can be obtained by inducing with 1.2-2% rhamnose for 17-20 h when OD₆₀₀ was 1.6-2.0, indicating a high protein yield.

**Table 6 Recombinant expression of human TTR (pETx-rhaPBAD-ttr (unoptimized))**

| (1) The protein yields were obtained by adding different concentrations of rhamnose when the bacteria were cultured to an OD₆₀₀ of 1.8 and induced at a temperature of 37°C for 18 h. | | | | | |
|---|---|---|---|---|---|
| Rhamnose (%) | 0.4 | 0.8 | 1.2 | 1.6 | 2.0 |
| Protein yield (mg/g wet cells) | 4.2±0.5 | 10.2±1.0 | 17.1±1.1 | 22.3±2.1 | 22.5±3.2 |

| (2) The protein yields were obtained by adding 1.6% rhamnose when the bacteria were cultured to different values of OD₆₀₀ values and induced at a temperature of 37°C for 18 h. | | | | | |
|---|---|---|---|---|---|
| OD₆₀₀ | 1.5 | 1.6 | 1.8 | 1.9 | 2.0 |
| Protein yield (mg/g wet cells) | 13.2±1.7 | 18.3±1.6 | 22.3±2.1 | 20.4±1.8 | 17.7±0.9 |

| (3) The protein yields were obtained by adding 1.6% rhamnose when the bacteria were cultured to an OD₆₀₀ of 1.8 for different times at a temperature of 37°C. | | | | | |
|---|---|---|---|---|---|
| Induction time (h) | 16 | 17 | 18 | 19 | 20 |
| Protein yield (mg/g wet cells) | 9.6±1.1 | 17.8±2.0 | 22.3±2.1 | 23.4±3.2 | 20.4±1.8 |

### Part II: transthyretin can enter vitreous cavity and fundus oculi through corneal barrier by itself or fusing with a protein

### Example 7: human TTR enters vitreous cavity and fundus oculi through corneal barrier in the manner of eye dropping

(1) The human TTR obtained in Example 5 was prepared as 10 µmol/L (containing saline), and one drop (~30 µL) was administered by eyedropping to C57BL/6 mice (purchased from Shanghai Experimental Animal Research Center, 8 weeks old) and SD rats (purchased from Shanghai Experimental Animal Research Center, 8 weeks old) respectively. The animals were sacrificed after waiting for 3 h, and the cornea, vitreous body and fundus oculi samples (retina and choroid) were taken out, and the proteins were extracted respectively. Since recombinant human TTR has a His-tag label, rabbit anti-His-tag antibody was used as the primary antibody and donkey anti-rabbit antibody was used as the secondary antibody. Western-blot was conducted to determine whether recombinant human TTR was present in the cornea, vitreous body and fundus oculi samples of C57BL/6 mice and SD rats. The results showed that the signal was not significant in proteins extracted from the corneal tissue, while the signals in vitreous body and fundus oculi samples were significant (Figure 6), indicating that by the manner of eye dropping, TTR can enter the vitreous body and fundus oculi through the corneal barrier.
(2) The human TTR obtained in Example 5 was prepared as 5-30 µmol/L (containing saline, and 0-6% hyaluronic acid with low molecular weight), and was administered by eye dropping to C57BL/6 mice (8 weeks old) and SD rats (8 weeks old), respectively. The animals were sacrificed after treatment for 3-72 h, and the proteins were extracted from the vitreous body and fundus oculi samples. Rabbit anti-His-tag antibody was used as the primary antibody, and donkey anti-rabbit antibody was used as the secondary antibody for ELISA to determine the content of human TTR in vitreous body and fundus oculi samples of C57BL/6 mice and SD rats. The results show that when the content of TTR in the eye drops was 10-15 µmol/L, the content of human TTR in the vitreous body and fundus oculi samples reached the peak 3 h after eye dropping; when the concentration of TTR reached 15 µmol/L, further increased concentration of TTR has little effect on increasing the concentration of human TTR in the vitreous body and fundus oculi samples; when the content of hyaluronic acid in the eye drops was 2%, the content of human TTR in the vitreous body and fundus oculi samples reached the peak 3 h after eye dropping; after eye dropping, the content half-life of human TTR in the vitreous body and fundus oculi samples of C57BL/6 mice and SD rats was close to 60 h, indicating that it can effectively exist in the vitreous body and fundus oculi samples for 60 h, with a sufficient therapeutic concentration and therapeutic duration (Table 7-1).

**Table 7-1 Human TTR entering the vitreous cavity and fundus oculi through the corneal barrier of C57BL/6 mice and SD rats /**

| (1) The human TTR content in vitreous body and fundus oculi 3 h after eye dropping using human recombinant TTR (in saline solution). | | | | | | |
|---|---|---|---|---|---|---|
| Concentration of TTR drops(µmol/L) | 5 | 10 | 15 | 20 | 25 | 30 |
| Concentration of human TTR in vitreous body and fundus oculi samples of C57BL/6 mice (µmol/L) | 0.84± 0.07 | 1.81± 0.19 | 1.75± 0.20 | 1.83± 0.21 | 1.88± 0.17 | 1.87± 0.20 |
| Concentration of human TTR in vitreous body and fundus oculi samples of SD rats (µmol/L) | 1.14± 0.11 | 2.43± 0.21 | 2.37± 0.30 | 2.42± 0.36 | 2.45± 0.42 | 2.44± 0.36 |

| (2) The human TTR content in vitreous body and fundus oculi 3 h after eye dropping using human recombinant TTR (10 (µmol/L) (in saline solution added with hyaluronic acid of different concentration). | | | | | | |
|---|---|---|---|---|---|---|
| Concentration of hyaluronic acid (%) | 0 | 1 | 2 | 4 | 6 | |
| Concentration of human TTR in vitreous body and fundus oculi samples of C57BL/6 mice (µmol/L) | 1.81± 0.19 | 2.07± 0.16 | 2.77± 0.16 | 2.13± 0.20 | 1.47±0.11 | |
| Concentration of human TTR in vitreous body and fundus oculi samples of SD rats (µmol/L) | 2.43± 0.21 | 2.74± 0.32 | 3.11± 0.31 | 3.09± 0.29 | 3.12±0.34 | |

| (3) The peak of the human TTR content in vitreous body and fundus oculi was reached 3 h after eye dropping using human recombinant TTR (10 µmol/L, comprising saline solution and 2% hyaluronic acid), and the human TTR content thereof at different times thereafter are shown as below | | | | | | |
|---|---|---|---|---|---|---|
| Sampling time after eyedropping (h) | 3 | 18 | 36 | 60 | 72 | |
| Concentration of human TTR in vitreous body and fundus oculi samples of C57BL/6 mice (µmol/L) | 2.77± 0.16 | 2.01± 0.22 | 1.73± 0.13 | 1.40± 0.11 | 0.87±0.10 | |
| Concentration of human TTR in vitreous body and fundus oculi samples of SD rats (µmol/L) | 3.11± 0.31 | 2.74± 0.26 | 1.98± 0.22 | 1.54± 0.17 | 1.02±0.06 | |

(3) The human TTR and human TTR-Modified obtained in Example 5 was prepared as 10 µmol/L (containing saline, and 2% hyaluronic acid with low molecular weight), and was administered by eyedropping to C57BL/6 mice (8 weeks old) and SD rats (8 weeks old) respectively. The animals were sacrificed after treatment for 3-72 h, and the proteins were extracted from the vitreous body and fundus oculi samples (retina and choroid). Rabbit anti-His-tag antibody was used as the primary antibody, and donkey anti-rabbit antibody was used as the secondary antibody for ELISA to determine the content of human TTR in vitreous body and fundus oculi samples of C57BL/6 mice and SD rats. The results show that the efficiency of the human TTR-Modified modified by long hydrophobic fragment entering the vitreous body and fundus oculi is significantly higher than that of unmodified human TTR (Table 7-2).

**Table 7-2 Comparison of efficiency of human TTR and human TTR-Modified entering the vitreous cavity and fundus oculi**

| | | | | | |
|---|---|---|---|---|---|
| The contents of human TTR and human TTR-Modified in vitreous body and fundus oculi reached the peak 3 h after eyedropping human recombinant TTR and human TTR-Modified (10 (µmol/L) (in saline solution added with 2% hyaluronic acid), and the contents thereof at different times thereafter | | | | | |
| Sampling time after eyedropping (h) | 3 | 18 | 36 | 60 | 72 |
| Concentration of human TTR in vitreous body and fundus oculi samples of C57BL/6 mice (µmol/L) | 2.77± 0.16 | 2.01± 0.22 | 1.73± 0.13 | 1.40± 0.11 | 0.87± 0.10 |
| Concentration of human TTR-Modified in vitreous body and fundus oculi samples of C57BL/6 mice (µmol/L) | 3.62± 0.33 | 2.86± 0.27 | 2.11± 0.19 | 1.89± 0.17 | 1.14± 0.10 |
| Concentration of human TTR in vitreous body and fundus oculi samples of SD rats (µmol/L) | 3.11± 0.31 | 2.74± 0.26 | 1.98± 0.22 | 1.54± 0.17 | 1.02± 0.06 |
| Concentration of human TTR-Modified in vitreous body and fundus oculi samples of SD rats (µmol/L) | 3.94± 0.41 | 3.16± 0.34 | 2.44± 0.24 | 2.07± 0.18 | 1.44± 0.13 |

### Example 8: TTR enters the vitreous cavity through the corneal barrier by means of eye dropping

The purified TTR protein (at a concentration of 4 µmol/L) prepared in Example 1 after removal of endotoxin and bacteria was subject to treating healthy SD rats (*rattus norregicus*) (6 weeks old) and New Zealand big-eared rabbits *(Oryctolagus cuniculus)* (2 months old, about 2.5 kg) by means of eye dropping, the left eye was the sample eye dropped with the protein, and the right eye was the blank control eye dropped with saline. The protein was administered 1-3 times per day at a dosage of 0.4-0.8 nmol. Two weeks later, the eyeball was removed and separated to obtain the vitreous body sample. It was preliminarily verified by western-blot method (Figure 7A) that TTR can enter the vitreous cavity from the ocular surface. The western-blot results with Anti-His tag antibody as the primary antibody show that the signal intensity of the exogenous TTR in the vitreous body of the left eye is 28.7 times that of the right eye in SD rats, and the signal intensity of the exogenous TTR in the vitreous body of the left eye is 35.6 times that of the right eye in New Zealand big-ear rabbits; the content of TTR in the vitreous body sample was determined by ELISA with the anti-His tag antibody as the primary antibody (Table 8 and 9). The results show that after dropping at the content of 0.6 nmol twice a day, higher levels of content of exogenous TTR were detected in the vitreous body of SD rats and New Zealand big-ear rabbits.

In addition, Figure 3 shows that the sequence similarity of human/rat/mouse/rabbit-derived transthyretin reaches nearly 95% by homology alignment, indicating that the background positive signal of the TTR in Figure 7A is intraocular homologous protein signal.

**Table 8 Effects of TTR entering the eye of SD rats in different treatment manners**

| (1) Treatment: 0.6 nmol TTR drops in left eye every time, with different times for dropping | | | | |
|---|---|---|---|---|
| times for dropping per day | | 1 | 2 | 3 |
| TTR content in vitreous body (nmol/L) | Day 2 | 23.2±1.8 | 40.3±5.2 | 41.1±4.8 |
| | Day 6 | 48.9±5.4 | 69.7±7.4 | 70.6±7.6 |
| | Day 10 | 61.2±7.3 | 85.3±7.5 | 89.2±9.3 |
| | Day 14 | 70.8±6.9 | 103.2±9.9 | 107.4±10.1 |

| (2) Treatment: drops twice every day in left eye with different content each time | | | | |
|---|---|---|---|---|
| Content for each drops (nmol) | | 0.4 | 0.6 | 0.8 |
| TTR content in vitreous body (nmol/L) | Day 2 | 20.7±2.3 | 40.3±5.2 | 42.8±4.8 |
| | Day 6 | 41.5±5.2 | 69.7±7.4 | 70.2±5.9 |
| | Day 10 | 57.6±4.9 | 85.3±7.5 | 87.9±9.2 |
| | Day 14 | 66.6±7.1 | 103.2±9.9 | 107.7±10.3 |

**Table 9 Effects of TTR entering the eye of New Zealand big-ear rabbits in different treatment manners**

| (1) Treatment: 0.6 nmol TTR drops in left eye every time, with different times for dropping | | | | |
|---|---|---|---|---|
| times for dropping per day | | 1 | 2 | 3 |
| TTR content in vitreous body | Day 2 | 31.5±4.2 | 49.5±4.5 | 50.2±4.3 |
| (nmol/L) | Day 6 | 51.7±6.3 | 76.3±6.2 | 76.7±7.1 |
| | Day 10 | 82.6±7.4 | 97.6±8.7 | 99.2±8.6 |
| | Day 14 | 89.6±8.8 | 112.3±9.5 | 113.2±10.6 |

| (2) Treatment: drops twice every day in left eye with different content each time | | | | |
|---|---|---|---|---|
| Content for each drops (nmol) | | 0.4 | 0.6 | 0.8 |
| TTR content in vitreous body (nmol/L) | Day 2 | 36.6±3.5 | 49.5±4.5 | 51.1±4.6 |
| | Day 6 | 53.4±5.7 | 76.3±6.2 | 79.2±6.3 |
| | Day 10 | 81.6±7.2 | 97.6±8.7 | 101.2±9.8 |
| | Day 14 | 89.8±8.3 | 112.3±9.5 | 115.4±10.6 |

### Example 9: TTR-GFP fusion protein enters the vitreous cavity through the corneal barrier by means of eye dropping

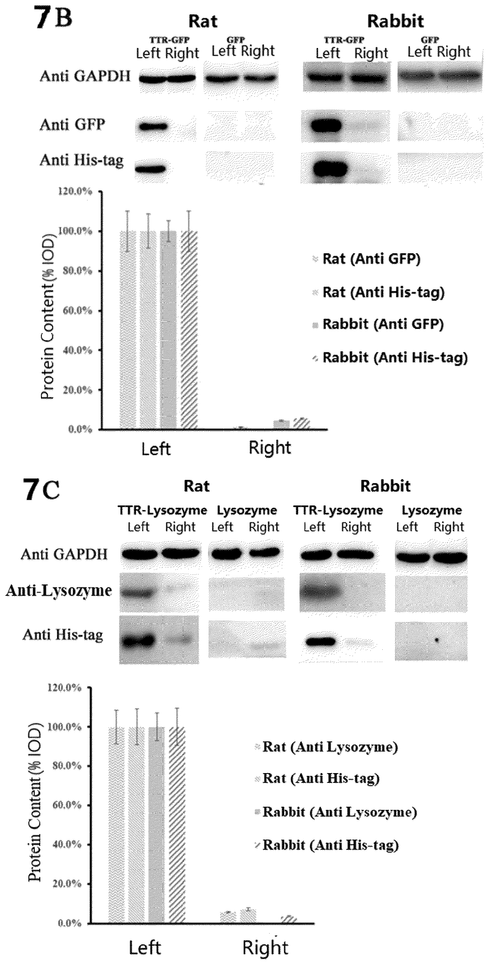

The purified TTR-GFP protein (at a concentration of 4 µmol/L) prepared in Example 2 after removal of endotoxin and bacteria was subject to treating healthy SD rats *(rattus norregicus*) (6 weeks old) and New Zealand big-eared rabbits *(Oryctolagus cuniculus)* (2 months old, about 2.5 kg) by means of eye dropping, the left eye was the sample eye dropped with the protein, and the right eye was the blank control eye dropped with saline. The protein was administered 1-3 times per day at a dosage of 0.4-0.8 nmol. Two weeks later, the eyeball was removed and separated to obtain the vitreous body sample. It was preliminarily verified by western-blot method (Figure 7B) that TTR-GFP can enter the vitreous cavity from the ocular surface. The western-blot results with Anti-GFP antibody as the primary antibody show that the signal intensity of the exogenous GFP in the vitreous body of the left eye is 62.3 times that of the right eye in SD rats, and the signal intensity of the exogenous GFP in the vitreous body of the left eye is 47.6 times that of the right eye in New Zealand big-ear rabbits; the western-blot results with Anti-His tag antibody as the primary antibody show that the signal intensity of the exogenous TTR in the vitreous body of the left eye was strong while there was no signal in that of the right eye in SD rats, and the signal intensity of the exogenous TTR in the vitreous body of the left eye is 45.4 times that of the right eye in New Zealand big-ear rabbits. The content of TTR-GFP in the vitreous body sample was determined by ELISA with the anti-His tag antibody as the primary antibody (Table 10 and 11). The results show that after dropping at the content of 0.6 nmol twice a day, higher levels of content of exogenous TTR-GFP were detected in the vitreous body of SD rats and New Zealand big-ear rabbits.

**Table 10 Effects of TTR-GFP entering the eye of SD rats in different treatment manners**

| (1) Treatment: 0.6 nmol TTR-GFP drops in left eye every time, with different times for dropping | | | | |
|---|---|---|---|---|
| times for dropping per day | | 1 | 2 | 3 |
| TTR-GFP content in vitreous body (nmol/L) | Day 2 | 18.7±1.3 | 32.2±2.7 | 33.3±3.1 |
| | Day 6 | 33.2±2.8 | 51.6±4.9 | 54.3±6.2 |
| | Day 10 | 40.2±3.6 | 70.3±6.4 | 74.2±6.9 |
| | Day 14 | 47.8±5.1 | 89.9±8.2 | 91.3±8.7 |

| (2) Treatment: drops twice every day in left eye with different content each time | | | | |
|---|---|---|---|---|
| Content for each drops (nmol) | | 0.4 | 0.6 | 0.8 |
| TTR-GFP content in vitreous body (nmol/L) | Day 2 | 11.6±1.7 | 32.2±2.7 | 34.6±4.0 |
| | Day 6 | 28.8±3.4 | 51.6±4.9 | 54.2±4.9 |
| | Day 10 | 46.7±5.2 | 70.3±6.4 | 73.4±7.1 |
| | Day 14 | 55.7±6.1 | 89.9±8.2 | 92.2±10.4 |

**Table 11 Effects of TTR-GFP entering the eye of New Zealand big-ear rabbits in different treatment manners**

| (1) Treatment: 0.6 nmol TTR-GFP drops in left eye every time, with different times for dropping | | | | |
|---|---|---|---|---|
| times for dropping per day | | 1 | 2 | 3 |
| TTR-GFP content in vitreous body (nmol/L) | Day 2 | 25.3±2.4 | 42.4±3.8 | 43.5±4.2 |
| | Day 6 | 40.1±3.8 | 61.3±5.4 | 64.1±5.7 |
| | Day 10 | 58.3±4.6 | 77.6±6.8 | 79.3±7.5 |
| | Day 14 | 72.2±7.3 | 90.2±8.7 | 94.3±8.6 |

| (2) Treatment: drops twice every day in left eye with different content each time | | | | |
|---|---|---|---|---|
| Content for each drops (nmol) | | 0.4 | 0.6 | 0.8 |
| TTR-GFP content in vitreous | Day 2 | 36.1±3.2 | 42.4±3.8 | 44.1±4.5 |
| body (nmol/L) | Day 6 | 49.9±5.6 | 61.3±5.4 | 62.3±5.8 |
| | Day 10 | 57.8±5.7 | 77.6±6.8 | 79.2±7.0 |
| | Day 14 | 72.4±6.6 | 90.2±8.7 | 91.3±9.2 |

### Example 10: TTR-Lysozyme fusion protein enters the vitreous cavity through the corneal barrier by means of eye dropping

The purified TTR-Lysozyme protein (at a concentration of 4 µmol/L) prepared in Example 3 after removal of endotoxin and bacteria was subject to treating healthy SD rats *(rattus norregicus*) (6 weeks old) and New Zealand big-eared rabbits *(Oryctolagus cuniculus)* (2 months old, about 2.5 kg) by means of eye dropping, the left eye was the sample eye dropped with the protein, and the right eye was the blank control eye dropped with saline. The protein was administered 1-3 times per day at a dosage of 0.4-0.8 nmol. Two weeks later, the eyeball was removed and separated to obtain the vitreous body sample. It was preliminarily verified by western-blot method (Figure 7C) that TTR-Lysozyme can enter the vitreous cavity from the ocular surface. The western-blot results with Anti-Lysozyme antibody as the primary antibody show that the signal intensity of the exogenous Lysozyme in the vitreous body of the left eye is 34.6 times that of the right eye in SD rats, and the signal intensity of the exogenous Lysozyme in the vitreous body of the left eye is strong while there is no signal in that of the right eye in New Zealand big-ear rabbits; the western-blot results with Anti-His tag antibody as the primary antibody show that the signal intensity of the exogenous TTR in the vitreous body of the left eye was 30.2 times that of the right eye in SD rats, and the signal intensity of the exogenous TTR in the vitreous body of the left eye is 46.3 times that of the right eye in New Zealand big-ear rabbits. The content of TTR-Lysozyme in the vitreous body sample was determined by ELISA with the anti-His tag antibody as the primary antibody (Table 12 and 13). The results show that after dropping at the content of 0.6 nmol twice a day, higher levels of content of exogenous TTR-Lysozyme were detected in the vitreous body of SD rats and New Zealand big-ear rabbits.

**Table 12 Effects of TTR-Lysozyme entering the eye of SD rats in different treatment manners**

| (1) Treatment: 0.6 nmol TTR-Lysozyme drops in left eye every time, with different times for dropping | | | | |
|---|---|---|---|---|
| times for dropping per day | | 1 | 2 | 3 |
| TTR-Lysozyme content in vitreous body (nmol/L) | Day 2 | 22.1±1.7 | 37.8±3.2 | 39.6±4.2 |
| | Day 6 | 39.8±3.4 | 59.2±5.3 | 61.7±5.6 |
| | Day 10 | 49.9±5.1 | 75.6±6.9 | 80.5±5.9 |
| | Day 14 | 55.2±5.5 | 96.4±8.8 | 99.4±10.3 |

| (2) Treatment: drops twice every day in left eye with different content each time | | | | |
|---|---|---|---|---|
| Content for each drops (nmol) | | 0.4 | 0.6 | 0.8 |
| TTR-Lysozyme content in vitreous body (nmol/L) | Day 2 | 17.7±1.5 | 37.8±3.2 | 39.7±5.0 |
| | Day 6 | 36.5±3.1 | 59.2±5.3 | 66.3±5.7 |
| | Day 10 | 50.2±4.8 | 75.6±6.9 | 79.9±7.2 |
| | Day 14 | 60.3±5.4 | 96.4±8.8 | 100.4±9.6 |

**Table 13 Effects of TTR-Lysozyme entering the eye of New Zealand big-ear rabbits in different treatment manners**

| (1) Treatment: 0.6 nmol TTR-Lysozyme drops in left eye every time, with different times for dropping | | | | |
|---|---|---|---|---|
| times for dropping per day | | 1 | 2 | 3 |
| TTR-Lysozyme content in vitreous body (nmol/L) | Day 2 | 29.7±3.1 | 49.2±4.6 | 50.8±5.3 |
| | Day 6 | 48.6±5.3 | 63.4±5.8 | 65.4±6.3 |
| | Day 10 | 61.2±5.6 | 85.7±7.8 | 87.9±9.0 |
| | Day 14 | 70.4±6.7 | 107.2±9.6 | 110.2±10.4 |

| (2) Treatment: drops twice every day in left eye with different content each time | | | | |
|---|---|---|---|---|
| Content for each drops (nmol) | | 0.4 | 0.6 | 0.8 |
| TTR-Lysozyme content in vitreous body (nmol/L) | Day 2 | 36.1±3.2 | 49.2±4.6 | 51.8±4.7 |
| | Day 6 | 49.4±5.0 | 63.4±5.8 | 67.5±6.2 |
| | Day 10 | 70.3±6.5 | 85.7±7.8 | 89.9±9.3 |
| | Day 14 | 79.6±6.2 | 107.2±9.6 | 110.8±12.1 |

### Example 11: TTR-Ovalbumin fusion protein enters the vitreous cavity through the corneal barrier by means of eye dropping

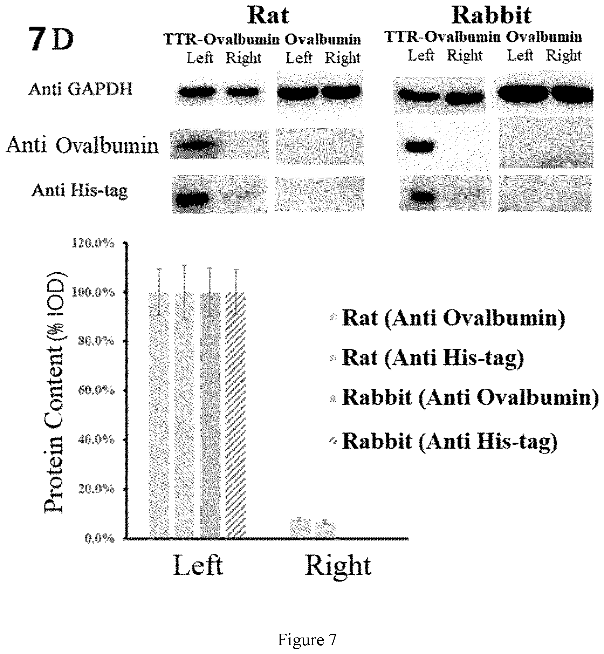

The purified TTR-Ovalbumin protein (at a concentration of 4 µmol/L) prepared in Example 4 after removal of endotoxin and bacteria was subject to treating healthy SD rats *(rattus norregicus*) (6 weeks old) and New Zealand big-eared rabbits *(Oryctolagus cuniculus)* (2 months old, about 2.5 kg) by means of eye dropping, the left eye was the sample eye dropped with the protein, and the right eye was the blank control eye dropped with saline. The protein was administered 1-3 times per day at a dosage of 0.4-0.8 nmol. Two weeks later, the eyeball was removed and separated to obtain the vitreous body sample. It was preliminarily verified by western-blot method (Figure 7D) that TTR-Ovalbumin can enter the vitreous cavity from the ocular surface. The western-blot results with Anti-Ovalbumin antibody as the primary antibody show that the signal intensity of the exogenous Ovalbumin in the vitreous body of the left eye is 25.3 times that of the right eye in SD rats, and the signal intensity of the exogenous Ovalbumin in the vitreous body of the left eye is strong while there is no signal in that of the right eye in New Zealand big-ear rabbits; the western-blot results with Anti-His tag antibody as the primary antibody show that the signal intensity of the exogenous TTR in the vitreous body of the left eye is 37.8 times that of the right eye in SD rats, and the signal intensity of the exogenous Ovalbumin in the vitreous body of the left eye is strong while there is no signal in that of the right eye in New Zealand big-ear rabbits. The content of TTR-Ovalbumin in the vitreous body sample was determined by ELISA with the anti-His tag antibody as the primary antibody (Table 14 and 15). The results show that after dropping at the content of 0.6 nmol twice a day, higher levels of content of exogenous TTR-Ovalbumin were detected in the vitreous body of SD rats and New Zealand big-ear rabbits.

**Table 14 Effects of TTR-Ovalbumin entering the eye of SD rats in different treatment manners**

| (1) Treatment: 0.6 nmol TTR-Ovalbumin drops in left eye every time, with different times for dropping | | | | |
|---|---|---|---|---|
| times for dropping per day | | 1 | 2 | 3 |
| TTR-Ovalbumin content in vitreous body (nmol/L) | Day 2 | 14.4±1.2 | 27.7±1.9 | 28.9±3.0 |
| | Day 6 | 24.3±2.1 | 39.4±3.2 | 41.3±3.5 |
| | Day 10 | 30.5±2.9 | 57.8±4.9 | 60.4±5.7 |
| | Day 14 | 36.7±3.5 | 70.2±6.4 | 73.6±8.1 |

| (2) Treatment: drops twice every day in left eye with different content each time | | | | |
|---|---|---|---|---|
| Content for each drops (nmol) | | 0.4 | 0.6 | 0.8 |
| TTR-Ovalbumin content in vitreous body (nmol/L) | Day 2 | 9.5±0.7 | 27.7±1.9 | 29.2±2.6 |
| | Day 6 | 20.2±1.9 | 39.4±3.2 | 43.1±3.0 |
| | Day 10 | 31.7±3.4 | 57.8±4.9 | 59.8±6.2 |
| | Day 14 | 43.5±4.1 | 70.2±6.4 | 74.5±6.3 |

**Table 15 Effects of TTR-Ovalbumin entering the eye of New Zealand big-ear rabbits in different treatment manners**

| (1) Treatment: 0.6 nmol TTR-Ovalbumin drops in left eye every time, with different times for dropping | | | | |
|---|---|---|---|---|
| times for dropping per day | | 1 | 2 | 3 |
| TTR-Ovalbumin content in vitreous body (nmol/L) | Day 2 | 23.9±2.1 | 31.2±2.8 | 33.6±3.7 |
| | Day 6 | 30.5±3.2 | 44.7±5.0 | 47.2±4.5 |
| | Day 10 | 47.5±4.6 | 61.3±5.7 | 65.6±5.9 |
| | Day 14 | 59.9±6.1 | 75.4±8.0 | 77.3±8.0 |

| (2) Treatment: drops twice every day in left eye with different content each time | | | | |
|---|---|---|---|---|
| Content for each drops (nmol) | | 0.4 | 0.6 | 0.8 |
| TTR-Ovalbumin content in vitreous body (nmol/L) | Day 2 | 19.8±2.4 | 31.2±2.8 | 34.3±3.6 |
| | Day 6 | 34.2±2.9 | 44.7±5.0 | 46.5±4.5 |
| | Day 10 | 47.6±5.1 | 61.3±5.7 | 65.1±6.4 |
| | Day 14 | 58.7±6.2 | 75.4±8.0 | 78.7±8.0 |

### Part III: Treating ocular diseases such as DR with TTR

### Example 12: treating DR (diabetic retinopathy) SD rats with human TTR in the manner of eye dropping

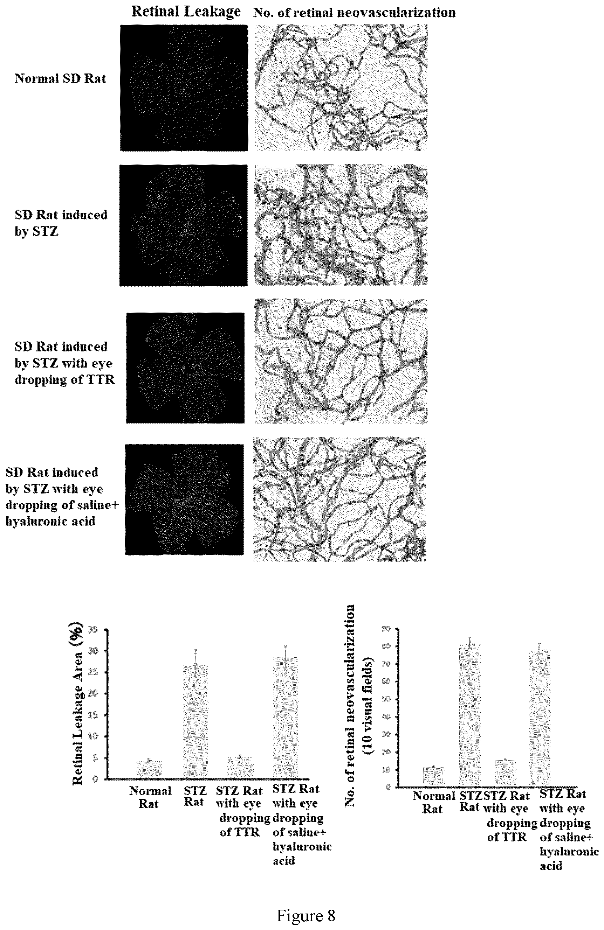

8-week-old SD rats weighing 200-250 g was fasted for 12-18 h, and treated with intraperitoneally injection of 2% STZ (60 mg/kg), followed by cutting the tail and collecting blood thereof after 48 h and 72 h. The blood glucose was detected higher than 16.7 mM by test paper, indicating that the model was successfully established, and DR SD rats were obtained. DR SD rats were divided into 2 large groups, one is DR SD rats without any treatment (5 rats), and the other is the human TTR eye dropping group (25 rats) dropped with the human TTR prepared in Example 5, each left eye dropped with human TTR (5-20 µmol/L) (normal saline+2% hyaluronic acid) twice a day, and each right eye dropped with saline+2% hyaluronic acid; in addition, another group of normal SD rats (No eye dropping) served as a normal control group (5 rats). All SD rats were fed for 3 months, followed by stripping the retina for staining with Evans Blue (EB) to observe the retinal vascular leakage, for Trypsin enzymatic hydrolysis to observe the neovascularization density; the results show that compared with the normal control, retinal vascular leakage and the neovascularization number were significantly increased after feeding STZ induced SD rats for 3 months, while the eyeball retinal leakage condition of SD rats treated with human TTR was significantly inhibited, and the number of retinal neovascularization was reduced significantly (Figure 8), indicating the clinical pathological condition of DR had been alleviated. Wherein, the treatment of administering 10 µmol/L human TTR (saline+2% hyaluronic acid) per day has the best effect (Table 16).

**Table 16 DR pathological condition of SD rats induced by STZ treated with human TTR**

| The DR pathological condition of SD rats induced by STZ after 3 months of eyedropping treatment using human recombinant TTR (saline solution+2% hyaluronic acid) | | | | | |
|---|---|---|---|---|---|
| Concentration of TTR for eyedropping (µmol/L) | 0 | 5 | 10 | 15 | 20 |
| Retinal leakage area (%) | 29.4±3.1 | 6.2±0.5 | 5.5±0.4 | 5.6±0.5 | 5.3±0.5 |
| No. of retinal neovascularization (10 visual fields) | 77±6 | 14±2 | 11±1 | 13±2 | 12±1 |

### Example 13: treating ROP (retinopathy of prematurity) SD rats with human TTR in the manner of eye dropping

One-week-old SD suckling rats were fed in hyperbaric oxygen chamber, and the normal control group was fed in normal environment (normal control group, 6 rats). In hyperbaric oxygen chamber, one group of suckling rats were treated with eye dropping of 5-20 µmol/L TTR prepared in Example 5 (saline+2% hyaluronic acid), once per day, each drop of 30 µL (ROP/TTR (modeling) group, 6 rats); the other groups of suckling rats was in hyperbaric oxygen chamber without any treatment (ROP group, 24 rats); after fed for 5 days in hyperbaric oxygen chamber, all suckling rats were transferred from hyperbaric oxygen chamber to normal environment for feeding, and one subgroup was separated from ROP group for eye dropping with 5-20 µmol/L TTR prepared in Example 5 (saline+2% hyaluronic acid), once per day, each drop of 30 µL (ROP/TTR (model) group); other suckling rats in ROP group served as control without any treatment; the suckling rats were sacrificed after feeding for 5 days in normal environment, and the retina was stripped for staining with EB.

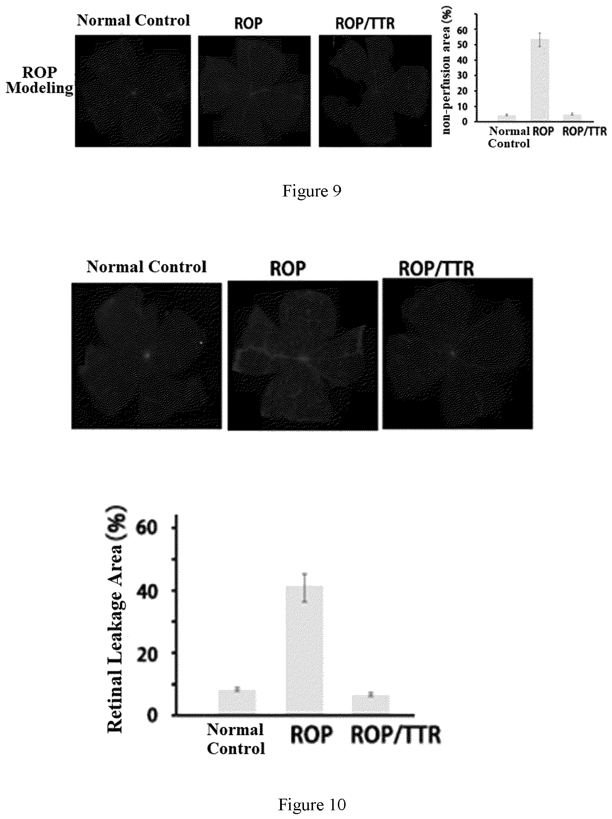

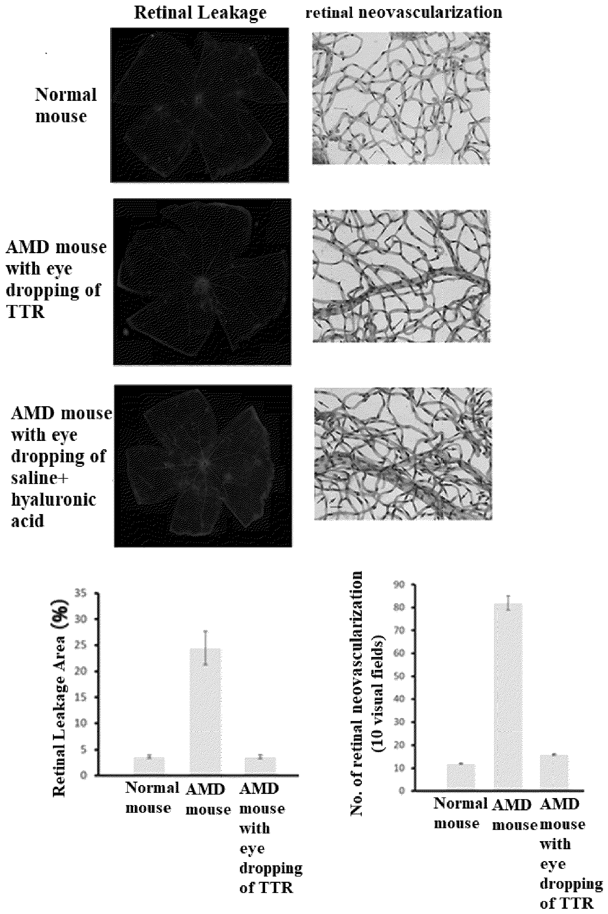

The results are shown in Figure 9. It is illustrated that in the modeling process, the retina stained with EB of normal control group has normal morphology; ROP group presents obvious no-perfusion area and neovascularization; while modeling, no obvious no-perfusion area and abnormal neovascularization were formed in ROP/TTR (modeling) group treated with eye dropping of TTR, indicating that TTR has protective effect on normal vessels under anoxic condition, and has inhibitory effect on neovascularization (Figure 9).

The model ROP suckling rats were treated with eye dropping of TTR (10 µmol/L), 5 days. Compare ROP and ROP/TTR (model) groups, and in the late stage of experiment (5 days), a large number of leakage areas appeared when abnormal neovascularization covered the retina in ROP group, and eye dropping of TTR could reverse this trend (Figure 10).

The model ROP suckling rats were treated with eye dropping of different concentrations of TTR (5-20 µmol/L), 5 days. In the late stage of experiment (5 days), a large number of leakage areas appeared when abnormal neovascularization covered the retina in ROP control group, and eye dropping of TTR could reverse this trend, wherein the dosage of 10 µmol/L TTR has the best effect (Table 17).

**Table 17 ROP pathological condition of SD suckling rats induced by hyperbaric oxygen chamber treated with human TTR**

| The pathological condition of SD suckling rats induced by hyperbaric oxygen chamber after 5 days of eyedropping treatment using human recombinant TTR (saline solution+2% hyaluronic acid). | | | | | |
|---|---|---|---|---|---|
| Concentration of TTR for eyedropping (µmol/L) | 0 | 5 | 10 | 15 | 20 |
| Retinal leakage area (%) | 44.7±5.1 | 9.8±1.0 | 3.4±0.2 | 3.2±0.2 | 3.3±0.2 |

### Example 14: treating AMD (age-related macular degeneration) C57BL/6 mice with human TTR in the manner of eye dropping

9-week-old C57BL/6 mice were subject to retinal photocoagulation by krypton laser (647 nm), with a power of 360 mW, a diameter of 50 µm, a time of 0.05 s, 8 photocoagulation site for each eye, for inducing the neovascularization in choroid, and gradually proceeding to retinal hyperplasia to obtain AMD C57BL/6 mice. AMD C57BL/6 mice were divided into a dropping group and a non-dropping group. The dropping group has 14 mice treating with eye dropping of TTR, with the right eye of 5-20 µmol/L TTR prepared in Example 5 (saline+2% hyaluronic acid) twice per day, 30 µL each time, the left eye of saline +2% hyaluronic acid as control; the non-dropping group has 6 mice without any treatment; there was another group (2 mice, without eye dropping) without laser radiation as normal control group; the animals were sacrificed after eye dropping for 2 weeks, and the retina was stripped for EB staining to observe the retinal leakage conditions, and for Trypsin enzymatic hydrolysis to observe the density of neovascularization.

The results are shown in Figure 11. It is illustrated that the retina of normal control group has normal morphology in EB staining; AMD group has significant retinal leakage and neovascularization; the retinal leakage conditions and neovascularization conditions of the AMD/TTR group treated with 10 µmol/L TTR have achieved significant alleviation (Figure 11).

The modeled AMD mice were treated with eye dropping of different concentrations of TTR (5-20 µmol/L), 2 weeks. More leakage areas appeared, and the number of neovascularization increased significantly in AMD control group, and eye dropping of TTR could reverse this trend, wherein the dosage of 10 µmol/L has the best effect (Table 18).

**Table 18 AMD pathological condition of C57BL/6 mice induced by laser retinal photocoagulation treated with human TTR**

| The AMD pathological condition of C57BL/6 mice induced by laser retinal photocoagulation after 2 weeks of eyedropping treatment using human recombinant TTR (saline solution+2% hyaluronic acid). | | | | | |
|---|---|---|---|---|---|
| Concentration of TTR for eyedropping (µmol/L) | 0 | 5 | 10 | 15 | 20 |
| Retinal leakage area (%) | 24.7±2.2 | 7.2±0.5 | 4.5±0.3 | 4.5±0.2 | 4.3±0.2 |
| No. of retinal neovascularization (10 visual fields) | 80±7 | 18±2 | 12±2 | 13±1 | 14±3 |

It can be known from the examples described above that the pathological conditions of DR, AMD and ROP in respective animal models can be effectively treated by TTR in the manner of eyedropping.

### Example 15: treating DR (diabetic retinopathy) SD rats with human TTR /rat TTR in the manner of eye dropping

8-week-old SD rats weighing 200-250 g was fasted for 12-18 h, and treated with intraperitoneally injection of 2% STZ (60 mg/kg), followed by cutting the tail and collecting blood thereof after 48 h and 72 h. The blood glucose was detected higher than 16.7 mM by test paper, indicating that the model was successfully established, and DR SD rats were obtained. DR SD rats were divided into 5 large groups, one group is DR SD rats without any treatment (5 rats), one group is the human TTR eye dropping group (5 rats) dropped with the human TTR prepared in Example 5, each left eye dropped with human TTR (10 µmol/L) (saline+2% hyaluronic acid) twice per day, and each right eye dropped with saline+2% hyaluronic acid; one group is the rat TTR eye dropping group (5 rats) dropped with the rat TTR prepared in Example 5, each left eye dropped with rat TTR (10 µmol/L) (saline+2% hyaluronic acid) twice per day, and each right eye dropped with saline+2% hyaluronic acid; one group is the human TTR-CL eye dropping group (5 rats) dropped with the human TTR-CL prepared in Example 5, each left eye dropped with human TTR-CL (10 µmol/L) (saline+2% hyaluronic acid) twice per day, and each right eye dropped with saline+2% hyaluronic acid; the last one is the human TTR-Modified eye dropping group (5 rats) dropped with the human TTR-Modified prepared in Example 5, each left eye dropped with human TTR-Modified (10 µmol/L) (saline+2% hyaluronic acid) twice per day, and each right eye dropped with saline+2% hyaluronic acid; in addition, another group of normal SD rats (No eye dropping) served as a normal control group (5 rats). All SD rats were fed for 3 months, followed by stripping the retina for staining with Evans Blue (EB) to observe the retinal vascular leakage, for Trypsin enzymatic hydrolysis to observe the density of neovascularization; the results show that compared with the normal control, retinal vascular leakage and the number of neovascularization was significantly increased after feeding STZ induced SD rats for 3 months, while the eyeball retinal leakage conditions of SD rats treated with human TTR, human TTR-CL, human TTR-Modified and rat TTR were significantly inhibited, and the number of retinal neovascularization was reduced significantly, indicating the clinical pathological condition of DR had been alleviated.

**Table 19 DR pathological condition of SD rats induced by STZ treated with human TTR/rat TTR**

| The DR pathological condition of SD rats induced by STZ after 3 months of eyedropping treatment using human recombinant TTR/rat recombinant TTR (saline solution+2% hyaluronic acid). | | | | | | |
|---|---|---|---|---|---|---|
| | normal eye control | no TTR dropping control | human TTR | human TTR-CL | human TTR-Mod ified | rat TTR |
| Concentration of TTR for eyedropping (µmol/L) | - | 0 | 10 | 10 | 10 | 10 |
| Retinal leakage area (%) | 4.9±0.5 | 29.4±3.1 | 5.5±0.4 | 5.7±0.9 | 10.2±1.3 | 6.2±0.7 |
| No. of retinal neovascularization (10 visual fields) | 11±2 | 77±6 | 11±1 | 10±1 | 19±4 | 13±2 |

### Example 16: treating ROP (retinopathy of prematurity) SD rats with human TTR/rat TTR in the manner of eye dropping

One-week-old SD suckling rats were fed in hyperbaric oxygen chamber, and after 5 days in hyperbaric oxygen chamber, the modeling was successfully completed, then transferred all suckling rats from the hyperbaric oxygen chamber to normal environment for feeding, and some model suckling rats were treated with eye dropping of 10 µmol/L TTR, once per day, 5 days. Compare ROP non-eye dropping group (5 rats), ROP+human TTR eye dropping group (5 rats), ROP+human TTR-CL eye dropping group (5 rats), ROP+human TTR-Modified eye dropping group (5 rats) and ROP+rat TTR eye dropping group (5 rats), and there was another non-ROP model rats (non-eye dropping) as normal control group (5 rats). A large number of leakage areas appeared when abnormal neovascularization covered the retina in ROP group, and eye dropping of TTR could reverse this trend.

**Table 20 The ROP pathological condition of SD suckling rats induced by hyperbaric oxygen chamber treated with human TTR/rat TTR**

| The pathological condition of SD suckling rats induced by hyperbaric oxygen chamber after 5 days of eyedropping treatment using human recombinant TTR/rat recombinant TTR (saline solution+2% hyaluronic acid). | | | | | | |
|---|---|---|---|---|---|---|
| | normal eye control | no TTR dropping control | human TTR | human TTR-CL | human TTR-Mod ified | rat TTR |
| Concentration of TTR for eyedropping (µmol/L) | - | 0 | 10 | 10 | 10 | 10 |
| Retinal leakage area (%) | 3.3±0.3 | 44.7±5.1 | 3.4±0.2 | 3.0±0.1 | 7.8±1.0 | 3.6±0.4 |

### Example 17: treating AMD (age-related macular degeneration) C57BL/6 mice with human TTR/mouse TTR in the manner of eye dropping

9-week-old C57BL/6 mice were subject to retinal photocoagulation by krypton laser (647 nm), with a power of 360 mW, a diameter of 50 µm, a time of 0.05 s, 8 photocoagulation site for each eye, for inducing the neovascularization in choroid, and gradually proceeding to retinal hyperplasia to obtain AMD C57BL/6 mice. AMD C57BL/6 mice were divided into a dropping group and a non-dropping group. The dropping group was treated with eye dropping of TTR, with the right eye of 10 µmol/L human TTR or mouse TTR (saline+2% hyaluronic acid) twice per day, 30 µL each time, the left eye of saline +2% hyaluronic acid as control; the dropping groups treated with human TTR, human TTR-CL, human TTR-Modified, or mouse TTR has 5 mice in each group; the non-dropping group has 5 mice without any treatment; another group (5 mice) without laser photocoagulation was served as normal control group (non-dropping). The animals were sacrificed after eye dropping for 2 weeks, and the retina was stripped for EB staining to observe the retinal leakage conditions, and for Trypsin enzymatic hydrolysis to observe the density of neovascularization. It can be known in Table 21 that the eyeball retinal leakage conditions of groups treated with human TTR, human TTR-CL, human TTR-Modified and mouse TTR were inhibited significantly, and the number of retinal neovascularization was reduced significantly, indicating that the clinical pathological conditions of AMD were effectively alleviated.

**Table 21 AMD pathological condition of C57BL/6 mice induced by laser retinal photocoagulation treated with human TTR/mouse TTR**

| The AMD pathological condition of C57BL/6 mice induced by laser retinal photocoagulation after 2 weeks of eyedropping treatment using human recombinant TTR/mouse recombinant TTR (saline solution+2% hyaluronic acid). | | | | | | |
|---|---|---|---|---|---|---|
| | normal eye control | no TTR dropping control | human TTR | human TTR-CL | human TTR-Mod ified | mouse TTR |
| Concentration of TTR for eyedropping (µmol/L) | - | 0 | 10 | 10 | 10 | 10 |
| Retinal leakage area (%) | 3.9±0.4 | 24.7±2.2 | 4.5±0.3 | 4.8±0.6 | 9.4±1.1 | 4.2±0.5 |
| No. of retinal neovascularization (10 visual fields) | 11±3 | 80±7 | 12±2 | 11±2 | 20±3 | 10±3 |

### Part IV: Improvement of the excipient

### Example 18: the use of sodium carboxymethyl cellulose as an excipient can promote TTR to cross the corneal barrier

(1) The human TTR prepared in Example 5 was prepared as 5-30 µmol/L (containing saline), and was administered by eyedropping to C57BL/6 mice (8 weeks old) and SD rats (8 weeks old) respectively. The animals were sacrificed after treatment for 3 h, and the proteins were extracted from the vitreous body and fundus oculi samples. The rabbit anti-His-tag antibody was used as the primary antibody and donkey anti-rabbit antibody was used as the secondary antibody. The content of human TTR in the vitreous body and fundus oculi samples from C57BL/6 mice and SD rats was determined by ELISA.
(2) The human TTR prepared in Example 5 was prepared as 10 µmol/L (containing saline and 0-8 mg/mL sodium carboxymethyl cellulose (purchased from SinoPharm with a viscosity of 800-1200 CP)), and was administered by eyedropping to C57BL/6 mice (8 weeks old) and SD rats (8 weeks old) respectively. The animals were sacrificed after treatment for 3-72 h, and the proteins were extracted from the vitreous body and fundus oculi samples. The rabbit anti-His-tag antibody was used as the primary antibody and donkey anti-rabbit antibody was used as the secondary antibody. The content of human TTR in the vitreous body and fundus oculi samples from C57BL/6 mice and SD rats was determined by ELISA.

The results show that the content of human TTR in the vitreous body and fundus oculi samples reached the peak 3 h after eye dropping; when sodium carboxymethyl cellulose was added to the eye drops, the content of human TTR in the vitreous body and fundus oculi samples were both increased significantly (increased more than 10%), and when the content of sodium carboxymethyl cellulose was 6 mg/mL, the content of human TTR in the vitreous body and fundus oculi samples reached the peak 3 h after eye dropping; after eye dropping, the content half-life of human TTR in the vitreous body and fundus oculi samples of C57BL/6 mice and SD rats was close to 72 h, indicating that it can effectively exist in the vitreous body and fundus oculi samples for 72 h, with a sufficient therapeutic concentration and therapeutic duration (Table 22).

**Table 22 Human TTR entering the vitreous body and fundus oculi through the corneal barrier of C57BL/6 mice and SD rats**

| (1) The human TTR content in vitreous body and fundus oculi 3 h after eye dropping using human recombinant TTR (in saline solution). | | | | | | |
|---|---|---|---|---|---|---|
| Concentration of TTR drops (µmol/L) | 5 | 10 | 15 | 20 | 25 | 30 |
| Concentration of human TTR in vitreous body and fundus oculi samples of C57BL/6 mice (µmol/L) | 0.84± 0.07 | 1.81± 0.19 | 1.75± 0.20 | 1.83± 0.21 | 1.88± 0.17 | 1.87± 0.20 |
| Concentration of human TTR in vitreous body and fundus oculi samples of SD rats (µmol/L) | 1.14± 0.11 | 2.43± 0.21 | 2.37± 0.30 | 2.42± 0.36 | 2.45± 0.42 | 2.44± 0.36 |

| (2) The human TTR content in vitreous body and fundus oculi 3 h after eye dropping using human recombinant TTR (10 (µmol/L) (in saline solution added with sodium carboxymethyl cellulose of different concentration). | | | | | | |
|---|---|---|---|---|---|---|
| Concentration of sodium carboxymethyl cellulose (mg/mL) | 0 | 2 | 4 | 6 | 8 | |
| Concentration of human TTR in vitreous body and fundus oculi samples of C57BL/6 mice (µmol/L) | 1.81± 0.19 | 2.01± 0.17 | 2.44± 0.18 | 2.85± 0.25 | 2.80±0.17 | |
| Concentration of human TTR in vitreous body and fundus oculi samples of SD rats (µmol/L) | 2.43± 0.21 | 2.88± 0.19 | 3.05± 0.26 | 3.24± 0.29 | 3.20±0.30 | |

| (3) The peak of human TTR content in vitreous body and fundus oculi was reached 3 h after eyedropping using human recombinant TTR (10 (µmol/L) (in saline solution added with 6 mg/mL sodium carboxymethyl cellulose), and the human TTR content thereof at different times thereafter are shown as below | | | | | | |
|---|---|---|---|---|---|---|
| Sampling time after eyedropping (h) | 3 | 18 | 36 | 60 | 72 | |
| Concentration of human TTR in vitreous body and fundus oculi samples of C57BL/6 mice (µmol/L) | 2.85± 0.25 | 2.54± 0.22 | 2.14± 0.10 | 1.66± 0.10 | 1.44±0.10 | |
| Concentration of human TTR in vitreous body and fundus oculi samples of SD rats (µmol/L) | 3.24± 0.29 | 2.96± 0.27 | 2.32± 0.19 | 1.83± 0.13 | 1.63±0.15 | |

### Example 19: the use of dextran 70 as an excipient can promote TTR to cross the corneal barrier

(1) The human TTR prepared in Example 5 was prepared as 5-30 µmol/L (containing saline), and was administered by eyedropping to C57BL/6 mice (8 weeks old) and SD rats (8 weeks old) respectively. The animals were sacrificed after treatment for 3 h, and the proteins were extracted from the vitreous body and fundus oculi samples. The rabbit anti-His-tag antibody was used as the primary antibody and donkey anti-rabbit antibody was used as the secondary antibody. The content of human TTR in the vitreous body and fundus oculi samples from C57BL/6 mice and SD rats was determined by ELISA.
(2) The human TTR prepared in Example 5 was prepared as 10 µmol/L (containing saline and 0-0.8 mg/mL dextran 70 (purchased from SinoPharm with a molecular weight of 64000-76000)), and was administered by eyedropping to C57BL/6 mice (8 weeks old) and SD rats (8 weeks old) respectively. The animals were sacrificed after treatment for 3-72 h, and the proteins were extracted from the vitreous body and fundus oculi samples. The rabbit anti-His-tag antibody was used as the primary antibody and donkey anti-rabbit antibody was used as the secondary antibody. The content of human TTR in the vitreous body and fundus oculi samples from C57BL/6 mice and SD rats was determined by ELISA.

The results show that the content of human TTR in the vitreous body and fundus oculi samples reached the peak 3 h after eye dropping; when dextran 70 was added to the eye drops, the content of human TTR in the vitreous body and fundus oculi samples were both increased significantly (increased more than 15%), and when the content of dextran 70 was 0.4 mg/mL, the content of human TTR in the vitreous body and fundus oculi samples reached the peak 3 h after eye dropping; after eye dropping, the content half-life of human TTR in the vitreous body and fundus oculi samples of C57BL/6 mice and SD rats was close to 60 h, indicating that it can effectively exist in the vitreous body and fundus oculi samples for 60 h, with a sufficient therapeutic concentration and therapeutic duration (Table 23).

**Table 23 Human TTR entering the vitreous body and fundus oculi through the corneal barrier of C57BL/6 mice and SD rats**

| (1) The human TTR content in vitreous body and fundus oculi 3 h after eye dropping using human recombinant TTR (in saline solution). | | | | | | |
|---|---|---|---|---|---|---|
| Concentration of TTR drops (µmol/L) | 5 | 10 | 15 | 20 | 25 | 30 |
| Concentration of human TTR in vitreous body and fundus oculi samples of C57BL/6 mice (µmol/L) | 0.84± 0.07 | 1.81± 0.19 | 1.75± 0.20 | 1.83± 0.21 | 1.88± 0.17 | 1.87± 0.20 |
| Concentration of human TTR in vitreous body and fundus oculi samples of SD rats (µmol/L) | 1.14± 0.11 | 2.43± 0.21 | 2.37± 0.30 | 2.42± 0.36 | 2.45± 0.42 | 2.44± 0.36 |

| (2) The human TTR content in vitreous body and fundus oculi 3 h after eye dropping using human recombinant TTR (10 (µmol/L) (in saline solution added with dextran 70 of different concentration). | | | | | | |
|---|---|---|---|---|---|---|
| Concentration of dextran 70 (mg/mL) | 0 | 0.2 | 0.4 | 0.6 | 0.8 | |
| Concentration of human TTR in vitreous body and fundus oculi samples of C57BL/6 mice (µmol/L) | 1.81± 0.19 | 2.47± 0.20 | 2.91± 0.23 | 2.90± 0.24 | 2.87±0.21 | |
| Concentration of human TTR in vitreous body and fundus oculi samples of SD rats (µmol/L) | 2.43± 0.21 | 2.81± 0.22 | 3.22± 0.27 | 3.18± 0.30 | 3.12±0.28 | |

| (3) The peak of human TTR content in vitreous body and fundus oculi was reached 3 h after eyedropping using human recombinant TTR (10 (µmol/L) (in saline solution added with 0.4 mg/mL dextran 70), and the human TTR content thereof at different times thereafter are shown as below | | | | | | |
|---|---|---|---|---|---|---|
| Sampling time after eyedropping (h) | 3 | 18 | 36 | 60 | 72 | |
| Concentration of human TTR in vitreous body and fundus oculi samples of C57BL/6 mice (µmol/L) | 2.91± 0.23 | 2.21± 0.16 | 1.73± 0.14 | 1.48± 0.13 | 0.87±0.06 | |
| Concentration of human TTR in vitreous body and fundus oculi samples of SD rats (µmol/L) | 3.22± 0.27 | 2.53± 0.20 | 1.86± 0.11 | 1.59± 0.14 | 0.93±0.10 | |

### Example 20: the use of chondroitin sulfate A sodium salt as an excipient can promote TTR to cross the corneal barrier

(1) The human TTR prepared in Example 5 was prepared as 5-30 µmol/L (containing saline), and was administered by eyedropping to C57BL/6 mice (8 weeks old) and SD rats (8 weeks old) respectively. The animals were sacrificed after treatment for 3 h, and the proteins were extracted from the vitreous body and fundus oculi samples. The rabbit anti-His-tag antibody was used as the primary antibody and donkey anti-rabbit antibody was used as the secondary antibody. The content of human TTR in the vitreous body and fundus oculi samples from C57BL/6 mice and SD rats was determined by ELISA.
(2) The human TTR prepared in Example 5 was prepared as 10 µmol/L (containing saline and 0-40 mg/mL chondroitin sulfate A sodium salt (purchased from SinoPharm)), and was administered by eyedropping to C57BL/6 mice (8 weeks old) and SD rats (8 weeks old) respectively. The animals were sacrificed after treatment for 3-72 h, and the proteins were extracted from the vitreous body and fundus oculi samples. The rabbit anti-His-tag antibody was used as the primary antibody and donkey anti-rabbit antibody was used as the secondary antibody. The content of human TTR in the vitreous body and fundus oculi samples from C57BL/6 mice and SD rats was determined by ELISA.

The results show that the content of human TTR in the vitreous body and fundus oculi samples reached the peak 3 h after eye dropping; when chondroitin sulfate A sodium salt was added to the eye drops, the content of human TTR in the vitreous body and fundus oculi samples were both increased significantly (increased more than 17%), and when the content of chondroitin sulfate A sodium salt was 20 mg/mL, the content of human TTR in the vitreous body and fundus oculi samples reached the peak 3 h after eye dropping; after eye dropping, the content half-life of human TTR in the vitreous body and fundus oculi samples of C57BL/6 mice and SD rats was close to 72 h, indicating that it can effectively exist in the vitreous body and fundus oculi samples for 72 h, with a sufficient therapeutic concentration and therapeutic duration (Table 24).

**Table 24 Human TTR entering the vitreous body and fundus oculi through the corneal barrier of C57BL/6 mice and SD rats**

| (1) The human TTR content in vitreous body and fundus oculi 3 h after eye dropping using human recombinant TTR (in saline solution). | | | | | | |
|---|---|---|---|---|---|---|
| Concentration of TTR drops (µmol/L) | 5 | 10 | 15 | 20 | 25 | 30 |
| Concentration of human TTR in vitreous body and fundus oculi samples of C57BL/6 mice (µmol/L) | 0.84± 0.07 | 1.81± 0.19 | 1.75± 0.20 | 1.83± 0.21 | 1.88± 0.17 | 1.87± 0.20 |
| Concentration of human TTR in vitreous body and fundus oculi samples of SD rats (µmol/L) | 1.14± 0.11 | 2.43± 0.21 | 2.37± 0.30 | 2.42± 0.36 | 2.45± 0.42 | 2.44± 0.36 |

| (2) The human TTR content in vitreous body and fundus oculi 3 h after eye dropping using human recombinant TTR (10 (µmol/L) (in saline solution added with chondroitin sulfate A sodium salt of different concentration). | | | | | | |
|---|---|---|---|---|---|---|
| Concentration of chondroitin sulfate A sodium salt (mg/mL) | 0 | 10 | 20 | 30 | 40 | |
| Concentration of human TTR in vitreous body and fundus oculi samples of C57BL/6 mice (µmol/L) | 1.81± 0.19 | 2.57± 0.22 | 3.01± 0.27 | 2.97± 0.26 | 2.84±0.26 | |
| Concentration of human TTR in vitreous | 2.43± | 2.85± | 3.25± | 3.20± | 3.10±0.30 | |
| body and fundus oculi samples of SD rats (µmol/L) | 0.21 | 0.27 | 0.31 | 0.30 | | |

| (3) The peak of human TTR content in vitreous body and fundus oculi was reached 3 h after eyedropping using human recombinant TTR (10 (µmol/L) (in saline solution added with 20 mg/mL chondroitin sulfate A sodium salt), and the human TTR content thereof at different times thereafter are shown as below | | | | | | |
|---|---|---|---|---|---|---|
| Sampling time after eyedropping (h) | 3 | 18 | 36 | 60 | 72 | |
| Concentration of human TTR in vitreous body and fundus oculi samples of C57BL/6 mice (µmol/L) | 3.01± 0.27 | 2.66± 0.27 | 1.98± 0.20 | 1.67± 0.14 | 1.48±0.11 | |
| Concentration of human TTR in vitreous body and fundus oculi samples of SD rats (µmol/L) | 3.25± 0.31 | 2.78± 0.25 | 2.03± 0.19 | 1.83± 0.17 | 1.58±0.17 | |

### Example 21: treating DR (diabetic retinopathy) SD rats with human TTR-excipient in the manner of eye dropping

8-week-old SD rats weighing 200-250 g, was fasted for 12-18 h, and treated with intraperitoneally injection of 2% STZ (60 mg/kg), followed by cutting the tail and collecting blood thereof after 48 h and 72 h. The blood glucose was detected higher than 16.7 mM by test paper, indicating that the model was successfully established, and DR SD rats were obtained. DR SD rats were divided into 6 groups, one is DR SD rats without any treatment (5 rats), and the other 5 groups each has 5 rats, and the rats were treated with eye dropping twice per day for the left eye and the right eye, 30 µL each time. Wherein, the left eye was dropped with human TTR prepared in Example 5, or human TTR prepared in Example 5 added with the excipient, specifically with 10 µmol/L human TTR (saline solution), 10 µmol/L human TTR (saline solution+6 mg/mL sodium carboxymethyl cellulose), 10 µmol/L human TTR (saline solution+6 mg/mL PEG400), 10 µmol/L human TTR (saline solution+0.4 mg/mL dextran 70) and 10 µmol/L human TTR (saline solution+20 mg/mL chondroitin sulfate A sodium salt); the right eye was dropped with saline solution, or saline solution added with the excipient as control, specifically with saline solution, saline solution+6 mg/mL sodium carboxymethyl cellulose, saline solution+6 mg/mL PEG400, saline solution+0.4 mg/mL dextran 70 and saline solution+20 mg/mL chondroitin sulfate A sodium salt. In addition, there was another group of normal SD rats as a normal control group (5 rats). All SD rats were fed for 3 months, followed by stripping the retina for staining with Evans Blue (EB) to observe the retinal vascular leakage, for Trypsin enzymatic hydrolysis to observe the density of neovascularization. The results show that compared with the normal control, retinal vascular leakage and the number of neovascularization was significantly increased after feeding STZ induced SD rats for 3 months, while the eyeball retinal leakage condition of SD rats treated with human TTR was significantly inhibited, and the number of retinal neovascularization was reduced significantly, indicating the clinical pathological condition of DR had been alleviated. Wherein, the treatment of administering 10 µmol/L human TTR (saline solution+20 mg/mL chondroitin sulfate A sodium salt) per day has best therapeutic effect (Table 25), while the treatment of administering 10 µmol/L human TTR (saline solution+6 mg/mL PEG400) per day has comparatively poor therapeutic effect.

**Table 25 DR pathological condition of SD rats induced by STZ treated with human TTR/human TTR-excipient**

| The DR pathological condition of SD rats induced by STZ after 3 months of eyedropping treatment using human recombinant TTR (saline solution+no excipient/6 mg/mL sodium carboxymethyl cellulose/6 mg/mL PEG400/0.4 mg/mL dextran 70/20 mg/mL chondroitin sulfate A sodium salt). | | | | | | | | | | | | |
|---|---|---|---|---|---|---|---|---|---|---|---|---|
| | no r m al co nt ro l eye | no T T R dr op pi ng cont ro l | hu m an T T R (s ali neso lu ti on ) | sa li ne so lu ti on | huma n TTR (salin e soluti on+6 mg/m Lsodiu m carbo xyme thyl cellul ose) | sali ne solu tion +6 mg/ mL sodi um carb oxy met hyl cell ulos e | hu man TT R (sali ne solu tion +6 mg/ mL PE G40 0) | sali ne solu tion +6 mg/ mL PE G400 | hu man TT R (sali ne solu tion +0.4 mg/ mL dext ran 70) | sali ne solu tion +0. 4 mg/ mL dextran 70 | huma n TTR (salin e soluti on+2 0 mg/m L chon droiti n sulfat e A sodiu m salt) | salin e solut ion+ 20 mg/ mL chon droit in sulfa te A sodi um salt |
| Concentration of TTR for eyedropping (µmol/L) | - | 0 | 10 | 0 | 10 | 0 | 10 | 0 | 10 | 0 | 10 | 0 |
| Retinal leakage area (%) | 4. 9± 0. 5 | 29 .4 ± 3. 1 | 6. 0 ± 0. 5 | 30 .8 ± 2. 5 | 5.7±0 .9 | 30. 4±3 .5 | 6.7 ±0. 5 | 34. 4±3 .5 | 5.0 ±0. 3 | 32. 8±2 .9 | 4.8±0 .4 | 28.6 ±3.1 |
| No. of retinal neovascularizatio n (10 visual fields) | 11 ±2 | 77 ± 6 | 13 ± 2 | 80 ± 9 | 10±1 | 80± 7 | 14± 3 | 79± 7 | 10± 2 | 75± 9 | 9±1 | 82±5 |

### Example 22: treating AMD (age-related macular degeneration) C57BL/6 mice with human TTR-excipient in the manner of eye dropping

9-week-old C57BL/6 mice were subject to retinal photocoagulation by krypton laser (647 nm), with a power of 360 mW, a diameter of 50 µm, a time of 0.05 s, 8 photocoagulation site for each eye, for inducing the neovascularization in choroid, and gradually proceeding to retinal hyperplasia to obtain AMD C57BL/6 mice. AMD C57BL/6 mice were divided into 6 groups, one is AMD C57BL/6 mice without any treatment (5 mice), and the other 5 groups each has 5 mice, and the mice were treated with eye dropping twice per day for the left eye and the right eye, 30 µL each time. Wherein, the left eye was dropped with human TTR prepared in Example 5, or human TTR prepared in Example 5 added with the excipient, specifically with 10 µmol/L human TTR (saline solution), 10 µmol/L human TTR (saline solution+6 mg/mL sodium carboxymethyl cellulose), 10 µmol/L human TTR (saline solution+6 mg/mL PEG400), 10 µmol/L human TTR (saline solution+0.4 mg/mL dextran 70) and 10 µmol/L human TTR (saline solution+20 mg/mL chondroitin sulfate A sodium salt); the right eye was dropped with saline solution, or saline solution added with the excipient as control, specifically with saline solution, saline solution+6 mg/mL sodium carboxymethyl cellulose, saline solution+6 mg/mL PEG400, saline solution+0.4 mg/mL dextran 70 and saline solution+20 mg/mL chondroitin sulfate A sodium salt). In addition, there was another group of normal AMD C57BL/6 mice as a normal control group (5 mice). The animals were sacrificed after eye dropping for 2 weeks, and the retina was stripped for EB staining to observe the retinal leakage conditions, and for Trypsin enzymatic hydrolysis to observe the density of neovascularization.

The results show that more leakage areas appeared in AMD C57BL/6 mouse control group, and the number of neovascularization increased significantly, while eye dropping of TTR could reverse this trend (Table 26), and TTR represented better therapeutic effect in the groups added with sodium carboxymethyl cellulose, dextran 70 and chondroitin sulfate A sodium salt.

**Table 26 AMD pathological condition of C57BL/6 mice induced by laser retinal photocoagulation treated with human TTR/human TTR-excipient**

| The AMD pathological condition of C57BL/6 mice induced by laser retinal photocoagulation after 2 weeks of eyedropping treatment using human recombinant TTR (saline solution+no excipient/6 mg/mL sodium carboxymethyl cellulose/6 mg/mL PEG400/0.4 mg/mL dextran 70/20 mg/mL chondroitin sulfate A sodium salt). | | | | | | | | | | | | |
|---|---|---|---|---|---|---|---|---|---|---|---|---|
| | no r m al co nt ro l ey e | no T T R dr op pi ng co nt ro l | hu m an T T R (s ali ne so lut io n) | sa lin e so lut io n | huma n TTR (salin e soluti on+6 mg/m L sodiu m carbo xyme thyl cellul ose) | saline soluti on+6 mg/m L sodiu m carbo xyme thyl cellul ose | hu man TT R (sali ne solu tion +6 mg/ mL PE G40 0) | sali ne solu tion +6 mg/ mL PE G40 0 | hu man TT R (sali ne solu tion +0. 4 mg/ mL dext ran 70) | sali ne solu tion +0. 4 mg/ mL dext ran 70 | human TTR (saline solutio n+20 mg/mL chondr oitin sulfate A sodium salt) | salin e solut ion+ 20 mg/ mL chon droit in sulfa te A sodi um salt |
| Concentration of TTR for eyedropping (µmol/L) | - | 0 | 10 | 0 | 10 | 0 | 10 | 0 | 10 | 0 | 10 | 0 |
| Retinal leakage area (%) | 3. 9± 0. 4 | 24 .7 ±2 .2 | 5. 5± 0. 4 | 26 .3 ±3 .0 | 4.0±0 .2 | 25.8± 3.2 | 4.7 ±0. 5 | 23. 9±3 .0 | 4.0 ±0. 4 | 29. 9±2 .9 | 3.7±0.3 | 30.2 ±3.1 |
| No. of retinal neovasculariz ation (10 visual fields) | 11 ±3 | 80 ±7 | 13 ±2 | 82 ±9 | 11±1 | 79±8 | 12± 3 | 83± 9 | 10± 1 | 84± 11 | 10±1 | 83±8 |

### Example 23: treating ROP (retinopathy of prematurity) SD rats with human TTR-excipient in the manner of eye dropping

One-week-old SD suckling rats were fed in hyperbaric oxygen chamber, and the normal control group was fed in normal environment (normal control group, 5 rats). The rats were taken out after feeding for 5 days in hyperbaric oxygen chamber to obtain ROP SD rats. All ROP SD rats and rats of normal control group were fed in normal environment for succeeding 5 days. In the 5 days, ROP SD rats were divided into 6 groups, one is ROP SD rats without any treatment (5 rats), and the other 5 groups each has 5 rats, and the rats were treated with eye dropping once per day for the left eye and the right eye, 30 µL each time. Wherein, the left eye was dropped with human TTR prepared in Example 5, or human TTR prepared in Example 5 added with the excipient, specifically with 10 µmol/L human TTR (saline solution), 10 µmol/L human TTR (saline solution+6 mg/mL sodium carboxymethyl cellulose), 10 µmol/L human TTR (saline solution+6 mg/mL PEG400), 10 µmol/L human TTR (saline solution+0.4 mg/mL dextran 70) and 10 µmol/L human TTR (saline solution+20 mg/mL chondroitin sulfate A sodium salt); the right eye was dropped with saline solution, or saline solution added with the excipient as control, specifically with saline solution, saline solution+6 mg/mL sodium carboxymethyl cellulose, saline solution+6 mg/mL PEG400, saline solution+0.4 mg/mL dextran 70 and saline solution+20 mg/mL chondroitin sulfate A sodium salt. The animals were sacrificed after eye dropping for 5 days, and the retina was stripped for EB staining to observe the retinal leakage conditions. A large number of leakage areas appeared when abnormal neovasculars covered the retina in ROP SD rats control group, and eye dropping of TTR could reverse this trend (Table 27), and TTR represented better therapeutic effect in the groups added with sodium carboxymethyl cellulose, dextran 70 and chondroitin sulfate A sodium salt.

**Table 27 ROP pathological condition of SD suckling rats induced by hyperbaric oxygen chamber treated with human TTR/human TTR-excipient**

| The ROP pathological condition of SD suckling rats induced by hyperbaric oxygen chamber after 5 days of eyedropping treatment using human recombinant TTR (saline solution+no excipient/6 mg/mL sodium carboxymethyl cellulose/6 mg/mL PEG400/0.4 mg/mL dextran 70/20 mg/mL chondroitin sulfate A sodium salt). | | | | | | | | | | | | |
|---|---|---|---|---|---|---|---|---|---|---|---|---|
| | no r m al co nt ro l ey e | no T T R dr op pi ng co nt rol | hu man TT R (sali ne solu tion ) | sa li ne so lu ti on | human TTR (saline solutio n+6 mg/mL sodium carbox ymethyl cellulos e) | saline soluti on+6 mg/m L sodiu m carbo xyme thyl cellul ose | huma n TTR (salin e soluti on+6 mg/m L PEG4 00) | sali ne solu tion +6 mg/ mL PE G40 0 | huma n TTR (salin e soluti on+0. 4 mg/m L dextr an 70) | sali ne solu tion +0. 4 mg/ mL dext ran 70 | human TTR (saline solutio n+20 mg/mL chondr oitin sulfate A sodium salt) | saline solutio n+20 mg/m L chondr oitin sulfate A sodiu m salt |
| Conc entrat ion of TTR for eyedr oppin g (µmo l/L) | - | 0 | 10 | 0 | 10 | 0 | 10 | 0 | 10 | 0 | 10 | 0 |
| Retin al leaka ge area (%) | 3. 3 ± 0. 5 | 44 .7 ±5 .1 | 5.2 ±0. 4 | 46 .3 ± 6. 1 | 3.2±0.2 | 45.8± 4.7 | 4.0±0 .3 | 39. 7±4 .0 | 4.1±0 .4 | 48. 6±5 .2 | 3.0±0.2 | 42.6±4 .7 |

### Part V: Improvement of ligands

### Example 24: In silicon simulation of the binding forms of TTR and each ligand molecule

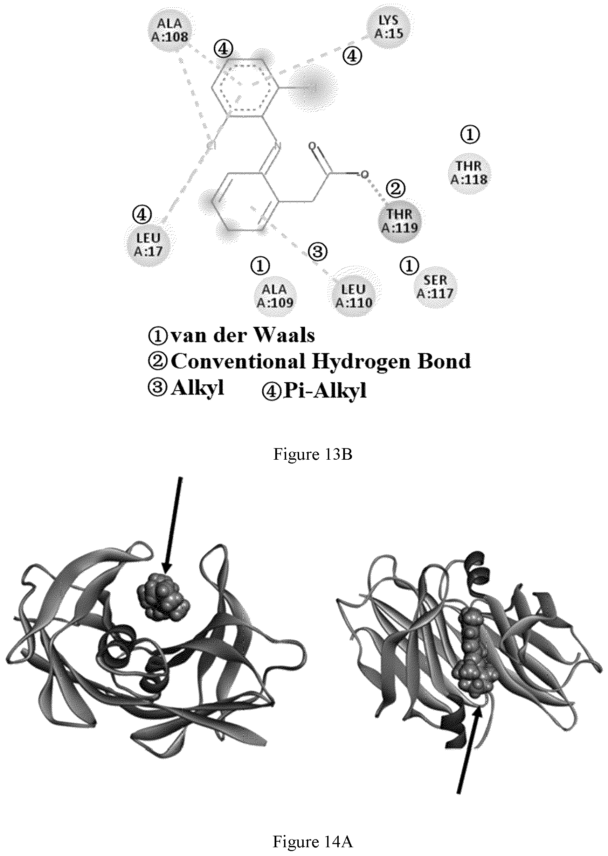

The static co-crystallization model in the PDB database (PDF: 3CFQ) of TTR dimer and diclofenac is shown in Figure 13A. In Figure 13A, the protein structure is TTR dimer, the diclofenac ligand molecule is represented by arrows, and one molecule of TTR dimer can bind to two molecules of diclofenac. Figure 13B shows the interaction between diclofenac and amino acid residues of TTR.

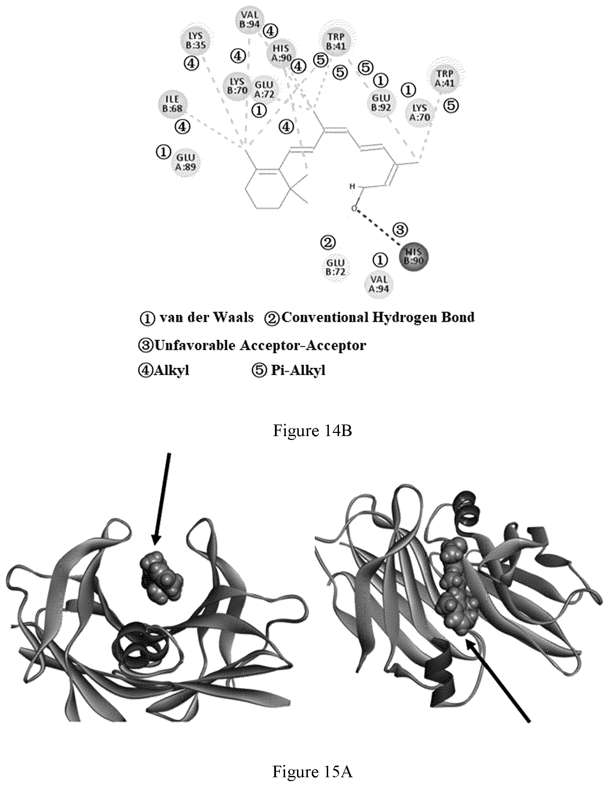

Through molecular simulation with Discovery studio software, it is found that vitamin A1 (retinol) can bind stably to the hydrophobic passage of a TTR polymer. In Figure 14A, the protein structure is TTR dimer, vitamin A1 ligand molecule is represented by arrows, and one molecule of TTR dimer can bind to one molecule of vitamin A1. Figure 14B shows the interaction between vitamin A1 and amino acid residues of TTR.

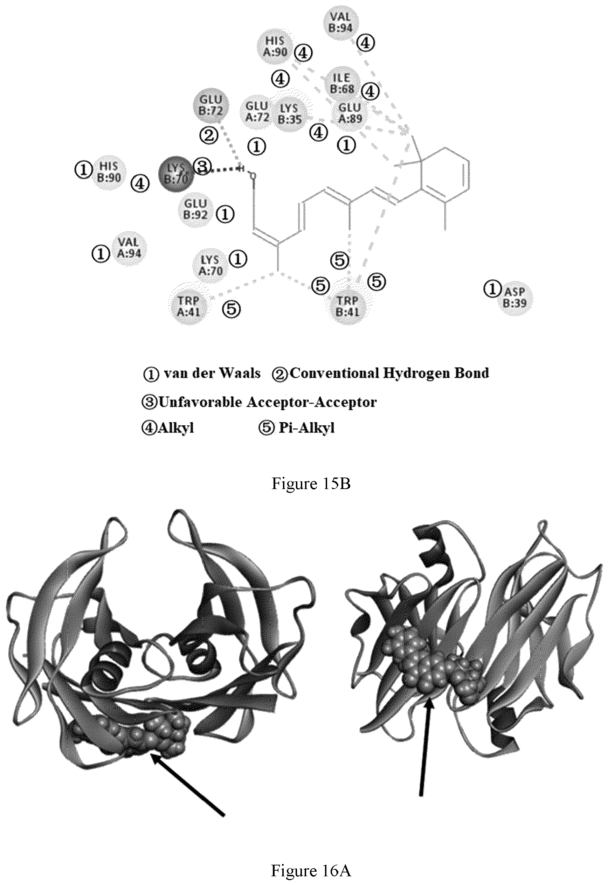

Through molecular simulation with Discovery studio software, it is found that vitamin A2 (3-dehydroretinol) can bind stably to the hydrophobic passage of a TTR polymer. In Figure 15A the protein structure is TTR dimer, vitamin A2 ligand molecule is represented by arrows, and one molecule of TTR dimer can bind to one molecule of vitamin A2. Figure 15B shows the interaction between vitamin A2 and amino acid residues of TTR.

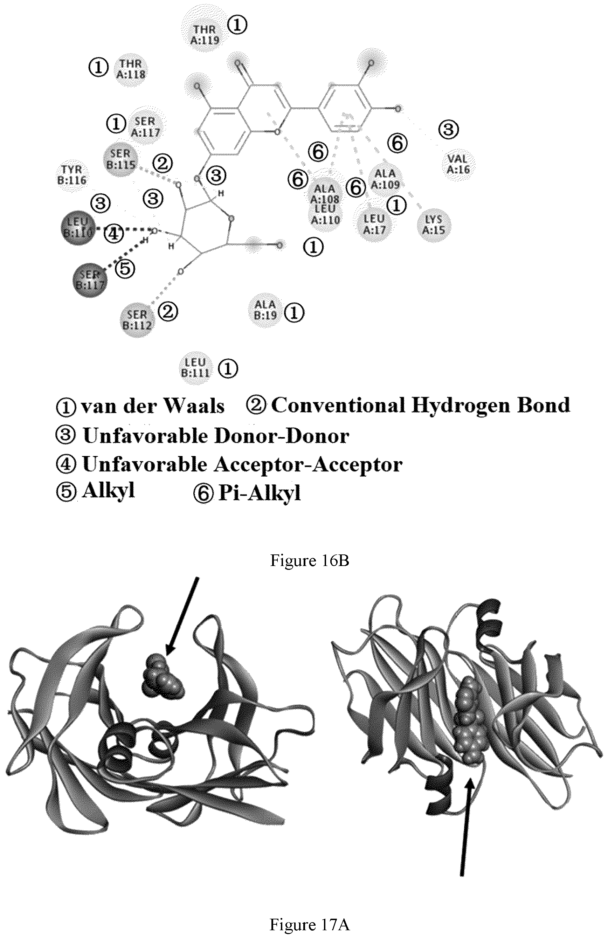

Through molecular simulation with Discovery studio software, it is found that luteoloside can bind stably to the hydrophobic passage of a TTR polymer. In Figure 16A, the protein structure is TTR dimer, luteoloside ligand molecule is represented by arrows, and one molecule of TTR dimer can bind to one molecule of luteoloside. Figure 16B shows the interaction between luteoloside and amino acid residues of TTR.

It can be seen from the results described above, the forces between TTR and these ligand molecules are mainly non-covalent forces such as van der Waals forces, hydrogen bonding and hydrophobic interaction, which will not essentially change the chemical structure between TTR and ligands.

### Example 25: Determination of dynamic specific binding parameters of TTR and each potential ligand molecule

The affinity binding equilibrium dissociation constant K_{d} of TTR and each ligand molecule or salts thereof described in Example 24 was determined by nano ITC (TA). In 10 µmol/L TTR solution (1000 µL), 100 µmol/L of each ligand solution described above was dropped at the velocity of 1 µL/min, and the affinity binding equilibrium dissociation constant K_{d} was calculated using built-in software (Table 28).

**Table 28 The affinity binding equilibrium dissociation constant K_{d} of each ligand molecule and TTR**

| Concentration of TTR (10 µmol/L) | Diclofenac sodium (purchased from SinoPharm) | Vitamin A (purchased from SinoPharm, with Serial No.: CATOCCHM700908, CAS: 68-26-8) | Vitamin A2 (purchased from J & K Scientific Co., Ltd., D230075) | Luteoloside (purchased from SinoPharm) |
|---|---|---|---|---|
| Affinity binding equilibrium dissociation constants (mol/L) | 4.5×10⁻⁸ | 2.27×10⁻⁸ | 2.35×10⁻⁸ | 1.29×10⁻⁷ |

It is shown in Table 28 that the affinity binding equilibrium dissociation constant of each ligand molecule to TTR is nearly 10⁻⁸ mol/L, which is close to the capability of a monoclonal antibody to recognized single antigen epitope, indicating that the ligand molecules described above can recognize and bind TTR specifically.

### Example 26: The complex of human TTR-ligand molecule enters the vitreous body and fundus oculi through the corneal barrier

The human TTR prepared in Example 5 was prepared as 10 µmol/L (containing saline, 2% hyaluronic acid with low molecular weight and 10 µmol/L diclofenac sodium/5 µmol/L vitamin A+5% (v/v) Tween 80/5 µmol/L luteoloside (the addition amount refers to the *in silicon* simulation results in Example 24)), and was administered by eye dropping to C57BL/6 mice (8 weeks old) and SD rats (8 weeks old) respectively. The animals were sacrificed after treatment for 3-72 h, and the proteins were extracted from the vitreous body and fundus oculi samples. The rabbit anti-His-tag antibody was used as the primary antibody and donkey anti-rabbit antibody was used as the secondary antibody. The content of human TTR in the vitreous body and fundus oculi samples from C57BL/6 mice and SD rats was determined by ELISA. The content of diclofenac sodium in a sample was determined according to the item of diclofenac sodium, page 115, Volume II, Pharmacopoeia of the People's Republic of China (2020); the content of vitamin A in a sample was determined according to the item of vitamin A, page 1472, Volume II, Pharmacopoeia of the People's Republic of China (2020); the content of luteoloside in a sample was determined according to the item of Luteolin-7-glucoside, British Pharmacopoeia BP2017.

After eye dropping, the half-life of human TTR and each ligand molecule content in the vitreous body and fundus oculi samples of C57BL/6 mice and SD rats was close to 60 h, indicating that they can effectively exist in the vitreous body and fundus oculi samples for 60 h, with a sufficient therapeutic concentration and therapeutic duration (Table 29).

**Table 29 Human TTR and ligand molecules entering the vitreous body and fundus oculi through the corneal barrier of C57BL/6 mice and SD rats**

| The peaks of human TTR and diclofenac sodium contents in vitreous body and fundus oculi were reached 3 h after eye dropping using human recombinant TTR (10 (µmol/L) (in saline solution added with 2% hyaluronic acid and 10 µmol/L diclofenac sodium), and the human TTR and diclofenac sodium contents thereof at different times thereafter are shown as below | | | | | |
|---|---|---|---|---|---|
| Sampling time after eye dropping (h) | 3 | 18 | 36 | 60 | 72 |
| Concentration of human TTR in vitreous body and fundus oculi samples of C57BL/6 mice (µmol/L) | 2.72± 0.21 | 1.97± 0.16 | 1.69± 0.14 | 1.37± 0.10 | 0.85± 0.10 |
| Concentration of diclofenac sodium in vitreous body | 2.54± | 1.81± | 1.55± | 1.28± | 0.81± |
| and fundus oculi samples of C57BL/6 mice (µmol/L) | 0.17 | 0.13 | 0.10 | 0.10 | 0.10 |
| Concentration of human TTR in vitreous body and fundus oculi samples of SD rats (µmol/L) | 3.22± 0.25 | 2.81± 0.28 | 1.93± 0.20 | 1.65± 0.15 | 0.93± 0.07 |
| Concentration of diclofenac sodium in vitreous body and fundus oculi samples of SD rats (µmol/L) | 3.01± 0.19 | 2.66± 0.21 | 1.74± 0.15 | 1.48± 0.10 | 0.92± 0.08 |

| The peaks of human TTR and vitamin A contents in vitreous body and fundus oculi were reached 3 h after eye dropping using human recombinant TTR (10 (µmol/L) (in saline solution added with 2% hyaluronic acid, 5 µmol/L vitamin A and 5% Tween 80), and the human TTR and vitamin A contents thereof at different times thereafter are shown as below | | | | | |
|---|---|---|---|---|---|
| Sampling time after eye dropping (h) | 3 | 18 | 36 | 60 | 72 |
| Concentration of human TTR in vitreous body and fundus oculi samples of C57BL/6 mice (µmol/L) | 2.83± 0.30 | 2.04± 0.18 | 1.77± 0.15 | 1.44± 0.13 | 1.02± 0.10 |
| Concentration of vitamin A in vitreous body and fundus oculi samples of C57BL/6 mice (µmol/L) | 1.52± 0.11 | 0.96± 0.10 | 0.85± 0.09 | 0.73± 0.07 | 0.60± 0.04 |
| Concentration of human TTR in vitreous body and fundus oculi samples of SD rats (µmol/L) | 3.34± 0.27 | 2.99± 0.30 | 2.06± 0.22 | 1.70± 0.14 | 1.23± 0.10 |
| Concentration of vitamin A in vitreous body and fundus oculi samples of SD rats (µmol/L) | 1.68± 0.11 | 1.53± 0.10 | 1.02± 0.10 | 0.88± 0.06 | 0.65± 0.06 |

| The peaks of human TTR and luteoloside contents in vitreous body and fundus oculi were reached 3 h after eye dropping using human recombinant TTR (10 (µmol/L) (in saline solution added with 2% hyaluronic acid and 5 µmol/L luteoloside), and the human TTR and luteoloside contents thereof at different times thereafter are shown as below | | | | | |
|---|---|---|---|---|---|
| Sampling time after eye dropping (h) | 3 | 18 | 36 | 60 | 72 |
| Concentration of human TTR in vitreous body and fundus oculi samples of C57BL/6 mice (µmol/L) | 2.56± 0.22 | 1.97± 0.20 | 1.54± 0.13 | 1.30± 0.10 | 0.90± 0.06 |
| Concentration of luteoloside in vitreous body and fundus oculi samples of C57BL/6 mice (µmol/L) | 1.20± 0.10 | 0.92± 0.08 | 0.72± 0.05 | 0.60± 0.06 | 0.40± 0.00 |
| Concentration of human TTR in vitreous body and fundus oculi samples of SD rats (µmol/L) | 3.12± 0.25 | 2.80± 0.25 | 2.01± 0.20 | 1.57± 0.10 | 1.01± 0.05 |
| Concentration of luteoloside in vitreous body and | 1.48± | 1.35± | 0.96± | 0.71± | 0.52± |
| fundus oculi samples of SD rats (µmol/L) | 0.10 | 0.10 | 0.05 | 0.05 | 0.00 |

### Example 27: treating DR (diabetic retinopathy) SD rats with human TTR-ligand molecule complex in the manner of eye dropping

8-week-old SD rats weighing 200-250 g, was fasted for 12-18 h, and treated with intraperitoneally injection of 2% STZ (60 mg/kg), followed by cutting the tail and collecting blood thereof after 48 h and 72 h. The blood glucose was detected higher than 16.7 mM by test paper, indicating that the model was successfully established, and DR SD rats were obtained. DR SD rats were divided into 5 groups, one is DR SD rats without any treatment (5 rats), and the other 4 groups each has 5 rats, and the rats were treated with eye dropping twice per day for the left eye and the right eye, 30 µL each time. Wherein, the left eye was dropped with human TTR prepared in Example 5, or human TTR prepared in Example 5 added with the ligand, specifically with 10 µmol/L human TTR (saline solution+2% hyaluronic acid), 10 µmol/L human TTR (saline solution+2% hyaluronic acid+10 µmol/L diclofenac sodium), 10 µmol/L human TTR (saline solution+2% hyaluronic acid+5 µmol/L vitamin A+5% Tween 80) and 10 µmol/L human TTR (saline solution+2% hyaluronic acid+5 µmol/L luteoloside); the right eye was dropped with saline solution+2% hyaluronic acid, or saline solution +2% hyaluronic acid+ligand as control, specifically with saline solution+2% hyaluronic acid, saline solution+2% hyaluronic acid+10 µmol/L diclofenac sodium, saline solution+2% hyaluronic acid+5 µmol/L vitamin A+5% Tween 80 and saline solution+2% hyaluronic acid+5 µmol/L luteoloside. In addition, there was another group of normal SD rats as a normal control group (5 rats). All SD rats were fed for 3 months, followed by stripping the retina for staining with Evans Blue (EB) to observe the retinal vascular leakage, for Trypsin enzymatic hydrolysis to observe the density of neovascularization.

The results show that compared with the normal control, retinal vascular leakage and the number of neovascularization were significantly increased in DR SD rats control group without any treatment after feeding STZ induced SD rats for 3 months, while the eyeball retinal leakage condition of groups treated with human TTR or human TTR/diclofenac sodium, human TTR/vitamin A, human TTR/luteoloside was significantly inhibited, and the number of retinal neovascularization was reduced significantly, indicating the clinical pathological condition of DR had been alleviated (Table 30).

**Table 30 DR pathological condition of SD rats induced by STZ treated with human TTR/human TTR-ligand molecule complex**

| The DR pathological condition of SD rats induced by STZ after 3 months of eye dropping treatment using human recombinant TTR (in saline solution added with 2% hyaluronic acid, and no ligand/10 µmol/L diclofenac sodium/5 µmol/L vitamin A+5% Tween 80/5 µmol/L luteoloside). | | | | | | |
|---|---|---|---|---|---|---|
| | normal control eye | no TTR dropping control | human TTR (no ligand) | human TTR/diclofe nac sodium | human TTR/vita min A | human TTR/lut eoloside |
| Concentration of TTR for eye dropping (µmol/L) | - | 0 | 10 | 10 | 10 | 10 |
| Retinal leakage area (%) | 4.9±0. 5 | 29.4±3.1 | 5.5±0.4 | 2.2±0.1 | 3.2±0.2 | 4.5±0.3 |
| No. of retinal neovascularization (10 visual fields) | 11±2 | 77±6 | 11±1 | 7±2 | 7±3 | 9±2 |

### Example 28: treating AMD (age-related macular degeneration) C57BL/6 mice with human TTR-excipient in the manner of eye dropping

9-week-old C57BL/6 mice were subject to retinal photocoagulation by krypton laser (647 nm), with a power of 360 mW, a diameter of 50 µm, a time of 0.05 s, 8 photocoagulation site for each eye, for inducing the neovascularization in choroid, and gradually proceeding to retinal hyperplasia to obtain AMD C57BL/6 mice. AMD C57BL/6 mice were divided into 5 groups, one is AMD C57BL/6 mice without any treatment (5 mice), and the other 4 groups each has 5 mice, and the mice were treated with eye dropping twice per day for the left eye and the right eye, 30 µL each time. Wherein, the left eye was dropped with human TTR prepared in Example 5, or human TTR prepared in Example 5 added with the ligand, specifically with 10 µmol/L human TTR (saline solution+2% hyaluronic acid), 10 µmol/L human TTR (saline solution+2% hyaluronic acid+10 µmol/L diclofenac sodium), 10 µmol/L human TTR (saline solution+2% hyaluronic acid+5 µmol/L vitamin A+5% Tween 80) and 10 µmol/L human TTR (saline solution+2% hyaluronic acid+5 µmol/L luteoloside); the right eye was dropped with saline solution+2% hyaluronic acid, or saline solution +2% hyaluronic acid+ligand as control, specifically with saline solution+2% hyaluronic acid, saline solution+2% hyaluronic acid+10 µmol/L diclofenac sodium, saline solution+2% hyaluronic acid+5 µmol/L vitamin A+5% Tween 80 and saline solution+2% hyaluronic acid+5 µmol/L luteoloside. In addition, there was another group of normal C57BL/6 mice as a normal control group (5 mice). The animals were sacrificed after eye dropping for 2 weeks, and the retina was stripped for EB staining to observe the retinal leakage conditions, and for Trypsin enzymatic hydrolysis to observe the density of neovascularization.

The results are shown in Table 31. In Table 31, it shows that compared with the normal control, retinal vascular leakage and the number of neovascularization were significantly increased in AMD C57BL/6 mice control group without any treatment, while the eyeball retinal leakage condition and neovascularization condition of groups treated with human TTR or human TTR/diclofenac sodium, human TTR/vitamin A, human TTR/luteoloside were significantly alleviated.

**Table 31 AMD pathological condition of C57BL/6 mice induced by laser retinal photocoagulation treated with human TTR/human TTR-ligand molecule complex**

| The AMD pathological condition of C57BL/6 mice induced by laser retinal photocoagulation after 2 weeks of eye dropping treatment using human recombinant TTR (in saline solution added with 2% hyaluronic acid, and no ligand/10 µmol/L diclofenac sodium/5 µmol/L vitamin A+5% Tween 80/5 µmol/L luteoloside). | | | | | | |
|---|---|---|---|---|---|---|
| | normal control eye | no TTR dropping control | human TTR (no ligand) | human TTR/diclo fenac sodium | human TTR/vita min A | human TTR/lute oloside |
| Concentration of TTR for eye dropping (µmol/L) | - | 0 | 10 | 10 | 10 | 10 |
| Retinal leakage area (%) | 3.9±0.4 | 24.7±2.2 | 4.5±0.3 | 2.8±0.1 | 3.2±0.1 | 4.4±0.35 |
| No. of retinal neovascularization (10 visual fields) | 11±3 | 80±7 | 12±2 | 6±3 | 7±2 | 12±1 |

### Example 29: treating ROP (retinopathy of prematurity) SD rats with human TTR-ligand molecule complex in the manner of eye dropping

One-week-old SD suckling rats were fed in hyperbaric oxygen chamber, and the normal control group was fed in normal environment (normal control group, 5 rats). The rats were taken out after feeding for 5 days in hyperbaric oxygen chamber to obtain ROP SD rats. All ROP SD rats and rats of normal control group were fed in normal environment for succeeding 5 days. In the 5 days, ROP SD rats were divided into 5 groups, one is ROP SD rats without any treatment (5 rats), and the other 4 groups each has 5 rats, and the rats were treated with eye dropping once per day for the left eye and the right eye, 30 µL each time. Wherein, the left eye was dropped with human TTR prepared in Example 5, or human TTR prepared in Example 5 added with the ligand, specifically with 10 µmol/L human TTR (saline solution+2% hyaluronic acid), 10 µmol/L human TTR (saline solution+2% hyaluronic acid+10 µmol/L diclofenac sodium), 10 µmol/L human TTR (saline solution+2% hyaluronic acid+5 µmol/L vitamin A+5% Tween 80) and 10 µmol/L human TTR (saline solution+2% hyaluronic acid+5 µmol/L luteoloside); the right eye was dropped with saline solution+2% hyaluronic acid, or saline solution +2% hyaluronic acid+ligand as control, specifically with saline solution+2% hyaluronic acid, saline solution+2% hyaluronic acid+10 µmol/L diclofenac sodium, saline solution+2% hyaluronic acid+5 µmol/L vitamin A+5% Tween 80 and saline solution+2% hyaluronic acid+5 µmol/L luteoloside. The animals were sacrificed after eye dropping for 5 days, and the retina was stripped for EB staining to observe the retinal leakage conditions.

The results are shown in Table 32. It is shown in the table that compared with the normal control, retinal vascular leakage were significantly increased in ROP SD rats control group without any treatment, while the eyeball retinal leakage condition of groups treated with human TTR or human TTR/diclofenac sodium, human TTR/vitamin A, human TTR/luteoloside were significantly alleviated.

**Table 32 ROP pathological condition of SD suckling rats induced by hyperbaric oxygen chamber treated with human TTR/human TTR-ligand molecule complex**

| The ROP pathological condition of SD suckling rats induced by hyperbaric oxygen chamber after 5 days of eye dropping treatment using human recombinant TTR (in saline solution added with 2% hyaluronic acid, and no ligand/10 µmol/L diclofenac sodium/5 µmol/L vitamin A+5% Tween 80/5 µmol/L luteoloside). | | | | | | |
|---|---|---|---|---|---|---|
| | normal control eye | no TTR dropping control | human TTR (no ligand) | human TTR/diclofe nac sodium | human TTR/vita min A | human TTR/lut eoloside |
| Concentration of TTR for eye dropping (µmol/L) | - | 0 | 10 | 10 | 10 | 10 |
| Retinal leakage area (%) | 3.3±0.3 | 44.7±5.1 | 3.4±0.2 | 2.0±0.1 | 2.3±0.1 | 4.0±0.3 |

Combining the data of Examples 27-29, it can be seen that even in normal SD rats, the retinal leakage phenomenon is more severe in adult SD rats than that in juvenile SD rats. In addition, other causes such as experimental operations and individual difference, also can lead to retinal leakage and increased number of neovascularization, which indicates that even normal control eyes without modeling have a certain degree of retinal leakage and increased neovascularization. By using human TTR/diclofenac sodium or human TTR/vitamin A, it can not only treat DR, AMD and ROP, but also have the effect of nourishment, which has certain alleviation effect on retinal leakage and increased neovascularization caused by other reasons in an eye itself.

### Comparative example 1: Human TTR is recombinant expressed with different plasmids

(1) Construction of recombinant plasmids pET-28a-*ttr*, pQE-30-*ttr* and pQE-60-*ttr*: the optimized DNA sequence (as shown in SEQ ID NO: 2) of mature TTR was ligated to plasmid pET-28a (pET-28a, pQE-30 and pQE-60 all purchased from ATCC) by two restriction enzyme sites *Nde* I and *Hind* III to construct plasmid pET-28a-*ttr*; the optimized DNA sequence (as shown in SEQ ID NO: 2) of mature TTR was ligated to plasmid pQE-30 by two restriction enzyme sites *BamH* I and *Hind* III to construct plasmid pQE-30-*ttr*; the optimized DNA sequence (as shown in SEQ ID NO: 2) of mature TTR was ligated to plasmid pQE-60 by two restriction enzyme sites *EcoR* I and *Hind III* to construct plasmid pQE-60-*ttr*. The plasmids were subject to be verified successful constructions by sequencing (the sequencing was performed by Nanjing GenScript Biotechnology Ltd.).
(2) Expression and purification of recombinant human TTR: plasmids pET-28a-*ttr*, pQE-30-*ttr* and pQE-60-*ttr* constructed in step (1) and pETx-rhaPBAD-*ttr* constructed in Example 5 were transformed into *E. coli* BL21 (DE3) cells, and the recombinant *E. coli* BL21 (DE3) cells obtained were cultured in LB medium to prepare an inoculum, then inoculating 5% of the inoculum into 5 L of TB medium, incubating at a temperature of 37°C and paddle speed of 150 rpm until OD₆₀₀ of the culture reaches 1.5-2.0; 0.2 mM IPTG was added into the former three media to induce for 18 h, and 1.6% rhamnose was added into the last medium to induce for 18 h. The bacteria was broken by high pressure homogenization, and human TTR was prepared by Ni+ column chromatography of the supernatant thereof. The endotoxin of the protein obtained was removed by an endotoxin absorption column (Pierce^{™} High Capacity Endotoxin Removal Spin Columns, ThermoFisher) and the residue bacteria was removed by a filter membrane with a pore size of 0.22 µm. The human TTR protein yield was shown in Table 33.

**Table 33 Human TTR is recombinant expressed with different plasmids**

| The protein yields were obtained by adding 0.2 mM IPTG or 1.6% rhamnose when the bacteria were cultured to an OD₆₀₀ of 1.8 and induced at a temperature of 37°C for 18 h. | | | | |
|---|---|---|---|---|
| Plasmid | *pET-28a-ttr* | *pQE-30-ttr* | *pQE-60-ttr* | pETx- rhaPBAD-*ttr* |
| Protein yield (mg/g wet cells) | no soluble expression | no soluble expression | no soluble expression | 50.9±4.8 |

It can be known from the results described above that, when the protein is expressed using pET-28a, pQE-30, pQE-60, etc., insoluble expression occurred.

### Comparative example 2:

Compare with Table 7-1 in Example 7, when the concentration of TTR for eye dropping is 1 µmol/L, the concentration of TTR in the vitreous body and fundus oculi is only 0.10±0.00 µmol/L.

### Comparative example 3:

The detailed operation manner is the same with Example 9 except the difference that, GFP protein (Genbank Accession No.: QAA95705.1) not fused with TTR was expressed according to the method described in Example 2, followed by eye dropping test on SD rats and New Zealand big-ear rabbits according to the operation steps which are the same with Example 9. The results show that GFP did not enter the vitreous body of SD rats or New Zealand big-ear rabbits (Table 34 and 35).

**Table 34 Effects of GFP entering the eye of SD rats with different times of drops at the GFP dosage of 0.6 nmol**

| Times of dropping per day | | 1 | 2 | 3 |
|---|---|---|---|---|
| GFP content in vitreous body (nmol/L) | day 2 | - | - | - |
| | day 6 | - | - | - |
| | day 10 | - | - | - |
| | day 14 | - | - | - |

Wherein, "-"represents undetected (the same below).

**Table 35 Effects of GFP entering the eye of New Zealand big-ear rabbits with different times of drops at the GFP dosage of 0.6 nmol**

| Times of dropping per day | | 1 | 2 | 3 |
|---|---|---|---|---|
| GFP content in vitreous body (nmol/L) | day 2 | - | - | - |
| | day 6 | - | - | - |
| | day 10 | - | - | - |
| | day 14 | - | - | - |

### Comparative example 4:

The detailed operation manner is the same with Example 10 except the difference that, Lysozyme protein (Genbank Accession No.: AAL69327.1) not fused with TTR was expressed according to the method described in Example 3, followed by eye dropping test on SD rats and New Zealand big-ear rabbits according to the operation steps which are the same with Example 10. The results show that Lysozyme did not enter the vitreous body of SD rats or New Zealand big-ear rabbits (Table 36 and 37).

**Table 36 Effects of Lysozyme entering the eye of SD rats with different times of drops at the Lysozyme dosage of 0.6 nmol**

| Times of dropping per day | | 1 | 2 | 3 |
|---|---|---|---|---|
| Lysozyme content in vitreous body (nmol/L) | day 2 | - | - | - |
| | day 6 | - | - | - |
| | day 10 | - | - | - |
| | day 14 | - | - | - |

**Table 37 Effects of Lysozyme entering the eye of New Zealand big-ear rabbits with different times of drops at the Lysozyme dosage of 0.6 nmol**

| Times of dropping per day | | 1 | 2 | 3 |
|---|---|---|---|---|
| Lysozyme content in vitreous body (nmol/L) | day 2 | - | - | - |
| | day 6 | - | - | - |
| | day 10 | - | - | - |
| | day 14 | - | - | - |

### Comparative example 5:

The detailed operation manner is the same with Example 11 except the difference that, Ovalbumin protein (UniProt Accession No.: P01012) not fused with TTR was expressed according to the method described in Example 4, followed by eye dropping test on SD rats and New Zealand big-ear rabbits according to the operation steps which are the same with Example 11. The results show that Ovalbumin did not enter the vitreous body of SD rats or New Zealand big-ear rabbits (Table 38 and 39).

**Table 38 Effects of Ovalbumin entering the eye of SD rats with different times of drops at the Ovalbumin dosage of 0.6 nmol**

| Times of dropping per day | | 1 | 2 | 3 |
|---|---|---|---|---|
| Ovalbumin content in vitreous body (nmol/L) | day 2 | - | - | - |
| | day 6 | - | - | - |
| | day 10 | - | - | - |
| | day 14 | - | - | - |

**Table 39 Effects of Ovalbumin entering the eye of New Zealand big-ear rabbits with different times of drops at the Ovalbumin dosage of 0.6 nmol**

| Times of dropping per day | | 1 | 2 | 3 |
|---|---|---|---|---|
| Ovalbumin content in vitreous body (nmol/L) | day 2 | - | - | - |
| | day 6 | - | - | - |
| | day 10 | - | - | - |
| | day 14 | - | - | - |

### Comparative example 6:

(1) The human TTR prepared in Example 5 was prepared as 5-30 µmol/L (containing saline), and was administered by eye dropping to C57BL/6 mice (8 weeks old) and SD rats (8 weeks old) respectively. The animals were sacrificed after treatment for 3 h, and the proteins were extracted from the vitreous body and fundus oculi samples. The rabbit anti-His-tag antibody was used as the primary antibody and donkey anti-rabbit antibody was used as the secondary antibody. The content of human TTR in the vitreous body and fundus oculi samples from C57BL/6 mice and SD rats was determined by ELISA.
(2) The human TTR prepared in Example 5 was prepared as 10 µmol/L (containing saline and 0-8 mg/mL PEG400 (purchased from SinoPharm, molecular weight of 360-440)), and was administered by eye dropping to C57BL/6 mice (8 weeks old) and SD rats (8 weeks old) respectively. The animals were sacrificed after treatment for 3-72 h, and the proteins were extracted from the vitreous body and fundus oculi samples. The rabbit anti-His-tag antibody was used as the primary antibody and donkey anti-rabbit antibody was used as the secondary antibody. The content of human TTR in the vitreous body and fundus oculi samples from C57BL/6 mice and SD rats was determined by ELISA.

The results show that the content of human TTR in the vitreous body and fundus oculi samples reached the peak 3 h after eye dropping; when PEG400 was added to the eye drops, the content of human TTR in the vitreous body and fundus oculi samples were both increased by more than 20%, and when the content of PEG400 was 6 mg/mL, the content of human TTR in the vitreous body and fundus oculi samples reached the peak 3 h after eye dropping; after eye dropping, the content half-life of human TTR in the vitreous body and fundus oculi samples of C57BL/6 mice and SD rats was close to 60 h, indicating that it can effectively exist in the vitreous body and fundus oculi samples for 60 h, with a sufficient therapeutic concentration and therapeutic duration (Table 40).

Combining the data of Examples 21 and 22, it can be seen that PEG400 can effectively promote the content of TTR in the vitreous body and fundus oculi samples, but it has comparatively poor therapeutic effects when used to treat DR rats or AMD mice. It is indicated that the increasing permeation of TTR does not mean the improvement of therapeutic effect. TTR, the appropriate permeation of TTR and appropriate excipient should mutual check and synergistically cooperate to form an organic whole, thus eventually achieving a better therapeutic effect.

In addition, it can be known from Table 25 and Table 27, even in normal SD rats, the retinal leakage phenomenon is more severe in adult SD rats than that in juvenile SD rats. In addition, other causes such as experimental operations and individual difference, also can lead to retinal leakage and increased number of neovascularization, which indicates that even normal control eyes without modeling have a certain degree of retinal leakage and increased neovascularization. By using TTR combined with chondroitin sulfate A sodium salt, it can not only treat DR, AMD and ROP, but also have the effect of nourishment, which has certain alleviation effect on retinal leakage and increased neovascularization caused by other reasons in an eye itself.

**Table 40 Human TTR entering the vitreous body and fundus oculi through the corneal barrier of C57BL/6 mice and SD rats**

| (1) The human TTR content in vitreous body and fundus oculi 3 h after eye dropping using human recombinant TTR (in saline solution). | | | | | | |
|---|---|---|---|---|---|---|
| Concentration of TTR drops (µmol/L) | 5 | 10 | 15 | 20 | 25 | 30 |
| Concentration of human TTR in vitreous body and fundus oculi samples of C57BL/6 mice (µmol/L) | 0.84± 0.07 | 1.81± 0.19 | 1.75± 0.20 | 1.83± 0.21 | 1.88± 0.17 | 1.87± 0.20 |
| Concentration of human TTR in vitreous body and fundus oculi samples of SD rats (µmol/L) | 1.14± 0.11 | 2.43± 0.21 | 2.37± 0.30 | 2.42± 0.36 | 2.45± 0.42 | 2.44± 0.36 |

| (2) The human TTR content in vitreous body and fundus oculi 3 h after eye dropping using human recombinant TTR (10 (µmol/L) (in saline solution added with PEG400 of different concentration). | | | | | | |
|---|---|---|---|---|---|---|
| Concentration of PEG400 (mg/mL) | 0 | 2 | 4 | 6 | 8 | |
| Concentration of human TTR in vitreous body and fundus oculi samples of C57BL/6 mice (µmol/L) | 1.81± 0.19 | 2.29± 0.18 | 2.83± 0.21 | 3.02± 0.15 | 3.00±0.09 | |
| Concentration of human TTR in vitreous body and fundus oculi samples of SD rats (µmol/L) | 2.43± 0.21 | 2.99± 0.21 | 3.33± 0.29 | 3.51± 0.32 | 3.47±0.36 | |

| (3) The peak of the human TTR content in vitreous body and fundus oculi was reached 3 h after eye dropping using human recombinant TTR (10 (µmol/L) (in saline solution added with 6 mg/mL PEG400), and the human TTR content thereof at different times thereafter are shown as below | | | | | | |
|---|---|---|---|---|---|---|
| Sampling time after eye dropping (h) | 3 | 18 | 36 | 60 | 72 | |
| Concentration of human TTR in vitreous body and fundus oculi samples of C57BL/6 mice (µmol/L) | 3.02± 0.15 | 2.61± 0.20 | 2.01± 0.18 | 1.50± 0.12 | 1.00±0.09 | |
| Concentration of human TTR in vitreous body and fundus oculi samples of SD rats (µmol/L) | 3.51± 0.32 | 3.04± 0.21 | 2.13± 0.20 | 1.73± 0.11 | 1.14±0.10 | |

### Comparative example 7:

The detailed operation manner is the same with Example 26 except the difference that, each ligand molecule not binding to TTR was used to conduct the eye dropping test on C57BL/6 mice (8 week old) and SD rats (8 week old) according to the operation steps which are the same with Example 26. The results show that each ligand molecule could not enter independently the vitreous body and fundus oculi of C57BL/6 mice and SD rats (Table 41).

**Table 41 Each ligand molecule entering the vitreous body and fundus oculi through the corneal barrier of C57BL/6 mice and SD rats**

| The content of each ligand in the vitreous body and fundus oculi at different times after eye dropping using saline solution added with 2% hyaluronic acid, and 1 mmol/L diclofenac sodium/1 mmol/L vitamin A+5% Tween 80/1 mmol/L luteoloside. | | | | | |
|---|---|---|---|---|---|
| Sampling time after eye dropping (h) | 3 | 18 | 36 | 60 | 72 |
| Concentration of diclofenac sodium in vitreous body and fundus oculi samples of C57BL/6 mice (µmol/L) | - | - | - | - | - |
| Concentration of vitamin A in vitreous body and fundus oculi samples of C57BL/6 mice (µmol/L) | - | - | - | - | - |
| Concentration of luteoloside in vitreous body and fundus oculi samples of C57BL/6 mice (µmol/L) | - | - | - | - | - |
| Concentration of diclofenac sodium in vitreous body and fundus oculi samples of SD rats (µmol/L) | - | - | - | - | - |
| Concentration of vitamin A in vitreous body and fundus oculi samples of SD rats (µmol/L) | - | - | - | - | - |
| Concentration of luteoloside in vitreous body and fundus oculi samples of SD rats (µmol/L) | - | - | - | - | - |

### Comparative example 8:

The detailed operation manner is the same with Example 27, and the results of eye dropping in the right eye are shown in Table 42. The results show that each ligand molecule could not improve the DR pathological condition of SD rats induced by STZ with eye dropping independently (Table 42).

**Table 42 DR pathological condition of SD rats induced by STZ treated with each ligand independently**

| The DR pathological condition of SD rats induced by STZ after 3 months of eye dropping treatment using saline solution added with 2% hyaluronic acid, and 10 µmol/L diclofenac sodium/5 µmol/L vitamin A+5% Tween 80/5 µmol/L luteoloside | | | | | |
|---|---|---|---|---|---|
| | normal control eye | no dropping control | diclofen ac sodium | vitamin A | luteolosid e |
| Concentration of eye dropping (µmol/L) | - | 0 | 10 | 5 | 5 |
| Retinal leakage area (%) | 4.9±0.5 | 29.4±3.1 | 30.4±3.1 | 28.9±3.0 | 27.6±4.1 |
| No. of retinal neovascularization (10 visual fields) | 11±2 | 77±6 | 80±9 | 76±8 | 79±9 |

### Comparative example 9:

The detailed operation manner is the same with Example 28, and the results of eye dropping in the right eye are shown in Table 43. The results show that each ligand molecule could not improve the AMD pathological condition of C57BL/6 mice induced by laser retinal photocoagulation with eye dropping independently (Table 43).

**Table 43 AMD pathological condition of C57BL/6 mice induced by laser retinal photocoagulation treated with each ligand independently**

| The AMD pathological condition of C57BL/6 mice induced by laser retinal photocoagulation after 2 weeks of eye dropping treatment using saline solution added with 2% hyaluronic acid, and 10 µmol/L diclofenac sodium/5 µmol/L vitamin A+5% Tween 80/5 µmol/L luteoloside | | | | | |
|---|---|---|---|---|---|
| | normal control eye | no dropping control | diclofen ac sodium | vitamin A | luteolosid e |
| Concentration of eye dropping (µmol/L) | - | 0 | 10 | 5 | 5 |
| Retinal leakage area (%) | 3.9±0.4 | 24.7±2.2 | 25.2±3.0 | 26.7±3.4 | 21.3±2.2 |
| No. of retinal neovascularization (10 visual fields) | 11±3 | 80±7 | 85±6 | 79±10 | 82±8 |

### Comparative example 10:

The detailed operation manner is the same with Example 29, and the results of eye dropping in the right eye are shown in Table 44. The results show that each ligand molecule could not improve the ROP pathological condition of SD suckling rats induced by hyperbaric oxygen chamber with eye dropping independently (Table 44).

**Table 44 ROP pathological condition of SD suckling rats induced by hyperbaric oxygen chamber treated with each ligand independently**

| The ROP pathological condition of SD suckling rats induced by hyperbaric oxygen chamber after 5 days of eye dropping treatment using saline solution added with 2% hyaluronic acid, and 10 µmol/L diclofenac sodium/5 µmol/L vitamin A+5% Tween 80/5 µmol/L luteoloside | | | | | |
|---|---|---|---|---|---|
| | normal control eye | no dropping control | diclofenac sodium | vitamin A | luteoloside |
| Concentration of eye dropping (µmol/L) | - | 0 | 10 | 5 | 5 |
| Retinal leakage area (%) | 3.3±0.3 | 44.7±5.1 | 48.5±5.8 | 46.9±4.9 | 51.2±6.8 |

### Comparative example 11:

Sulfamethoxazole has a broad antibacterial spectrum and strong antibacterial effect. It can block the growth of bacteria, particularly effective against Staphylococcus and Escherichia coli; it is suitable for respiratory, urinary and intestinal infections; it is mainly used to treat avian cholera, etc. It can be used to prepare an eye drops such as compound sulfamethoxazole sodium eye drops (this product is a compound preparation, each 10 mL contains 400 mg sulfamethoxazole sodium, 200 mg aminocaproic acid, 10 mg dipotassium glycyrrhizinate, 2 mg chlorpheniramine maleate. It is mainly used for bacterial conjunctivitis, blepharitis (stye) and bacterial blepharitis caused by sensitive bacteria.

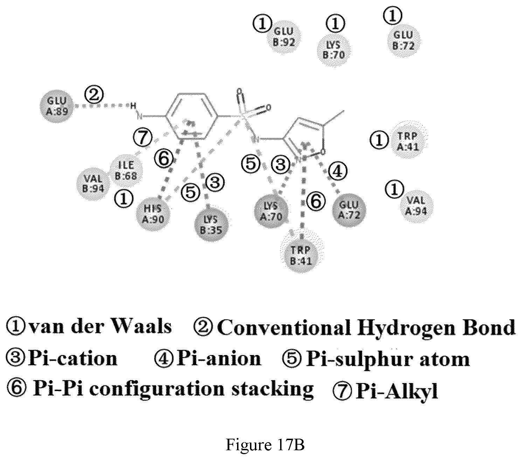

Through molecular simulation with Discovery studio software, it is found that sulfamethoxazole can bind stably to a TTR polymer. In Figure 17A, the protein structure is TTR dimer, sulfamethoxazole ligand molecule is represented by arrows, and one molecule of TTR dimer can bind to one molecule of sulfamethoxazole. Figure 17B shows the interaction between sulfamethoxazole and amino acid residues of TTR.

In 10 µmol/L TTR solution (1000 µL), 100 µmol/L of sulfamethoxazole solution was dropped at the velocity of 1 µL/min according to the steps described in Example 25, and the affinity binding equilibrium dissociation constant K_{d} was calculated with built-in software as 7.03×10⁻⁸ mol/L.

According to the steps described in Example 26, TTR/sulfamethoxazole sodium salts was used to treat rats and mice with eye dropping, and after 3-72 h,the cornea of rats and mice was damaged, i.e., sulfamethoxazole sodium salts used with TTR would burn the cornea and is not biologically safe. It can be seen that after screening for ligands having biological activity, having anti-inflammatory effects and having the ability to bind to TTR by molecular simulation with Discovery studio software, not all of the ligands can improve the therapeutic effect when verified by experiments.

Although the specific embodiments of the present disclosure are described above, those skilled in the art should understand that these are only examples, and various changes or modifications can be made to these embodiments without departing from the principle and essence of the present disclosure. Therefore, the protection scope of the present disclosure is defined by the appended claims.

## Claims

1. A use of transthyretin as a carrier for a protein and/or polypeptide medicament entering an eye through an ocular barrier, wherein the transthyretin is represented by (a), (b) or (c):
(a) a protein consisting of an amino acid sequence of SEQ ID NO: 1;
(b) a protein derived from (a) with an inhibitory function of neovascularization, which is shown by a sequence in which one or more amino acids are substituted, deleted or added in the amino acid sequence of (a);
(c) a protein having a sequence with a hydrophilic modification or hydrophobic modification in the amino acid sequence of (a) or (b).

2. The use of claim 1, wherein in (b), the protein derived from (a) is a protein in which 22 amino acids or 25 amino acids are substituted in, or 5 amino acids are deleted from the amino acid sequence of (a);
and/or, in (c), the hydrophilic modification or hydrophobic modification occurs at the cysteine at position 10 of the amino acid sequence of (a);
preferably:
in (b), the protein derived from (a) is a protein in which T3, T5, 126, N27, H31, R34, A36, A37, D38, D39, T40, S50, E61, E63, V65, 168, K70, 173, A81, H90, E92, P102, R104, T123, K126 and/or E127 are substituted in the amino acid sequence of (a); or a protein in which a deletion occurs at positions 123-127 of the amino acid sequence of (a); wherein, the protein derived from (a) is a protein in which T3G, T5A, I26V, N27D, H31K, R34K, A36T, D39G, T40S, S50A, E61D, E63K, V65T, I68V, K70R, I73L, H90Y, P102H, R104H, T123S, K126Q and E127N are preferably substituted in the amino acid sequence of (a); or a protein in which T3G, T5A, I26V, N27D, H31K, R34K, A36T, A37S, D38E, D39G, T40S, S50A, E61D, E63K, I68V, K70R, I73L, A81T, H90F, E92D, P102H, R104H, T123S, K126Q and E127N are substituted in the amino acid sequence of (a);
and/or, in (c), the hydrophobic modification is a modification with a long chain hydrophobic fragment such as *n*-dodecane at the cysteine at position 10 of the amino acid sequence of (a); or, the hydrophobic modification is a modification with *n*-dodecane via maleiamide at the cysteine at position 10 of the amino acid sequence of (a);
more preferably:
in (b), the protein derived from (a) has an amino acid sequence of SEQ ID NO: 9, SEQ ID NO: 10 or SEQ ID NO: 11.

3. The use of claim 1 or 2, wherein the transthyretin is expressed with the protein and/or polypeptide medicament as a fusion; and the fusion is preferably a fusion that the protein and/or polypeptide medicament are fused at the N-terminus or C-terminus of the transthyretin; preferably, the transthyretin is expressed with the protein and/or polypeptide medicament as a fusion in a microbial cell, followed by a purification of the fusion; wherein the purification is preferably to remove endotoxin by an endotoxin absorption column and then remove residue bacteria by a filter membrane with a pore size of 0.22 µm;
and/or, the protein and/or polypeptide medicament comprises lysozyme, albumin and/or EGFR antibody, with a molecular weight of no more than 45 kDa; the lysozyme is preferably hen egg white lysozyme with GenBank Accession No.: AAL69327.1; the albumin is preferably ovalbumin;
and/or, the protein and/or polypeptide medicament comprises a protein and/or polypeptide medicament for treating ocular diseases associated with ocular retina leakage and/or retinal neovascularization such as diabetic retinopathy, age-related macular degeneration and/or r retinopathy of prematurity.

4. The use of any one of claims 1-3, wherein the transthyretin is encoded by a nucleotide sequence of SEQ ID NO: 2;
and/or, the transthyretin is expressed by a recombinant expression vector, wherein the recombinant expression vector has a plasmid backbone comprising a rhamnose inducible promoter, preferably a rhaPBAD promoter;
and/or, the transthyretin is expressed by a recombinant expression vector, and the recombinant expression vector has a plasmid backbone of pET-21a or a vector having 25% or more homology therewith, and the vector having 25% or more homology therewith preferably has a sequence of SEQ ID NO: 8;
and/or, the recombinant plasmid expressing transthyretin has a nucleotide sequence of SEQ ID NO: 3;
and/or, the transthyretin is expressed in a microbial cell, and preferably followed by a purification of the transthyretin; the microbial cell is preferably an *E. coli,* and the *E coli* preferably comprises *E. coli* BL21, *E. coli* BL21 (DE3), *E. coli* JM109, *E*. *coli* DH5α, *E. coli* K12 or *E. coli* TOP10; the purification is preferably to remove endotoxin by an endotoxin absorption column and then remove residue bacteria by a filter membrane with a pore size of 0.22 µm;
and/or, the transthyretin is expressed by culturing the transformant comprising a gene of the transthyretin until the bacteria obtained reaches an OD₆₀₀ of 1.5-2.0, such as 1.6, 1.7, 1.8 or 1.9;
and/or, expression of the transthyretin is induced by a reagent for inducible expression, wherein the reagent for inducible expression has a mass volume percentage of 0.1-2%, such as 0.2%, 0.3%, 0.4%, 0.5%, 0.7%, 0.8%, 1.2% or 1.6%, the expression is preferably induced for a period of 8-20 h, such as 10 h, 12 h, 14 h, 16 h, 17 h, 18 h or 19 h; the reagent for inducible expression is preferably rhamnose or IPTG.

5. A use of transthyretin and/or a fusion protein consisting of a transthyretin and a medicament in the preparation of drops, wherein the medicament is protein and/or polypeptide medicament, and the transthyretin is represented by (a), (b) or (c):
(a) a protein consisting of an amino acid sequence of SEQ ID NO: 1;
(b) a protein derived from (a) with an inhibitory function of neovascularization, which is shown by a sequence in which one or more amino acids are substituted, deleted or added in the amino acid sequence of (a);
(c) a protein having a sequence with a hydrophilic modification or hydrophobic modification in the amino acid sequence of (a) or (b).

6. The use of claim 5, wherein in (b), the protein derived from (a) is a protein in which 22 amino acids or 25 amino acids are substituted in, or 5 amino acids are deleted from the amino acid sequence of (a);
and/or, in (c), the hydrophilic modification or hydrophobic modification occurs at the cysteine at position 10 of the amino acid sequence of (a);
preferably:
in (b), the protein derived from (a) is a protein in which T3, T5, 126, N27, H31, R34, A36, A37, D38, D39, T40, S50, E61, E63, V65, 168, K70, 173, A81, H90, E92, P102, R104, T123, K126 and/or E127 are substituted in the amino acid sequence of (a); or a protein in which a deletion occurs at positions 123-127 of the amino acid sequence of (a); preferably, the protein derived from (a) is a protein in which T3G, T5A, I26V, N27D, H31K, R34K, A36T, D39G, T40S, S50A, E61D, E63K, V65T, I68V, K70R, I73L, H90Y, P102H, R104H, T123S, K126Q and E127N are substituted in the amino acid sequence of (a); or a protein in which T3G, T5A, 126V, N27D, H31K, R34K, A36T, A37S, D38E, D39G, T40S, S50A, E61D, E63K, I68V, K70R, I73L, A81T, H90F, E92D, P102H, R104H, T123S, K126Q and E127N are substituted in the amino acid sequence of (a);
and/or, in (c), the hydrophobic modification is a modification with a long chain hydrophobic fragment such as *n*-dodecane at the cysteine at position 10 of the amino acid sequence of (a); or, the hydrophobic modification is a modification with *n*-dodecane via maleiamide at the cysteine at position 10 of the amino acid sequence of (a);
more preferably:
in (b), the protein derived from (a) has an amino acid sequence of SEQ ID NO: 9, SEQ ID NO: 10 or SEQ ID NO: 11.

7. The use of claim 5 or 6, wherein the fusion protein contained in the drops comprising a transthyretin and a medicament has a content of 4-30 µmol/L, preferably 10-15 µmol/L;
and/or, the transthyretin contained in the drops has a content of 4-30 µmol/L, preferably 5-30 µmol/L, more preferably 10-20 µmol/L, such as 10, 15, 20 µmol/L;
and/or, the drops further comprises saline;
and/or, the drops further comprise a surfactant, wherein the surfactant, for example, is Tween 80, and preferably has a content of 5% (v/v);
and/or, the drops further comprises a pharmaceutically acceptable excipient which is one or more selected from carboxymethyl cellulose or salts thereof, chondroitin sulfate or salts thereof, dextran, and, hyaluronic acid; wherein, the carboxymethyl cellulose or salts thereof preferably has a viscosity of 800-1200 CP; and/or, the chondroitin sulfate is preferably chondroitin sulfate A; and/or, the dextran is preferably dextran 70; and/or, the "salts" in the "carboxymethyl cellulose or salts thereof' or "chondroitin sulfate or salts thereof' is independently preferably sodium salts or calcium salts, such as sodium carboxymethyl cellulose or chondroitin sulfate A sodium salt; and/or, the hyaluronic acid has a preferred molecular weight of 10000-500000; and/or, the carboxymethyl cellulose or salts thereof preferably has a concentration of 0-8 mg/mL except 0, more preferably 2, 4, 6 or 8 mg/mL; and/or, the chondroitin sulfate or salts thereof preferably has a concentration of 0-40 mg/mL except 0, more preferably 10, 20, 30 or 40 mg/mL; and/or, the dextran preferably has a concentration of 0-0.8 mg/mL except 0, more preferably 0.2, 0.4, 0.6 or 0.8 mg/mL; and/or, the hyaluronic acid preferably has a content of no more than 6% by mass volume percentage, preferably 1-4%, more preferably 2%;
and/or, the drops further comprises a compound, pharmaceutically acceptable salts thereof, or glycoside thereof; the compound is one or more selected from diclofenac, vitamin A and luteolin; wherein, the pharmaceutically acceptable salt is preferably a sodium salt, such as diclofenac sodium; and/or, the glycoside is preferably luteoloside; and/or, the vitamin A is preferably vitamin A1 and/or vitamin A2; and/or, the diclofenac or salts thereof has a preferred content of 5-20 µmol/L, such as 10 µmol/L; and/or, the vitamin A has a preferred content of 2-10 µmol/L, such as 5 µmol/L; and/or, the luteolin or glycoside thereof has a preferred content of 2-10 µmol/L, such as 5 µmol/L;
and/or, the drops is preferably an eye drops;
and/or, the drops is a drops that inhibits ocular retina leakage and/or reduces the number of retinal neovascularization; preferably a drops that treats diabetic retinopathy, age-related macular degeneration and/or retinopathy of prematurity;
and/or, the drops is administered 1-3 times per day, preferably at an amount of 0.3-0.8 nmol protein per eye at each time;
and/or, the drops is administered twice per day, one drop each time, for 3 months; and/or, the drops is administered once per day, one drop each time, for 5 days; and/or, the drops is administered twice per day, one drop each time, for 2 weeks;
and/or, the transthyretin is encoded by a nucleotide sequence of SEQ ID NO: 2;
and/or, the transthyretin is expressed by a recombinant expression vector, the recombinant expression vector has a plasmid backbone comprising a rhamnose inducible promoter, preferably a rhaPBAD promoter;
and/or, the transthyretin is expressed by a recombinant expression vector, the recombinant expression vector has a plasmid backbone of pET-21a or a vector with 25% or more homology therewith, and preferably a vector with 25% or more homology therewith has a sequence of SEQ ID NO: 8;
and/or, the recombinant plasmid expressing transthyretin has a nucleotide sequence of SEQ ID NO: 3;
and/or, the transthyretin is expressed in a microbial cell, and preferably followed by a purification of the transthyretin; the microbial cell is preferably an *E. coli,* and the *E. coli* preferably comprises *E. coli* BL21, *E. coli* BL21 (DE3), *E. coli* JM109, *E*. *coli* DH5α, *E. coli* K12 or *E. coli* TOP10; the purification is preferably to remove endotoxin by an endotoxin absorption column and then remove residue bacteria by a filter membrane with a pore size of 0.22 µm;
and/or, the transthyretin is expressed by culturing the transformant comprising a gene of the transthyretin until the bacteria obtained reaches an OD₆₀₀ of 1.5-2.0, such as 1.6, 1.7, 1.8 or 1.9;
and/or, expression of the transthyretin is induced by a reagent for inducible expression, wherein the reagent for inducible expression has a mass volume percentage of 0.1-2%, such as 0.2%, 0.3%, 0.4%, 0.5%, 0.7%, 0.8%, 1.2% or 1.6%, and the expression is preferably induced for a period of 8-20 h, such as 10 h, 12 h, 14 h, 16 h, 17 h, 18 h or 19 h; the reagent for inducible expression is preferably rhamnose or IPTG;
and/or, the fusion protein has a sequence of SEQ ID NO: 6 or SEQ ID NO: 7;
and/or, the transthyretin is expressed with the protein and/or polypeptide medicament as a fusion; the fusion is preferably a fusion that the protein and/or polypeptide medicament are fused at the N-terminus or C-terminus of the transthyretin; preferably, the transthyretin is expressed with the protein and/or polypeptide medicament as a fusion in a microbial cell, followed by a purification of the fusion; wherein the purification is preferably to remove endotoxin by an endotoxin absorption column and then remove residue bacteria by a filter membrane with a pore size of 0.22 µm;
and/or, the protein and/or polypeptide medicament comprises lysozyme, albumin and/or EGFR antibody, with a molecular weight of no more than 45 kDa; the lysozyme is preferably hen egg white lysozyme with GenBank Accession No.: AAL69327.1; the albumin is preferably ovalbumin;
and/or, the protein and/or polypeptide medicament comprises a protein and/or polypeptide medicament for treating ocular diseases associated with ocular retina leakage and/or retinal neovascularization such as diabetic retinopathy, age-related macular degeneration and/or retinopathy of prematurity.

8. A drops comprising transthyretin and/or a fusion protein consisting of a transthyretin and a medicament; wherein the medicament is protein and/or polypeptide medicament, and the transthyretin is represented by (a), (b) or (c):
(a) a protein consisting of an amino acid sequence of SEQ ID NO: 1;
(b) a protein derived from (a) with an inhibitory function of neovascularization, which is shown by a sequence in which one or more amino acids are substituted, deleted or added in the amino acid sequence of (a);
(c) a protein having a sequence with a hydrophilic modification or hydrophobic modification in the amino acid sequence of (a) or (b).

9. (Currently Amended) The drops of claim 8, wherein in (b), the protein derived from (a) is a protein in which 22 amino acids or 25 amino acids are substituted in, or 5 amino acids are deleted from the amino acid sequence of (a);
and/or, in (c), the hydrophilic modification or hydrophobic modification occurs at the cysteine at position 10 of the amino acid sequence of (a);
preferably:
in (b), the protein derived from (a) is a protein in which T3, T5, 126, N27, H31, R34, A36, A37, D38, D39, T40, S50, E61, E63, V65, 168, K70, 173, A81, H90, E92, P102, R104, T123, K126 and/or E127 are substituted in the amino acid sequence of (a); or a protein in which a deletion occurs at positions 123-127 of the amino acid sequence of (a); preferably, the protein derived from (a) is a protein in which T3G, T5A, I26V, N27D, H31K, R34K, A36T, D39G, T40S, S50A, E61D, E63K, V65T, I68V, K70R, I73L, H90Y, P102H, R104H, T123S, K126Q and E127N are substituted in the amino acid sequence of (a); or a protein in which T3G, T5A, 126V, N27D, H31K, R34K, A36T, A37S, D38E, D39G, T40S, S50A, E61D, E63K, I68V, K70R, I73L, A81T, H90F, E92D, P102H, R104H, T123S, K126Q and E127N are substituted in the amino acid sequence of (a);
and/or, in (c), the hydrophobic modification is a modification with a long chain hydrophobic fragment such as *n*-dodecane at the cysteine at position 10 of the amino acid sequence of (a); or, the hydrophobic modification is a modification with *n*-dodecane via maleiamide at the cysteine at position 10 of the amino acid sequence of (a);
more preferably:
in (b), the protein derived from (a) has an amino acid sequence of SEQ ID NO: 9, SEQ ID NO: 10 or SEQ ID NO: 11.

10. The drops of claim 8 or 9, wherein the drops contain the fusion protein consisting of a transthyretin and a medicament, the fusion protein has a content of 4-30 µmol/L, preferably 10-15 µmol/L;
and/or, the transthyretin contained in the drops has a content of 4-30 µmol/L, preferably 5-30 µmol/L, more preferably 10-20 µmol/L, such as 10, 15, 20 µmol/L;
and/or, the drops further comprises saline;
and/or, the drops further comprise a surfactant, wherein the surfactant, for example, is Tween 80, which preferably has a content of 5% (v/v);
and/or, the drops is preferably an eye drops;
and/or, the drops is a drops that inhibits ocular retina leakage and/or reduces the number of retinal neovascularization; preferably a drops that treats diabetic retinopathy, age-related macular degeneration and/or retinopathy of prematurity;
and/or, the drops is administered 1-3 times per day, preferably at an amount of 0.3-0.8 nmol protein per eye at each time;
and/or, the drops is administered twice per day, one drop each time, for 3 months; and/or, the drops is administered once per day, one drop each time, for 5 days; and/or, the drops is administered twice per day, one drop each time, for 2 weeks;
and/or, the transthyretin is encoded by a nucleotide sequence of SEQ ID NO: 2;
and/or, the transthyretin is expressed by a recombinant expression vector, and the recombinant expression vector has a plasmid backbone comprising a rhamnose inducible promoter, preferably a rhaPBAD promoter;
and/or, the transthyretin is expressed by a recombinant expression vector, the recombinant expression vector has a plasmid backbone of pET-21a or a vector with 25% or more homology therewith, and preferably a vector with 25% or more homology therewith has a sequence of SEQ ID NO: 8;
and/or, the recombinant plasmid expressing transthyretin is encoded by a nucleotide sequence of SEQ ID NO: 3;
and/or, the transthyretin is expressed in a microbial cell, and preferably followed by a purification; the microbial cell is preferably an *E. coli,* and the *E. coli* preferably comprises *E. coli* BL21, *E. coli* BL21 (DE3), *E. coli* JM109, *E. coli* DH5α, *E. coli* K12 or *E. coli* TOP10; the purification is preferably to remove endotoxin by an endotoxin absorption column and then remove residue bacteria by a filter membrane with a pore size of 0.22 µm;
and/or, the transthyretin is expressed by culturing the transformant comprising a gene of the transthyretin until the bacteria obtained reaches an OD₆₀₀ of 1.5-2.0, such as 1.6, 1.7, 1.8 or 1.9;
and/or, expression of the transthyretin is induced by a reagent for inducible expression, wherein the reagent for inducible expression has a mass volume percentage of 0.1-2%, such as 0.2%, 0.3%, 0.4%, 0.5%, 0.7%, 0.8%, 1.2% or 1.6%, and the expression is preferably induced for a period of 8-20 h, such as 10 h, 12 h, 14 h, 16 h, 17 h, 18 h or 19 h; the reagent for inducible expression is preferably rhamnose or IPTG;
and/or, the fusion protein has a sequence of SEQ ID NO: 6 or SEQ ID NO: 7;
and/or, the transthyretin is expressed with the protein and/or polypeptide medicament as a fusion; and the fusion is preferably a fusion that the protein and/or polypeptide medicament are fused at the N-terminus or C-terminus of the transthyretin; preferably, the transthyretin is expressed with the protein and/or polypeptide medicament as a fusion in a microbial cell, followed by a purification; wherein the purification is preferably to remove endotoxin by an endotoxin absorption column and then remove residue bacteria by a filter membrane with a pore size of 0.22 µm;
and/or, the protein and/or polypeptide medicament comprises lysozyme, albumin and/or EGFR antibody, with a molecular weight of no more than 45 kDa; the lysozyme is preferably hen egg white lysozyme with GenBank Accession No.: AAL69327.1; the albumin is preferably ovalbumin;
and/or, the protein and/or polypeptide medicament comprises a protein and/or polypeptide medicament for treating ocular diseases associated with ocular retina leakage and/or retinal neovascularization such as diabetic retinopathy, age-related macular degeneration and/or retinopathy of prematurity.

11. The drops of any one of claims 8-10, wherein the drops further comprises a pharmaceutically acceptable excipient which is one or more selected from carboxymethyl cellulose or salts thereof, chondroitin sulfate or salts thereof, dextran, and, hyaluronic acid;
preferably:
the carboxymethyl cellulose or salts thereof has a viscosity of 800-1200 CP;
and/or, the chondroitin sulfate is chondroitin sulfate A;
and/or, the dextran is dextran 70;
and/or, the "salts" in the "carboxymethyl cellulose or salts thereof' or "chondroitin sulfate or salts thereof' is independently sodium salts or calcium salts, such as sodium carboxymethyl cellulose or chondroitin sulfate A sodium salt;
and/or, the hyaluronic acid has a molecular weight of 10000-500000;
and/or, the carboxymethyl cellulose or salts thereof has a concentration of 0-8 mg/mL except 0, preferably 2, 4, 6 or 8 mg/mL;
and/or, the chondroitin sulfate or salts thereof has a concentration of 0-40 mg/mL except 0, preferably 10, 20, 30 or 40 mg/mL;
and/or, the dextran has a concentration of 0-0.8 mg/mL except 0, preferably 0.2, 0.4, 0.6 or 0.8 mg/mL;
and/or, the hyaluronic acid has a content of no more than 6% by mass volume percentage, preferably 1-4%, more preferably 2%.

12. The drops of any one of claims 8-11, wherein the drops further comprises a compound, pharmaceutically acceptable salts thereof, or glycoside thereof; the compound is one or more selected from diclofenac, vitamin A and luteolin;
preferably:
the pharmaceutically acceptable salt is a sodium salt, such as diclofenac sodium;
and/or, the glycoside is luteoloside;
and/or, the vitamin A is vitamin A1 and/or vitamin A2;
and/or, the diclofenac or salts thereof has a content of 5-20 µmol/L, such as 10 µmol/L;
and/or, the vitamin A has a content of 2-10 µmol/L, such as 5 µmol/L;
and/or, the luteolin or glycoside thereof has a content of 2-10 µmol/L, such as 5 µmol/L.

13. A use of the drops of any one of claims 8-12 in preparing a medicament for inhibiting ocular retina leakage and/or reducing the number of retinal neovascularization; preferably in preparing the medicament for treating diabetic retinopathy, age-related macular degeneration and/or retinopathy of prematurity.

14. A use of the drops of any one of claims 8-12 in treating ocular diseases; wherein the ocular diseases are preferably ocular diseases associated with ocular retina leakage and/or retinal neovascularization, more preferably diabetic retinopathy, age-related macular degeneration and/or retinopathy of prematurity.

15. A method for treating ocular diseases associated with ocular retina leakage and/or retinal neovascularization such as diabetic retinopathy, age-related macular degeneration and/or retinopathy of prematurity, comprising administering the drops of any one of claims 8-12 to a patient in need thereof.
